# EUROPÄISCHE PATENTSCHRIFT

(11) **EP 2 387 391 B1**
(45) Veröffentlichungstag und Bekanntmachung des Hinweises auf die Patenterteilung: **11.01.2017**
(21) Anmeldenummer: 10757384.2
(22) Anmeldetag: 16.07.2010
(51) Int. Cl.: A61K 9/12, A61K 31/167, A61K 31/192, A61K 31/196, A61K 31/4174, A61K 31/573, A61K 31/7048

(54) **VERFAHREN ZUR ENTWICKLUNG EINER ALS SCHAUM AUF DIE HAUT ZU APPLIZIERENDEN FLÜSSIGEN ZUSAMMENSETZUNG SOWIE EINE TOPISCH APPLIZIERBARE ZUSAMMENSETZUNG**
METHOD FOR DEVELOPING A LIQUID COMPOSITION TO BE APPLIED TO THE SKIN AS A FOAM AND A COMPOSITION THAT CAN BE APPLIED TOPICALLY
PROCÉDÉ POUR METTRE AU POINT UNE COMPOSITION LIQUIDE À APPLIQUER SOUS FORME DE MOUSSE SUR LA PEAU ET COMPOSITION POUR APPLICATION TOPIQUE

(30) Priorität: 24.07.2009 DE 102009034603
(43) Veröffentlichungstag der Anmeldung: 23.11.2011
(73) Patentinhaber: MIKA Pharma Gesellschaft für die Entwicklung und Vermarktung pharmazeutischer Produkte mbH, 67059 Ludwigshafen (DE)
(72) Erfinder: SEIGFRIED, Bernd, G., 67117 Limburgerhof (DE)
(74) Vertreter: Lau-Loskill, Philipp
(86) Internationale Anmeldenummer: PCT/DE2010/000818
(87) Internationale Veröffentlichungsnummer: WO 2011/009436

(56) Entgegenhaltungen:
- EP-A1- 0 510 561
- EP-A1- 0 704 206
- EISVOGEL M: "Schaumschlagen fuer die Kosmetik", COSSMA: COSMETICS, SPRAY TECHNOLOGY, MARKETING, BRAUN FACHVERLAGE, KARLSRUHE, DE, Nr. 4, 1. April 2001 (2001-04-01), Seite 56,58, XP009149424, ISSN: 1439-7676

## Beschreibung

Die vorliegende Erfindung betrifft ein Verfahren zur Entwicklung einer als Schaum auf die Haut zu applizierende flüssige pharmazeutische Zusammensetzung mit den Merkmalen des Oberbegriffs des Patentanspruchs 1 sowie eine entsprechende topisch applizierbare Zusammensetzung mit den Merkmalen des Oberbegriffs des Patentanspruchs 10.

Topisch zu applizierende pharmazeutische Zusammensetzungen, die im flüssigen Zustand vorliegen und die durch Verschäumen bei der Anwendung dann als Schaum vorliegen, sind bekannt. So beschreibt beispielsweise die EP 0 510 561 B1 eine derartige als Schaum zu applizierende pharmazeutische Zusammensetzung, wobei bei der bekannten pharmazeutischen Zusammensetzung die zugrundeliegende Flüssigkeit ausschließlich mechanisch aufgeschäumt wird. Als Inhaltsstoffe enthält die zugrundeliegende flüssige Zusammensetzung, die mechanisch aufgeschäumt wird, als Schaumbildner ein Tensid, ein Lösungsmittel oder ein Lösungsmittelgemisch sowie einen pharmazeutischen Wirkstoff, wobei diese bekannte flüssige Zusammensetzung ohne Verwendung eines Treibmittels allein mechanisch aufschäumbar sein soll. Im Beispiel 5 der EP 0 510 561 B1 wird eine derartige bekannte Zusammensetzung beschrieben, die als Tensid eine wäßrige Alkylamidobetain-Lösung enthält. Zusätzlich ist die in diesem Beispiel beschriebene Zusammensetzung mit Lecithin versehen, wobei dieses, nicht näher beschriebene Lecithin ausschließlich dazu dient, Liposome auszubilden. Ebensowenig wird in der EP 0 510 561 B1 der Schaum bezüglich seines Schaumvolumens oder seiner Schaumstabilität näher beschrieben.

Um das Aufschäumverhalten von flüssigen Zusammensetzungen meßtechnisch festzustellen, sind diverse Meßverfahren bekannt und auch genormt, wie beispielsweise durch die DIN-Norm 53902. Hierbei wird die jeweils zu untersuchende flüssige Zusammensetzung mehr oder weniger reproduzierbar für eine vorgegebene Zeit mechanisch beaufschlagt, um so einen Schaum zu erzeugen, dessen Volumen und dessen Stabilität gemessen wird.

Nach Aussagen der DE 197 40 095 A sollen die bekannten Meßverfahren jedoch keine ausreichende Unterscheidung der Schaumvolumina und Schaumstabilitäten von solchen Zusammensetzungen ermöglichen, die bezüglich ihrer Inhaltsstoffe ähnlich sind.

Von daher schlägt die DE 197 40 095 A vor, die zu vermessende flüssige Zusammensetzung derart aufzuschäumen, daß hierfür ein Rührer mit einem vorgegebenen Profil verwendet wird, der mit einer vorgegebenen Umdrehungszahl angetrieben ist. Der so erzeugte Schaum wird visuell vermessen.

Eine Weiterbildung dieses Schaum-Meßverfahrens ist schließlich in der EP 1 092 970 B1 beschrieben, wobei diese Weiterbildung sich von der zuvor unter Hinweis auf die DE 197 40 095 A beschriebenen Meßtechnik dadurch unterscheidet, daß eine automatische, über Meßelektroden erfolgende Schaumhöhenbestimmung vorgesehen ist. Dieses Meßverfahren, das in dem vorliegenden Text als "SITA-Meßverfahren" bezeichnet wird, erlaubt es somit, einerseits das Schaumvolumen und andererseits die Schaumstabilität von Flüssigkeiten, insbesondere von Färb- und Reinigungslösungen, Galvanikbildern, Emulsionen, Spülmitteln, Körperpflegemitteln oder auch von Bier, zu bestimmen, um hieraus eine Qualitätsaussage zu den zuvor genannten schäumbaren Flüssigkeiten zu machen. Eine Kurzbeschreibung des zuvor genannten Schaummeßverfahrens gemäß EP 1 092 970 B1 findet sich bei Eisvogel M. "Schaumschlagen für die Kosmetik" COSSMA: COSMETICS, SPRAY TECHNOLOGY, MARKETING, BRAUN FACHVERLAGE, KARLSRUHE, DE, Nr. 4, 1. April 2001, Seite 56, 58. Hiernach ist es mit Hilfe dieses Meßverfahrens möglich, das Schaumvolumen, den Schaumaufbau und den Schaumzerfall exakt zu messen, jedoch betrifft diese Kurzbeschreibung ausschließlich kosmetische Schäume oder Waschmittel und erwähnt keine pharmazeutischen Schäume. Weitere Angaben zur chemischen Zusammensetzung der untersuchten Produkte sind in dieser Veröffentlichung nicht enthalten.

Sollen im pharmazeutischen Bereich Flüssigkeiten entwickelt werden, die aufschäumbar sind und somit als Schaum appliziert werden, wird üblicherweise derart vorgegangen, daß eine pharmazeutisch wirksame Flüssigkeit ausgewählt wird, zu der dann versucht wird, durch Variation der Technik des mechanischen Aufschäumens, insbesondere durch Variation des Luftdruckes, der Geometrie und Gestaltung des Schaumkopfes und des Ventils eines entsprechenden Schaumapplikators, aufgeschäumt wird. Ein derartiges Entwicklungsverfahren für aufschäumbare pharmazeutische, im Ursprung flüssige Zusammensetzungen ist jedoch sehr zeitintensiv und beinhaltet zudem noch den Nachteil, daß bei einer Variation der Gestaltung der zuvor angesprochenen Parameter des mechanischen Schaumapplikators ein sich bezüglich seiner Zusammensetzung unterschiedlicher und in sich inhomogener Schaum resultieren kann.

Der vorliegenden Erfindung liegt somit die Aufgabe zugrunde, ein Verfahren zur Entwicklung einer als Schaum auf die Haut zu applizierenden flüssigen pharmazeutischen Zusammensetzung zur Verfügung zu stellen, mit dem die Entwicklungszeit für derartige pharmazeutische Zusammensetzungen erheblich vereinfacht und verkürzt wird und bei der die zugrundeliegende flüssige pharmazeutische Zusammensetzung eine besonders lange Haltbarkeit aufweist.

Desweiteren liegt der vorliegenden Erfindung die Aufgabe zugrunde, eine topisch applizierbare Zusammensetzung, die eine besonders lange Haltbarkeit aufweist, zur Verfügung zu stellen, die als Schaum applizierbar ist und die einen systemisch oder lokal wirkenden pharmazeutischen Wirkstoff enthält, wobei diese topisch applizierbare Zusammensetzung vorzugsweise nach dem zuvor angesprochenen Entwicklungsverfahren entwickelt worden ist.

Die zuerst genannte Aufgabe wird durch ein Verfahren mit den kennzeichnenden Merkmalen des Patentanspruchs 1 und die desweiteren hiernach genannte Aufgabe wird durch eine topisch applizierbare Zusammensetzung mit den kennzeichnenden Merkmalen des Patentanspruchs 10 gelöst.

Das erfindungsgemäße Verfahren zur Entwicklung einer als Schaum auf die Haut zu applizierenden flüssigen pharmazeutischen Zusammensetzung, die als Mindestbestandteile ein Lösungsmittel, einen pharmazeutischen Wirkstoff sowie einen phospholipidischen Schaumbildner aufweist, sieht vor, daß der phospholipidische Schaumbildner ein aus Sojabohnen isolierter phospholipidischer Schaumbildner ist, der Phosphatidylcholin in einer Konzentration zwischen 50 Gew.% und 95 Gew.% enthält, wobei das Phosphatidylcholin eine Säurezahl von maximal 10, eine Peroxidzahl von maximal 10 und eine Ölkonzentration von maximal 6 Gew.%, bezogen auf das Trockengewicht des Phosphatidylcholins, aufweist, daß das Schaumvolumen und die Schaumstabilität des nach dem bei den Ausführungsbeispielen beschriebenen SITA-Meßverfahren ohne Verwendung eines Treibmittels erzeugten Schaumes bestimmt werden, wobei bei dem SITA-Meßverfahren 250 ml der flüssigen Zusammensetzung bei einer Rotordrehzahl von 2.000 U/min durch fünf Rotorzyklen von jeweils 20 Sekunden unter Einhaltung jeweils einer Meßpause von etwa 15 Sekunden zwischen den Rotorzyklen aufgeschäumt werden und das jeweilige Schaumvolumen über Nadeldetektoren alle 50 Sekunden über einen Zeitraum von 35 Minuten erfaßt wird, und daß in der flüssigen Zusammensetzung der mindestens eine Schaumbildner, das mindestens eine Lösungsmittel und der mindestens eine pharmazeutische Wirkstoff von ihrer chemischen Art und/oder ihrer Konzentration her so lange variiert werden, bis der so durch das SITA-Meßverfahren erzeugte Schaum eine Schaumdichte zwischen 0,05 g/ml und 0,8 g/ml besitzt, ein Schaumvolumen zwischen 450 ml und 1.400 ml aufweist und eine solche Schaumstabilität hat, daß der Schaum nach einer Verweilzeit von bis zu fünf Minuten noch mindestens 50 % des Schaumvolumens besitzt, das unmittelbar nach der Erzeugung des Schaumes ursprünglich vorgelegen hat.

Das zuvor beschriebene erfindungsgemäße Verfahren weist eine Reihe von Vorteilen auf. So konnte zunächst überraschend festgestellt werden, daß das erfindungsgemäße Verfahren eine besonders einfache und schnell durchzuführende Entwicklung von solchen flüssigen pharmazeutischen Zusammensetzungen erlaubt, die mit einer Reihe von Schaumapplikatoren reproduzierbar aufschäumbar sind, so daß im Gegensatz zum eingangs geschilderten Stand der Technik pharmazeutische Schäume entwickelt werden können, die bezüglich ihrer Schaumkonsistenz und ihrer Schaumzusammensetzung besonders reproduzierbar sind. Besteht beispielsweise das Ziel einer derartigen Entwicklung, eine flüssige Zusammensetzung zur Verfügung zu stellen, deren Schaum nach Aufschäumen mittels eines geeigneten mechanischen Schaumapplikators eine relativ kurze Stabilität aufweisen soll, so daß er dementsprechend nach dem Auftragen auf die Haut schnell zerfällt, so variiert man bei dem erfindungsgemäßen Verfahren die chemische Art und/oder die Konzentration der Inhaltsstoffe so lange, bis bei dem SITA-Meßverfahren ein Schaum resultiert, dessen Schaumstabilität sich dadurch auszeichnet, daß innerhalb von zwei bis vier Minuten das Schaumvolumen noch einen Wert einnimmt, der zwischen 50 % und 70 % des Schaumvolumens beträgt, das ursprünglich vorlag. Im Gegensatz hierzu sind bei der Zielsetzung, einen besonders stabilen Schaum zu erzeugen, die chemische Art und/oder die Konzentration der Mindestinhaltsstoffe der flüssigen Zusammensetzung, die später den Schaum ausbildet, so zu variieren, daß ein Schaum resultiert, dessen Schaumstabilität so groß ist, daß nach acht bis zehn Minuten noch zwischen 85 % und 99 % des Schaumvolumens vorliegt, bezogen auf das Schaumvolumen, das ursprünglich unmittelbar nach dem Aufschäumen vorgelegen hat. Besteht die Aufgabenstellung hingegen darin, eine flüssige, aufschäumbare Zusammensetzung zur Verfügung zu stellen, die ein besonders großes Schaumvolumen aufweisen soll, so sind die Art und/oder die Konzentration der Mindestinhaltsstoffe dieser flüssigen Zusammensetzung derart zu variieren, daß ein besonders hohes Schaumvolumen beim SITA-Meßverfahren resultiert, d.h. somit ein Schaumvolumen, das zwischen 600 ml und 1.200 ml oder sogar bis 1.400 ml variiert. Insgesamt ist somit festzuhalten, daß das erfindungsgemäße Verfahren ein standardisiertes Verfahren vorschlägt, bei dem durch Variation der chemischen Art der Inhaltsstoffe und/oder ihrer Konzentration ein bezüglich des Schaumvolumens und der Schaumstabilität im vorstehenden Sinne quantifizierten Schaum entwickelt werden kann, der dementsprechend auf der Haut bei Mensch und Tier anzuwenden ist. Durch Auswahl des aus Sojabohnen isolierten phospholipidischen Schaumbildners mit einer Konzentration an Phosphatidylcholin zwischen 50 Gew.% und 95 Gew.% und einer Säurezahl von maximal 10, einer Peroxidzahl von maximal 10 und einer Ölkonzentration von maximal 6 Gew.%, bezogen auf das Trockengewicht des Phosphatidylcholins, ist die dem Schaum zugrundeliegende flüssige Zusammensetzung besonders lange haltbar, ohne daß es hierfür einer erhöhten Konzentration an Antioxidationsmitteln bzw. Stabilisatoren bedarf. Desweiteren konnte festgestellt werden, daß derartige flüssige Zusammensetzungen hervorragende Schäume ergeben, die mit einer Reihe von unterschiedlich ausgebildeten, mechanisch arbeitenden Schaumapplikatoren, applizierbar sind, wenn bei dem erfindungsgemäßen Entwicklungsverfahren aus der flüssigen Zusammensetzung ein solcher Schaum durch das SITA-Meßverfahren erzeugt wird, der eine Schaumdichte zwischen 0,05 g/ml und 0,8 g/ml besitzt.

Eine erste Abwandlung des erfindungsgemäßen Verfahrens sieht vor, daß der durch das SITA-Meßverfahren erzeugte Schaum ein Schaumvolumen zwischen 600 ml und 1.200 ml aufweist.

Bei einer weiteren Abwandlung des erfindungsgemäßen Verfahrens besitzt der durch das SITA-Meßverfahren erzeugte Schaum eine solche Schaumstabilität, daß der Schaum nach einer Verweilzeit von bis zu 10 Minuten noch mindestens zwischen 55 % und 100 % des Schaumvolumens aufweist, das unmittelbar nach der Erzeugung des Schaumes ursprünglich vorgelegen hat.

Bei einer Weiterbildung der zuvor beschriebenen weiteren Abwandlung des erfindungsgemäßen Verfahrens besitzt der nach dem SITA-Meßverfahren erzeugte Schaum eine solche Schaumstabilität, daß er nach einer Verweilzeit von bis zu 10 Minuten noch mindestens zwischen 85 % und 99 % des Schaumvolumens, das unmittelbar nach der Erzeugung des Schaumes ursprünglich vorgelegen hat, aufweist.

Der in der vorliegenden Beschreibung verwendete Begriff "und/oder" bedeutet, daß alle oder einige Elemente der diesbezüglichen Aufzählung additiv oder daß einige oder alle Elemente der diesbezüglichen Aufzählung alternativ zu verstehen sind, während das standardisierte SITA-Meßverfahren, das bei der vorliegenden Erfindung eingesetzt wird, nachfolgend zu Beginn der Ausführungsbeispiele ausgiebig erläutert wird. Desweiteren ist festzuhalten, daß alle in der vorliegenden Beschreibung der Erfindung im Singular verwendeten Begriffe auch den Plural dieser Begriffe abdecken.

Eine erste Weiterbildung des erfindungsgemäßen Verfahrens zur Entwicklung einer als Schaum auf die Haut zu applizierenden flüssigen pharmazeutischen Zusammensetzung sieht vor, daß eine Korrelation zwischen dem nach dem SITA-Meßverfahren spezifizierten Schaum und den erwünschten pharmazeutischen Eigenschaften hergestellt wird. Unter dem Begriff erwünschte pharmazeutische Eigenschaften sind insbesondere der Wirkstofftransport durch die Schichten des Schaumes in und/oder durch die Haut, d.h. somit die Permeation und/oder Penetration der Haut und/oder die Feinverteilung des Wirkstoffes und/oder den hierdurch hervorgerufenen pharmazeutischen Effekt zu verstehen. Ist das Ziel der Entwicklung bei dem erfindungsgemäßen Verfahren beispielsweise darin zu sehen, daß der Wirkstoff in relativ hoher Konzentration schnell an die Hautoberfläche gelangen soll, variiert man in der flüssigen, durch das SITA-Meßverfahren aufzuschäumenden Zusammensetzung die Art und/oder Konzentration der Inhaltsstoffe derart, daß der erzeugte Schaum ein relativ geringes Schaumvolumen, so insbesondere ein Schaumvolumen zwischen 450 ml und 600 ml, und eine relativ geringe Schaumstabilität, so insbesondere eine Schaumstabilität nach fünf Minuten zwischen 55 % und 70 % des Schaumvolumens besitzt, das ursprünglich unmittelbar nach der Erzeugung des Schaumes vorgelegen hat.

In Weiterbildung der zuvor beschriebenen Ausführungsform des erfindungsgemäßen Verfahrens sieht eine andere Ausgestaltung vor, daß ein Schaumapplikator zum mechanischen Aufschäumen der flüssigen Zusammensetzung ausgewählt wird, daß aus der flüssigen Zusammensetzung durch den ausgewählten Schaumapplikators ein Schaum erzeugt wird und daß eine Korrelation zwischen den Eigenschaften, insbesondere den pharmazeutischen Eigenschaften, des über den Schaumapplikator erzeugten Schaumes und dem über das SITA-Meßverfahren ermittelten Schaumvolumen und/oder der Schaumstabilität hergestellt wird. Diese Ausgestaltung des erfindungsgemäßen Verfahrens ermöglicht besonders einfach und schnell, durch Variation der Art und/oder der Konzentration der Inhaltsstoffe der flüssigen pharmazeutischen Zusammensetzung Schäume mit definierten Schaumvolumina und Schaumstabilitäten, gemessen nach dem SITA-Meßverfahren, zu entwickeln, die dann mit den Schäumen korrelieren, die über den ausgewählten Schaumapplikator später als Schaum auf die Haut aufgetragen werden.

Insbesondere dann, wenn bei dem erfindungsgemäßen Entwicklungsverfahren aus der flüssigen Zusammensetzung ein solcher Schaum erzeugt wird, der durch das SITA-Schaummeßverfahren eine Schaumdichte zwischen 0,15 g/ml und 0,4 g/ml besitzt, konnte festgestellt werden, daß derartige flüssige Zusammensetzungen hervorragende Schäume ergeben, die sich mit einer Reihe von unterschiedlich ausgebildeten, mechanisch arbeitenden Schaumapplikatoren applizierbar sind.

Bereits vorstehend ist wiederholt darauf hingewiesen worden, daß bei dem erfindungsgemäßen Verfahren die chemische Art und/oder die Konzentration der Inhaltsstoffe (Lösungsmittel, Schaumbildner, Wirkstoff) derart variiert werden, daß das vorstehend beim erfindungsgemäßen Entwicklungsverfahren aufgeführte Schaumvolumen und die Schaumstabilität sichergestellt werden.

Insbesondere wird bei dem erfindungsgemäßen Verfahren das Lösungsmittel für die Herstellung der flüssigen Zusammensetzung aus der Gruppe ausgewählt, die Wasser, mindestens einen Alkohol, insbesondere mindestens einen 1- bis 3-wertigen Alkohol, mindestens einen Polyalkohol und Mischungen der zuvor genannten Lösungsmittel umfaßt. Selbstverständlich wird bei der Auswahl der Lösungsmittel darauf geachtet, daß diese Lösungsmittel hautverträglich sind und insbesondere auch zu keinen Hautirritationen führen, so daß sie dementsprechend pharmazeutisch anwendbar sind. Bevorzugte Lösungsmittel sind demnach 2-Propanol, Propylenglykol, Glycerin sowie Polyole, wobei der Begriff Wasser alle wäßrigen Systeme, insbesondere auch wäßrige, pharmazeutische Puffersysteme, umfaßt.

Grundsätzlich ist zu dem phospholipidischen Schaumbilder mit einer Phosphatidylcholin-Konzentration zwischen 50 Gew.% und 95 Gew.% festzuhalten, daß der Begriff Phosphatidylcholin auch hydriertes Phosphatidylcholin abdeckt.

Bezüglich der Wirkstoffe, die bei dem erfindungsgemäßen Verfahren verwendet werden, ist festzuhalten, daß es sich hierbei um solche Wirkstoffe bevorzugt handelt, wie diese nachfolgend in Verbindung mit der erfindungsgemäßen topisch applizierbaren Zusammensetzung näher beschrieben sind.

Neben dem zuvor wiederholt genannten Lösungsmittel, dem pharmazeutischen Wirkstoff und dem phospholipidischen Schaumbildner wird bei weiteren Ausführungsformen des erfindungsgemäßen Verfahrens zur Herstellung der flüssigen Zusammensetzung, die rein mechanisch zu ihrer Anwendung aufgeschäumt wird, insbesondere ein Komplexbildner, ein Puffer, ein Verdickungsmittel, ein Antioxidationsmittel und/oder ein Stabilisator vorgesehen. Hierbei ist jedoch darauf zu achten, daß diese zuvor aufgeführten Inhaltsstoffe ebenfalls einen Einfluß auf das Schaumvolumen und die Schaumstabilität haben können, was jedoch bei dem erfindungsgemäßen Verfahren recht einfach und unproblematisch und innerhalb von kürzester Zeit und reproduzierbar feststellbar ist.

Die vorliegende Erfindung betrifft, wie bereits vorstehend ausgeführt ist, desweiteren eine topisch applizierbare Zusammensetzung mit den Merkmalen des Oberbegriffs des Patentanspruchs 10.

Die erfindungsgemäß topisch applizierbare Zusammensetzung, die bevorzugt, jedoch nicht ausschließlich, nach dem zuvor beschriebenen erfindungsgemäßen Verfahren entwickelt wird, weist, wie der nächste Stand der Technik gemäß des Ausführungsbeispiels der EP 0 510 561 einen pharmazeutischen Wirkstoff, ein Lösungsmittel sowie einen phospholipidischen Schaumbildner auf. Im Gegensatz zu dieser bekannten flüssigen Zusammensetzung, die als Schaum applizierbar ist, sieht die erfindungsgemäße topisch applizierbare Zusammensetzung als Schaumbildner einen solchen phospholipidischen Schaumbildner vor, der aus Sojabohnen isoliert ist und der Phosphatidylcholin in einer Konzentration zwischen 50 Gew.% und 95 Gew.% enthält, wobei das Phosphatidylcholin eine Säurezahl von maximal 10, eine Peroxidzahl von maximal 10 und eine Ölkonzentration von maximal 6 Gew.%, bezogen auf das Trockengewicht des Phosphatidylcholins, aufweist. Desweiteren sind bei der erfindungsgemäßen Zusammensetzung der mindestens eine phospholipidische Schaumbildner, das mindestens eine Lösungsmittel und der mindestens eine Wirkstoff von ihrer chemischen Art und/oder ihrer Konzentration her derart aufeinander abgestimmt, daß die Zusammensetzung ohne Verwendung eines zusätzlichen Treibmittels ausschließlich mechanisch aufschäumbar ist. Der so, durch das mechanische Aufschäumen während der Applikation erzeugte Schaum weist eine Schaumdichte zwischen 0,05 g/ml und 0,8 g/ml, ein Schaumvolumen zwischen 450 ml und 1.400 ml auf. Desweiteren hat die erfindungsgemäße Zusammensetzung eine solche Schaumstabilität, daß der Schaum nach einer Verweilzeit von bis zu fünf Minuten noch mindestens 50 % des Schaumvolumens besitzt, das unmittelbar nach der Erzeugung des Schaumes ursprünglich vorgelegen hat, wobei sowohl das zuvor erwähnte Schaumvolumen als auch die zuvor genannte Schaumstabilität nach dem bei den Ausführungsbeispielen beschriebenen standardisierten SITA-Meßverfahren bestimmt sind und wobei das SITA-Meßverfahren 250 ml der flüssigen Zusammensetzung bei einer Rotordrehzahl von 2.000 U/min durch fünf Rotorzyklen von jeweils 20 Sekunden unter Einhaltung jeweils einer Meßpause von etwa 15 Sekunden zwischen den Rotorzyklen aufschäumt und das jeweilige Schaumvolumen über Nadeldetektoren alle 50 Sekunden über einen Zeitraum von 35 Minuten erfaßt. Mit anderen Worten wird die erfindungsgemäße, topisch applizierbare Zusammensetzung neben der Art ihrer Inhaltsstoffe und/oder ihrer Konzentration noch durch den hieraus erstellten Schaum charakterisiert, wobei der Schaum durch das standardisierte SITA-Meßverfahren hinsichtlich des Schaumvolumens und der Schaumstabilität spezifiziert und quantifiziert wird.

Die erfindungsgemäße Zusammensetzung weist eine Reihe von Vorteilen auf. Wird die erfindungsgemäße Zusammensetzung auf menschliche oder tierische Haut appliziert, wobei der Begriff Haut, wie bereits vorstehend definiert, die Schleimhäute von Mund, Nase, Vagina und Vorhaut, die Hautbereiche des Ohres, insbesondere die Innenohres, die Hautbereiche des Afters und des Enddarmes, die Nägel und die Augen, insbesondere die Bindehaut, die Hornhaut und die Tränensäcke, abdeckt, so besitzt der aus der erfindungsgemäßen Zusammensetzung erstellte Schaum an diesen Applikationsorten zunächst eine ausgezeichnete Haftung, so daß er nicht so ohne weiteres in unerwünschter Weise abzuwischen ist. Desweiteren besitzt der aus der erfindungsgemäßen flüssigen Zusammensetzung ausschließlich mechanisch hergestellte Schaum eine in sich homogene Zusammensetzung, wobei der pharmazeutische Wirkstoff in diesem Schaum in einer besonders gleichmäßigen Feinstverteilung vorliegt, so daß nach einer Zerstörung des Schaumes nach dem Auftragen diese Feinstverteilung in der dabei auf der Hautoberfläche entstehenden Flüssigkeitsschicht beibehalten wird, mit der Folge, daß der pharmazeutische Wirkstoff mit einer hohen Penetrations- und/oder Permeations-Rate in und/oder durch die Haut transportiert wird. Dies wiederum führt dazu, daß der so applizierte Wirkstoff eine hohe pharmazeutische Wirksamkeit hervorruft, so daß, falls erwünscht, die Wirkstoffkonzentration in der erfindungsgemäßen Zusammensetzung im Vergleich zu herkömmlichen Zusammensetzungen, reduziert werden kann oder -alternativ hierzu- die zeitlichen Intervalle zwischen aufeinanderfolgenden Anwendungen entsprechend verlängert werden können, sofern insbesondere der Wirkstoff in der Haut ein Wirkstoffdepot ausbildet. Bedingt dadurch, daß in der erfindungsgemäßen Zusammensetzung als Schaumbildner der zuvor spezifizierte phospholipidische Schaumbildner vorhanden ist, bewirkt dieser phospholipidische Schaumbildner, daß hierdurch zusätzlich eine Beschleunigung der Penetration bzw. Permeation in bzw. durch die Haut herbeigeführt wird, was sich als zusätzlicher Vorteil der erfindungsgemäßen Zusammensetzung herausstellt.

Insbesondere dann, wenn der aus der erfindungsgemäßen Zusammensetzung durch das SITA-Meßverfahren erzeugte Schaum eine Schaumdichte zwischen 0,15 g/ml und 0,4 g/ml besitzt, weisen derartige Ausgestaltungen der erfindungsgemäßen Zusammensetzungen die zuvor angesprochenen vorteilhaften Eigenschaften in erhöhtem Maße auf.

Bei einer ersten Abwandlung der erfindungsgemäßen Zusammensetzung besitzt der durch das SITA-Meßverfahren erzeugte Schaum ein Schaumvolumen zwischen 600 ml und 1.200 ml.

Bei einer weiteren Abwandlung der erfindungsgemäßen Zusammensetzung weist der durch das SITA-Meßverfahren erzeugte Schaum eine solche Schaumstabilität auf, bei der der Schaum nach einer Verweilzeit von bis zu 10 Minuten noch mindestens zwischen 55 % und 100 % des Schaumvolumens besitzt, das unmittelbar nach der Erzeugung des Schaumes ursprünglich vorgelegen hat.

Bei einer Weiterbildung der zuvor beschriebenen weiteren Abwandlung der erfindungsgemäßen Zusammensetzung besitzt der Schaum nach einer Verweilzeit von bis zu 10 Minuten noch mindestens zwischen 85 % und 99 % des Schaumvolumens, das unmittelbar nach der Erzeugung des Schaumes ursprünglich vorgelegen hat.

Wie bereits zuvor beim erfindungsgemäßen Verfahren ausführlich beschrieben ist, weist die erfindungsgemäße Zusammensetzung als Lösungsmittel insbesondere Wasser, mindestens einen Alkohol, insbesondere mindestens einen 1- bis 3-wertigen Alkohol und/oder mindestens einen Polyalkohol auf, wobei derartige Lösungsmittel insbesondere auch für solche phospholipidischen Schaumbildner ausgewählt werden, die das aus Sojabohnen isoliertes Phospholipid, wie es vorstehend spezifiziert ist, enthalten.

Klarstellend wird darauf hingewiesen, daß der Begriff Phosphatidylcholin auch hydriertes oder teilhydriertes Phosphatidylcholin abdeckt.

Bezüglich des Wirkstoffes ist festzuhalten, daß der mindestens eine Wirkstoff, der in der erfindungsgemäßen Zusammensetzung enthalten ist, ein solcher Wirkstoff ist, der zur Anwendung bei Mensch oder Tier und insbesondere zur topischen und systemischen und vorzugsweise zur Anwendung auf der Haut geeignet ist, wobei ein besonders bevorzugter Wirkstoff aus der Gruppe ausgewählt ist, die Lokalanästhetika, Antiallergika, Dermatika, Wirkstoffe gegen grippale Infekte und Erkältungskrankheiten, Wirkstoffe zur Behandlung von Neuropathien, Wirkstoffe zur Behandlung von Durchblutungsstörungen, Chemotherapeutika, Chinin, Antimykotika, Antibiotika, Thalidomid, Serotonin, Eicosanoide, Analgetika, Antikonvulsiva, Nicht-steroidale Antirheumatika, Leukotriene, Leukotrienhemmer, Androgene, Antiandrogene, Kortikoide, Opiatrezeptor-Antagonisten, Blutgerinnung hemmende Stoffe, Thrombozytenaggregationshemmer, Histaminantagonisten, regulatorisch und enzymatisch wirkende Peptide und Proteine, Nukleinsäuren (einzel- und doppelsträngige DNA, einzel- und doppelsträngige RNA, snRNA, DNA-Oligonukleotide, RNA-Oligonukleotide) und Oligopeptide, Antipruriginosa, Antidiabetika, Prostaglandine, Prostaglandinsynthesehemmer, Antiviral wirkende oder virostatisch wirkende Substanzen, Antimikrobiell-wirkende Substanzen, Wirkstoffe gegen Prione, Immunsupressiva, Hormone, Wirkstoffe zur Behandlung von Warzen oder Wunden, insbesondere chronischen Wunden, Vitamine, Pflanzenextrakte oder Auszüge aus Pflanzenextrakten, Psychopharmaka, den Schlaf beeinflussende Wirkstoffe, Analeptika, Allgemeinanästhetika, Muskelrelaxantien, Antiepileptika, Antiparkinsonmittel, Antiemetika, Antiparasitika, Ganglionär angreifende Substanzen, am Symphatikus angreifende Substanzen, am Parasymphatikus angreifende Substanzen, antibakteriell wirkende Pharmaka, Calciumantagonisten, Herz-/Kreislaufmittel, Antiasthmatika, Antitussiva, Expektorantien, Hepatika, Diuretika, Choleretika, Desinfektionsmittel, Spurenelemente, Antiinfektiva, Zytostatika, Antimetaboliten, Hormonantagonisten, Immunmodulatoren sowie Derivate und Salze der zuvor genannten Wirkstoffe umfaßt.

Abhängig von der jeweiligen Verwendung der erfindungsgemäßen Zusammensetzung weist diese einen speziellen Wirkstoff oder eine spezielle Wirkstoffmischung auf, der bzw. die aus den nachfolgend aufgeführten speziellen Wirkstoffen, die unter den jeweiligen Hauptgruppen aufgeführt sind, ausgewählt ist.

Vorzugsweise wird für die Hauptgruppe der 5α-Reduktase-Hemmer Alfatradiol und 17α-Estradiol; für die Hauptgruppe der Abmagerungsmittel, Appetitzügler oder Antiadiposita Norephedrin, Phenylpropanolamin, D-Norpseudoephedrin, Orlistat und Sibutramin; für die Hauptgruppe der ACE-Hemmer Benazepril, Cilazapril, Quinapril, Ramipril, Spirapril und Trandolapril; für die Hauptgruppe der Acidosetherapeutika oder Antihypoxämika Calcium-natrium-hydrogencitrat; für die Hauptgruppe der Adstringens Aluminiumchlorid, Aluminiumdiacetat, Aluminiumformiat, Bismutchloridoxid, Bismutgallat, Policresulen, Tannin und Zinkoxid; für die Hauptgruppe der Aknemittel Azelainsäure und Benzoylperoxid; für die Hauptgruppe der Aldosteron-Antagonist Canrenionsäure, Kaliumcanrenoat, Dolasetron und Eplerenon; für die Hauptgruppe der Alkoholentwöhnungsmittel Acamprosat und Disulfiram; für die Hauptgruppe der α₁-Rezeptorenblocker Alfuzosin, Bunazosin und Dihydroergotamin; für die Hauptgruppe der α₂-Rezeptoragonist Apraclonidin, Brimonidin, Doxozosin und Moxonidin; für die Hauptgruppe der α- und β- Sympathomimektikum Adrenalin, Dobutamin, Dopexamin und Epinephrin; für die Hauptgruppe der Aminoglykosid-Antibiotika Gentamicin, Kanamycin, Neomycin, Netilmicin, Streptomycin und Tobramycin; für die Hauptgruppe der Aminosäuren Alanin, Aminoessigsäure, Glycin, Arginin, Asparagin, Asparaginsäure, Cystein, Cystin, Glycocoll, Ornithin, Prolin und Serin; für die Hauptgruppe der Aminosäuresubstitution Alanylglutamin, Argininglutamat, Desmeninol, Glycilglutamin und Glycyltyrosin; für die Hauptgruppe der Analeptika oder Antihypoxämika Campher und Coffein; für die Hauptgruppe der Analgetika oder Antirheumatika Abatacept, Acetylsalicylsäure, Acetaminophen, Ademetionin, Anakinra, Aurothiomalat Natrium, Buprenorphin, Diethylaminsalicylat, Etanercept, Etoricoxid, Fentanyl, Flufenaminsäure, Flupirtin, Glucosamin, Hydromorphon, 2-Hydroxybenzoesäure, Diethylazan-Salz, Hydroxychloroquin, Hydroxyethylsalicylat, Leflunomid, Levomethadon, Meptazinol, Metamizol, Methylsalicylat, Misoprostol, Morphin, Nalbuphin, Natriumaurothiomalat, Nicoboxil, Nonivamid, Noramidopyrin, Novaminsulfon, Oxaceprol, Oxycodon, Paracetamol, Penicillamin, Pethidin, Phenazon, Piritramid, Propylnicotinat, Propyphenazon, Salazosulfapyridin, Sulfasalazin, Tilidin, Tramadol und Ziconotid; für die Hauptgruppe der Ansäuerungsmittel Apfelsäure; für die Hauptgruppe der Antacidum Almasilat, Aluminiumhydroxid, Aluminiumhydroxid-Magnesiumcarbonat-Gel, Aluminiumphosphat, Carbaldrat, Magaldrat, Magnesiumcarbonat, Magnesiumhydroxid und Magnesiumtrisilicat; für die Hauptgruppe der Anthelminthika Albendazol, Mebendazol, Niclosamid, Praziquantel, Pyrantel und Pyrviniumembonat; für die Hauptgruppe der Antiallergika Cromoglicinsäure, Lodoxamid, Mequitazin, Mizolastin und Olopatadin; für die Hauptgruppe der Antianämika Calciumfolinat, Darbepoeton alfa, Eisen, Eisencarboxymaltose, Eisen(II)-chlorid, Eisen (II)-fumarat, Eisen(II)-gluconat, Eisen(II)-succinat, Eisen(II)-sulfat, Eisenglycinsulfat, Eisen(III)-hydroxid-Dextran-Komplex, Eisen(III)- hydroxid-Polymaltose-Komplex, Eisen(III) - hydroxid-Saccharose-Komplex, Eisen(III)- natrium-gluconat-Komplex, Epoetin alfa, Epoetin beta, Epoetin zeta, Erythropoetin, Folsäure und Methoxy-Polyethylglycol-Epoetin beta; für die Hauptgruppe der Antiandrogene Bicalutamid, Chlormadion und Cyproteron; für die Hauptgruppe der Antiarrhythmika Ajmalin, Amiodaron, Chinidin, Detajmiumbitartrat, Flecainid, Lidocain, Mexiletin, Orciprenalin, Prajamaliumbitartrat, Propafenon und Sotalol; für die Hauptgruppe der Antibiotika oder Antiinfektiva Amikacin, Aminosidin, Paromomycin, Chloramphenciol, Ciprofloxacin, Clindamycin, Colistimethat-Natrium, Colistin, Enfuvirtid, Enoxacin, Flucloxacillin, Fosfomycin, Fusafungin, Levofloxacin, Linezolid, Mefloquin, Metronidazol, Mezlocillin, Moxifloxacin, Norfloxacin, Ofloxacin, Oxacillin, Penicillin G, Penicillin V, Phenoxymethylpenicillin, Phenoxymethylpenicillin-Benzathin, Pipemidinsäure, Piperacillin, Piperacillin + Tazobactam, Proguanil, Propicillin, Pyrimethamin, Retapamulin, Rifaximin, Roxithromycin, Sulbactam, Sulbactam + Ampicillin, Sulfadiazin, Spiramycin, Sultamicillin, Tazobactam + Piperacillin, Teicoplanin, Telithromycin, Tigecyclin und Vancomycin; für die Hauptgruppe der Antidementiva (Nootropika) Galantamin, Nicergolin, Nimodipin, Piracetem, Pyritinol und Rivastigmin; für die Hauptgruppe der Antidepressiva Agomelatin, Amitriptylin, Amitriptylinoxid, Bupropion, Citapram, Clomipramin, Duloxetin, Escitalopram, Fluoxetin, Fluvoxamin, Maprotilin, Mianserin, Mirtazapin, Nortriptylin, Opipramol, Paroxetin, Reboxetin, Sertralin, Tranylcypromin, Trazodon und Trimipramin; für die Hauptgruppe der Antidiabetika Acarbose, Exenatid, Glibenclamid, Gliclacid, Glimepirid, Gliquidon, Insulinaspart, Insulinaspart biphasisch, Insulindetemir, Insulinglargin, Insulinglulisin, Insulin human, Insulin human-Isophan biphasisch, Insulin-Isophan, Insulinlispro, Isophan-Insulin, Metformin, Miglitol, Nateglinid, Pioglitazon, Repaglinid, Rosiglitazon, Sitagliptin und Vildagliptin; für die Hauptgruppe der Antidota Bissulfanylpropansulfonsäure, Deferasirox, Deferoxamin, Deferipron, Dimercaptoppropansulfonsäure, Dimethylaminophenol, Dinatriumfolinat Eisenhexacyanoferrat, Eserin, Flumazenil, Fomepizol, Naloxon, Natriumfolinat, Natriumthiosulfat, Obidoximchlorid, Pentetsäure, Physostigmin, Silbinin und Toloniumchlorid; für die Hauptgruppe der Antiemetika oder Antivertiginosa Aprepitant, Betahistin, Domperidon, Flunarizin, Fosaprepitant, Granisetron, Ondansetron, Palonosetron und Tropisetron; für die Hauptgruppe der Antiepileptika Carbamazepin, Clonazepam, Diphenylhydantoin, Phenytoin, Dipropylessigsäure, Valproinsäure, Ethosuximid, Felbamat, Gabapentin, Kaliumbromid, Lacosamid, Lamotrigin, Levetiracetam, Mesuximid, Oxcarbazepin, Phenobarbital, Primidon, Propylvaleriansäure, Rufinamid, Sultiam, Tiagabin, Topiramat, Valproinsäure, Vigabatrin und Zonisamid; für die Hauptgruppe der Antiestrogene Clomife; für die Hauptgruppe der Antihämorrhagika, Antifibrinolytika und andere Hämostatika Aminomethylbenzoesäure, Blutgerinnungsfaktor I vom Menschen, Blutgerinnungsfaktor Vlla, Blutgerinnungsfaktor VII (CHO), Blutgerinnungsfaktor VIII, rekombinant, Blutgerinnungsfaktor VIII vom Menschen, Blutgerinnungsfaktor IX, rekombinant, Blutgerinnungsfaktor IX vom Menschen, Blutgerinnungsfaktor XIII, Eptacog alfa (aktiviert), Fibrinogen, Gelatine, Moroctocog alfa, Nonacog alfa, Octocog alfa (BHK), Phytomenadion, Plasmaproteine human, Plasmaproteine human mit Faktor-VIII-Inhibitor-Bypass-Aktivität, Proconvertin, Protaminhydrochlorid, Tranexamsäure und Troxerutin; für die Hauptgruppe der Antihistaminika Anazolin, Azelastin, Bamipin, Cetirizin, Chlorphenamin, Chlorphenoxamin, Cyproheptadin, Desloratadin, Dexchlorpheniramin, Dimenhydrinat, Dioxopromethazin, Diphenhydramin, Diphenylpyralin, Ebastin, Emedastin, Epinastin, Fexofenadin, Hydroxyzin, Levocabastin, Levocetirizin, Loratadin, Rupatadin, Terfenadin, Tripelennamin und Triprolidin; für die Hauptgruppe der Antihypertonika Aliskiren, Ambrisentan, Amilorid + Hydrochlorothiazid, Hydrochlorothiazid + Amilorid, Bosentan, Candesartan, Captopril, Clonidin, Delapril, Enalapril, Enalaprilat, Eprosartan, Hydralazin, Imidapril, Indapamid, Indoramin, Lercanidipin, Manidipin, Methyldopa, Minoxidil, Moexipril, Nilvadipin, Nitrendipin, Nitroprussidnatrium, Olmesartan, Prazosin, Reserpin, Stickstoffmonoxid, Sitaxentan, Telmisartan, Terazosin, Treprostinil und Urapidil; für die Hauptgruppe der Antihypoglykämika Diazoxid und Glucagon; für die Hauptgruppe der Antihypotonika Ameziniummetilsulfat, Cafedrin, Dopamin, Etilefrin, Levarterenol, Norepinephrin, Midodrin, Noradrenalin, Oxilofrin und Theodrenalin; für die Hauptgruppe der Antikoagulantia Bivalirudin, Certoparin-Natrium, Dabigatran, Dalteparin-Natrium, Danaparoid-Natrium, Drotrecogin alfa (aktiviert), Enoxaparin-Natrium, Fondaparinux, Heparin, Heparin (niedermolekular), Nadroparin-Calcium, Reviparin-Natrium, Tinzaparin-Natrium, Lepirudin, Nadroparin-Calcium, Pentosanpolysulfat-Natrium, Protein C, Reviparin-Natrium, Rivaroxaban, Tinzaparin-Natrium und Warfarin; für die Hauptgruppe der Antimykotika Amorolfin, Amphotericin B, Anidulafungin, Bifonazol, Caspofungin, Ciclopirox, Ciotrimazol, Econazol, Fenticonazol, Fluconazol, Flucytosin, Griseofulvin, Hexamidin, Isoconazol, Itraconazol, Ketoconazol, Micafungin, Miconazol, Naftifin, Natamycin, Nystatin, Oxiconazol, Posaconazol, Sertaconazol, Terbinafin, Tioconazol, Tolnaftat, Undecylensäure und Voriconazol; für die Hauptgruppe der Antineoplastische Mittel Alemtuzumab, Alitretinoin, Bevacizumab, Arsentrioxid, Asparaginase, Bexaroten, Buserelin, Celecoxib, Cetuximab und Colaspase; für die Hauptgruppe der Antiparasitäre Mittel Allethrin I, Essigsäure, Permethrin und Piperonylbutoxid; für die Hauptgruppe der Antiphlogistika Aescin, Ammoniumbituminosulfonat, Ammoniumbituminosulfonat hell, Benzydamin, Bufexamac, Cumarin, Dimethylsulfoxid, Guajazulen, Natriumbituminosulfonat und Serrapeptase; für die Hauptgruppe der Antipruriginosika Crotamiton, Levomenthol, Menthol und Steinkohlenteer; für die Hauptgruppe der Antipsoriatika Acitretin, Dimethylfumarat und Ethylhydrogenfumarat; für die Hauptgruppe der Antipsychotika Aripiprazol; für die Hauptgruppe der Antiseptika Ethacridin, Ethanol, Ethanol vergällt, Fenchon, Glyoxal, Hexetidin, Hydroxychinolinsulfat, Kaliumthiocyanat, Methenamin-Silbernitrat 1:2, Phenoxyethanol, Silber ionisch und Silber kolloidal; für die Hauptgruppe der Antiskabiosa Benzylbenzoat; für die Hauptgruppe der Antitussiva oder Expektorantia Anethol, Benproperin, Cineol, Codein, Dextromethorphan, Dihydrocodein, Dropropizin, Eucalyptol, Guaifenesin, Guajacolglycerinether, Levodropropizin, Narcotin, Natriumdibunat, Noscapin, Pentoxyverin, Thymol und Tyloxapol; für die Hauptgruppe der Antikoagulans Argatroban; für die Hauptgruppe der Anxiolytika Buspiron, für die Hauptgruppe der Appetitzügler Amfepramon und Cathin; für die Hauptgruppe der Aromatase-Hemmer Anastrozol, Exemestan und Letrozol; für die Hauptgruppe der Arteriosklerosemittel Dodecyltetradecylhydroxypolyoxyethylenpolyoxypropylen; für die Hauptgruppe der Balneotherapeutika und Mittel zur Wärmetherapie Huminsäuren; für die Hauptgruppe der β-Lactam-Antibiotika Aztreonam, Imipenem, Cilastatin, Doripenem, Ertapenem, Loracarbef, Meropenem; für die Hauptgruppe der Betarezeptoren-, Calciumkanalblocker und Hemmstoffe des Renin-Angiotensin-Aldosteron-Systems Acebutolol, Atenolol, Bisoprolol, Betaxolol, Bupranolol, Carteolol, Celiprolol, Esmolol, Fosinopril, Gallopamil, Irbesartan, Levobunolol, Lisinopril, Losartan, Metipranolol, Metoprolol, Nebivolol, Nifedipin, Nisoldipin, Oxprenolol, Penbutolol, Perindopril, Pindolol, Propranolol, Talinolol, Valsartan und Verapamil, für die Hauptgruppe der Bisphosphonate Alendronat, Alendronatsäure, Clodronat, Clodronsäure, Etidronat und Etidronsäure; für die Hauptgruppe der Breitenbandpenicillin Amoxicillin und Ampicillin; für die Hauptgruppe der Breitenbandpenicillin+β-Lactamase-Inhibitor Clavulansäure und Sulbactam; für die Hauptgruppe der Bronchodilatatoren Aminophyllin und Bambuterol; für die Hauptgruppe der Broncholytika oder Antiasthmatika Carbocistein, Ciclesonid, Clenbuterol, Fenoterol, Formoterol, Ipratropiumbromid, Ketotifen, Montelukast, Omalizumab, Reproterol, Salbutamol, Salmeterol, Terbutalin, Theophyllin, Theophyllin-Ethylendiamin, Tiotropiumbromid und Tulobuterol; für die Hauptgruppe der Calciumantagonisten Amlodipin, Diltiazem, Felodipin und Isradipin; für die Hauptgruppe der Calcium-Substitutionsmittel Calciumaminoethylphosphat, Calciumaspartat, Calciumbis (hydrogenaspartat), Calciumchlorid, Calciumcitrat, Calciumgluconat, Calciumhydrogenphosphat, Calciumhydrogenphosphat, Calciumlactobionat, Calciumlactogluconat und Calciumsalze; für die Hauptgruppe der Carboanhyrase-hemmer Acetazolamid, Binzolamid und Dorzolamid; für die Hauptgruppe der Cefalosporin Cefaclor, Cefadroxil, Cefalexin, Cefazolin, Cefepim, Cefixim, Cefotaxim, Cefotiam, Cefepodoxim, Ceftazidim, Ceftibuten, Ceftriaxon, Cefuroxim und Ceph; für die Hauptgruppe der Chemotherapeutika Co-trimoxazol, Dapson, Nifuratel, Nitrofural, Nitrofurantoin, Nitrofurazon, Nitroxolin, Octenidin, Pentamidin, Ulfamethoxazol, Taurolidin und Trimethoprim; für die Hauptgruppe der Cholagoga und Gallenwegstherapeutika Menthon, a-Pinen, β-Pinen und Ursodesoxycholsäure; für die Hauptgruppe der Cholinergika Acetylcholinchlorid, Carbachol, Distigminbromid, Neostigmin und Pyridostigminbromid; für die Hauptgruppe der Corticoide Fludrocortison; für die Hauptgruppe der Depotpenicillin Benzylpenicillin-Benzathin und Benzylpenicillin-Procain; für die Hauptgruppe der Dermatika Ammoniumdodecylsulfat, Betacaroten, DFMO, Eflornithin, Difluormethylornithin, Dodecylbenzolsulfonsäure, Nitrilotriethanol-Salz, Ectoin, Estradiolbenzoat, Ethyllinolat, Framycetin, Fusidinsäure, synthetischer Gerbstoff, Phenol-Methanal-Harnstoff-Polykondensat sulfoniert, Harnstoff, Hexamethylentetramin, Hydrochinon, Isotretinoin, Kaliumhydroxid, Keratin, Kupfer(II)-nitrat, Lithiumsuccinat, Methantheliniumbromid, Methenamin, Methoxypsoralen, Mupirocin, Nadifloxacin, Pimecrolimus, Podophyllotoxin, Salicylsäure, Salpetersäure, Selendisulfid, Sulfadiazin-Silber, Tacalcitol, Tretinoin und Tyrothricin; für die Hauptgruppe der Desinfektionsmittel Aminopropyldodecylpropandiamin, Cocospropylendiamin, Dodecylpropandiamin, Ethylendioxydimethanol und Triclosan; für die Hauptgruppe der Desinfizientia oder Antiseptika Aethacridin, Aluminiumacetattartrat, Amylmetacresol, Bibrocathol, Benzalkoniumchlorid, Benzethoniumchlorid, Benzylalkohol, Bishydroxymethylharnstoff, Biphenyl-2-ol, Bromchlorophen, Cetylpyridiniumchlorid, 8-Chinolinolsulfat, Clioquinol, Didecyldimethylammoniumchlorid, Didecylmethyloxyethlammoniumpropionat, Chinolinolsulfat-Kaliumsulfat, Chlorhexidin, Chlorhydroxybenzoesäure, Clorofen, Cocospropylendiaminguanidiniumacetat, Dequaliniumchlorid, Dibromhydroxybenzolsulfonsäure, Dichlorbenzylalkohol, Dithranol, Ethylhexanol, Formaldehyd, Glucoprotamin, Glutaral, Magnesiummonoperoxyphthalat, Mecetroniumetilsulfat, Oligodiiminoimidocarbonyliminohexamethylen, ortho-Phthalaldehyd, Peressigsäure, Polihexanid, Povidon-Iod, 1-Propanol, 2-Propanol, Tetrahydrotetrakishydroxymethylimidazoimidazoldion, Tosylchloramid-Natrium und Wasserstoffperoxid; für die Hauptgruppe der Desodorans Chlorophyllin; für die Hauptgruppe der Diätetika oder Ernährungstherapeutika Methyloxobuttersäure, Methyloxovaleriansäure(3), Methyloxovaleriansäure(4) und Oxophenylpropansäure; für die Hauptgruppe der Diagnostika und Mittel zur Diagnosevorbereitung Aminolävulinsäure, Aminolevulinsäure, 5-Amino-4oxopentansäure, Ceruletid, Corticorelin vom Menschen, Eisenoxid, Ferumoxsil, Fluorescein, Gadobensäure, Gadobutrol, Gadodiamid, Gadofosveset, Gadopentetsäure, Gadoteridol, Gadotersäure, Gadoxetsäure, Galactose, 13C-Harnstoff, Hexylaminooxopentanoat, Indocyaningrün, Mangafodipir, Palmitinsäure, Patentblau V, Perflutren, Polyvinylchlorid, Protirelin, Secretin, Stärkehydrolysat, Somatorelin, TRH und Tuberkulin zu Anwendung am Menschen gereinigt; für die Hauptgruppe der direkten Parasympathomimetika Bethanecholchlorid; für die Hauptgruppe der Diuretika Bumetanid, Furosemid, Piretanid, Spironolacton, Torasemid, Triamteren, Triamteren + Hydrochlorothiazid und Xipamid; für die Hauptgruppe der Dopamin-Agonisten α-Dihydroergocryptin; für die Hauptgruppe der durchblutungsfördernden Mittel Alprostadil, Cinnarizin, Moxaverin, Naftidrofuryl, Pentoxifyllin, Prostaglandin E₁ und Xantinolnicotinat, für die Hauptgruppe der Eisen-Substitution Ammoniumeisensulfat; für die Hauptgruppe der Emetika Apomorphin; für die Hauptgruppe der Entwöhnungsmittel/Mittel zur Behandlung von Suchterkrankungen Naltrexon, Nicotin und Vareniclin; für die Hauptgruppe der Enzym-Ersatz-Therapie bei Fabry-Syndrom Agalsidase alfa und Agalsidase beta; für die Hauptgruppe der Enzyminhibitoren, Präparate bei Enzymmangel und Transportproteine Carglumsäure, L-Carnitin, Levocarnitin, C₁-Esterase-Inhibitor, Galsulfase, Hyaluronidase, Idursulfase, Imiglucerase, Laronidase und Miglustat; für die Hauptgruppe der Enzymersatztherapie bei Morbus Pompe Alglucosidase alfa; für die Hauptgruppe der Estrogene Estriol, konjugierte Estrogene und Ethinylestradiol; für die Hauptgruppe der Fibrinolytika Alteplase, Reteplase, Streptokinase, Tenecteplase und Urokinase; für die Hauptgruppe der Filmbildner Carbomer und Carmellose; für die Hauptgruppe der Gallensteinauflöser Chenodesoxycholsäure; für die Hauptgruppe der Gestagene Dienogest, Drospirenon, Dydrogesteron, Gestoden, Hydroxyprogesteroncaproat, Levonorgestrel, Medrogeston, Medroxyprogesteron, Megestrolacetat, Norelgestromin, Norethisteron, Norgestimat und D-Norgestrel; für die Hauptgruppe der Glaukommittel Bimatoprost und Latanoprost; für die Hauptgruppe der Geriatrika Kaliummetabisulfit; für die Hauptgruppe der Gichtmittel Probenecid; für die Hauptgruppe der Glucocorticoide Alclometason, Amcinonid, Beclometason, Betamethason, Budesonid, Clobetasol, Clobetason, Clocortolon, Cloprednol, Deflazacort, Desoximetason, Dexamethason, Diflorason, Diflucortolon, Flumetason, Flunisolid, Fluocinolonacetonid, Fluocinonid, Fluocortolon, Fluorometholon, Flupredniden, Fluticason, Halometason, Hydrocortison, Hydrocortisonaceponat, Hydrocortisonacetat, Hydrocortison-17-butyrat, Hydrocortisonhydrogensuccinat, Methylprednisolon, Mometasonfuroat, Prednicarbat, Prednisolon, Prednison, Rimexolon, Triamcinolon, Triamcinolonacetonid, Triamcinolon-16,21-diacetat und Triamcinolonhexacetonid; für die Hauptgruppe der Gonadorelininhibito Cetrorelix; für die Hauptgruppe der Gynäkologika Gemeprost, Kupfer, Metergolin, Methylergometrin, Milchsäure, Nonoxinol, Progesteron, Prostaglandin E₂, Quinagolid und Sulproston; für die Hauptgruppe der Hämatopoetischer Wachstumsfaktoren Becaplermin; für die Hauptgruppe der Hämostyptika Cellulose oxidiert regeneriert, Desmoressin und Kollagen; für die Hauptgruppe der Hepatika Acetylmethionin, Betaindihydrogencitrat, Cholinhydrogentartrat, Kalium-Eisen-Phosphat-Citrat-Komplex, Ornithinaspartat und Zinkacetat; für die Hauptgruppe der Herzglykosid (Digitalis lanata) β-Acetyldigoxin, Digitoxin und Digoxin; für die Hauptgruppe der Hyperämisierungsmittel Benzylnicotinat und Isobornylacetat; für die Hauptgruppe der Hypnotika oder Sedativa Brotizolam, Chloralhydrat, Clomethiazol, Doxylamin, Flunitrazepam, Flurazepam, Lormetazepam, Melatonin, Midazolam, Nitrazepam, Temazepam, Zaleplon, Zolpidem und Zopiclon; für die Hauptgruppe der Hypophysen-,Hypothalamushormone, andere regulatorische Peptide und ihre Hemmstoffe Carbetocin, Choriogonadotropin alfa, Choriongonadotropin, Tetracosactid, β⁻¹⁻²⁴-Corticotropin, Follitropin alfa, Follitropin beta, Ganirelix, Gonadorelin, Gonadotrophinum chorionicum, Gonadotrophinum hypophysicum, Menotropin, Lanreotid, LH-RH, Lutropin alfa, Mecaserim, Nafarelin, Octreotid, Oxytocin, Somatostatin, Somatropin, Terlipressin, Thyrotrophin, Urofollitropin, Urogonatropin und menschliches Wachstumshormon; für die Hauptgruppe der Immunmodulatoren Eculizumab, Glatiramer, Lenalidomid, Lenograstim, Palivizumab und Pegvisomant; für die Hauptgruppe der Immunstimulans Aldesleukin Dimepranolacedoben, Filgrastim, Inosin, Interferon alfa-2a, Interferon alfa-2b, Interferon beta-1°, Interferon beta-1b, Interferon gamma-1b, Pegfilgrastim, Peginterferon alfa-2a und Peginterferon alfa-2b; für die Hauptgruppe der Immunsuppresiva Adalimumab, Azathioprin, Basiliximab, Ciclosporin, Cladribin, Cyclosporin, Daclizumab, Efalizumab, Everolimus, Immunglobulin G Kaninchen Antihuman-T-Zell, Infliximab, Muromonab-CD3, Mycophenolatmofetil, Mycophenolsäure, Natalizumab, Sirolimus, Tacrolimus und Tocilizumab; für die Hauptgruppe der Infusions- und Standardinjektionslösungen oder Organperfusionslösungen N-Acetyl-tyrosin, Gelatinepolysuccinat, Glucose, Glutamin, Glyceroldihydrogenphosphat, Humanalbumin, Kaliumhydrogenglutamat, Mannitol, Natriumaminoethylhydrogenphosphat, Natriumchlorid, Natrium-hydrogencarbonat, Ölsäure, 2-Oxoglutarsäure, Polyhydroxyethylstärke, Salzsäure, Taurin, Trometamol und Xylitol; für die Hauptgruppe der Inhalationsnarkotika Desfluran, Distickstoffmonoxid und Isofluran; für die Hauptgruppe der Intestinalen Antiphlogistika 5-Aminosalicylsäure, Mesalazin, (-)-α-Bisabolol, Levomenol, Bromelain und Cholinstearat; für die Hauptgruppe der Kalium-Substitutionsmittel Kaliumacetat, Kaliumchlorid, Kaliumhydrogenaspartat, Kaliumhydrogencarbonat, Kaliumlactat und Kaliummalat; für die Hauptgruppe der Kaliumsparende Diuretika Amilorid; für die Hauptgruppe der kapillarabdichtenden Mittel Calciumdobesilat; für die Hauptgruppe der Kardiaka Enoximon, Icatibant, β-Methyldigoxin" Metildigoxin, Milrinon und Oubain; für die Hauptgruppe der Karies-,Parodontosemittel und andere Dentalpräparate Dectaflur, Natriumfluorid und Olaflur; für die Hauptgruppe der Karminativum Dimethylpolysiloxan und Dimeticon; für die Hauptgruppe der Koronarmittel Ivabradin und Molsidomin; für die Hauptgruppe der Laxantia Bisacodyl, Glycerin, Glycerol, Lactulose, Macrogol, Magnesiumperoxid, Natriumdioctylsulfosuccinat, Natriumlaurylsulfoacetat, Natriummonohydrogenphosphat, Natriumpicosulfat, Natriumsulfat, dickflüssiges Paraffin, Polyethylenglycol und weißes Vaselinöl Paraffin; für die Hauptgruppe der Lichtschutzmittel Actinoquinol; für die Hauptgruppe der Lipidsenker Atorvastatin, Bezafibrat, Colestyramin, Cholestyramin, Etofibrat, Etofyllinclofibrat, Ezetimib, Fenofibrat, Fluvastatin, Gemfibrozil, Lovastatin, Magnesiumpyridoxalphosphatglutamat, Nicotinsäure, Omega-3-Säureethylester, Pravastatin und Simvastatin; für die Hauptgruppe der Lokalanästhetika oder Neuraltherapeutika Aethoform, p-Aminobenzoesäureethylester, Articain, Benzocain, Bupivacain, Carticain, Chlorethan, Cinchocain, Ethylchorid, Felypressin, Macrogollaurylether, Mepivacain, Prilocain, Procain, Proxymetacain, Quinisocain, Ropivacain und Tetracain; für die Hauptgruppe der Magen-Darm-Mittel Hydrotalcit, Lansoprazol, Loperamid, Methylnaltrexonbromid, Metoclopramid, Natriumalginat, Olsalazin, Omeprazol, Oxetacain, Pankreas-Pulver, Pankreatin, Pantoprazol, Pepsin, Pirenzepin, Polymethylsiloxan, Rabeprazol, Racecadotril, Ranitidin, Siliciumdioxid, Simeticon, Sucralfat, Smektit, Tannin-Eiweiß und Tilactase; für die Hauptgruppe der Magnesium- Substitutionsmittel Magnesiumchlorid, Magnesiumsalze und Magnesiumsulfat; für die Hauptgruppe der Makrolidantibiotika Azithromycin, Bacitracin, Clarithromycin, Daptomycin und Erythromycin; für die Hauptgruppe der Migränemittel Almotriptan, Eletriptan, Ergotamin, Frovatriptan, Naratriptan, Rizatriptan, Sumatriptan und Zolmitriptan; für die Hauptgruppe der Mineralstoffpräparate Calciumlactat, Calciumsaccharat, Eisenhydrogenaspartat, Kaliumaminoethylphosphat, Kaliumcitrat, Magnesiumaminoethylphosphat, Magnesiumaspartat, Magnesiumbis(hydrogenaspartat), Magnesiumcitrat, Magnesiumluconat, Magnesiumhydrgencitrat, Magnesiumhydrogenglutamat, Magnesium-hydrogenphosphat, Magnesiumorotat und Magnesiumoxid; für die Hauptgruppe der Mittel bei Homocysteinurie Betain; für die Hauptgruppe der Mittel bei Sklerodermie und Induratio penis plastica 4-Aminobenzoesäure; für die Hauptgruppe der Mucolytikum Acetylcystein; für die Hauptgruppe der Mukolytika Desoxyribonuclease und Dornase alfa; für die Hauptgruppe der Muskelrelaxanzien und -Reversoren Alcuroniumchlorid, Atracuriumbesilat, Chinin, Cisatracuriumbesilat, Dantrolen, Mivacuriumchlorid, Orphenadrin, Pancuroniumbromid, Pridinol, Rocuroniumbromid, Succinylcholinchlorid, Suxamethoniumchlorid, Sugammadex, Tetrazepam, Tizanidin, Tolperison und Vecuroniumbromid; für die Hauptgruppe der Myotonolytika Baclofen und Methocarbamol; für die Hauptgruppe der Narkosemittel Esketamin, Etomidat, Hydroxybuttersäure, Ketamin, Propofol, Remifentanil, Sevofluran, Sufentanil und Thiopental-Natrium; für die Hauptgruppe der Neuroleptika Amisulprid, Azaphenothiazine, Prothipendyl, Benperidol, Bromperidol, Butyrophenone, Chlorprothixen, Clozapin, Diphenylbutylpiperidine, Droperidol, Fluspirilen, Pimozid, Flupentixol, Fluphenazin, Levomepromazin und Melperon; für die Hauptgruppe der Neuropathiepräparate und andere neurotrope Mittel Cytidinphosphat, A-Liponsäure, Thioctsäure, Pregabalin, Riluzol und Uridinphosphat; für die Hauptgruppe der Nichtsteroidale Antiphlogistika Flurbiprofen, Ketorolac-Trometanol, Lornoxicam und Parecoxib; für die Hauptgruppe der nichtsteroidale Antirheumatika Acecolfenac, Acemetacin, Alizaprid, Chloroquin, Dexibuprofen, Diclofenac, Etofenamat, Ibuprofen, Indometacin, Ketoprofen, Meloxicam, Nabumeton, Naproxen, Phenylbutazon, Piroxicam, Proglumetacin und Tiaprofensäure; für die Hauptgruppe der Ophthalmika Chondroitinsulfat, Gramicidin, Hydroxypropyl-Guar, Hydroxypropylmethylcellulose, Hypromellose, Inosinphosphat, Lomefloxacin, Methylhydroxypropylcellulose, Naphazolin, Nedrocromil, Oxybuprocain, Pegaptanib, Pilocarpin, Polymyxin B, Poly(vinylalkohol), Povidon, Ranibizumab, Scopolamin, Sulfacetamid, Tafluprost, Tetryzolin, Timolol, Travoprost, Tropicamid, Verteporfin und Wollwachsalkohole; für die Hauptgruppe der Osteoporosemittel/Calcium-/Knochenstoffwechselregulatoren Alfacalcidol, Calcitonin, Dinatriumfluorophosphat, Eptotermin alfa, Hydroxycolecalciferole, Ibandronat, Ibandronsäure, Pamidronat, Pamidronsäure, Parathyroidhormon vom Menschen, Paricalcitol, Raloxifen, Ranelinsäure-Distrontiumsalz, Risedronat, Risedronsäure, Teriparatid, Tiludronat, Tiludronsäure, Zoledronat und Zoledronsäure; für die Hauptgruppe der Otologika Docusat-Natrium; für die Hauptgruppe der Parkinsonmittel und andere Mittel gegen extrapyramidale Störungen Benserazid, Bromocriptin, Budipin, Cabergolin, Carbidopa, Entacapon, Levodopa, Lisurid, Metixen, Pergolid, Piribedil, Pramipexol, Procyclidin, Rasagilin, Ropinirol, Rotigotin, Selegilin, Tetrabenazin, Tiaprid, Tolcapon und Trihexypenidyl; für die Hauptgruppe der Penicilline Benzylpenicillin und Dicloxacillin; für die Hauptgruppe der Phosphatbinder Algeldrat, wasserhaltiges Aluminiumoxid, Calciumacetat und Calciumcarbonat; für die Hauptgruppe der Phosphat-Substiutionsmittel Natriumglycerophosphat; für die Hauptgruppe der Photosensibilisatoren Ammoidin und Methoxsalen; für die Hauptgruppe der polyaromatische Retinoide Adapalen; für die Hauptgruppe der Progestagenika Desogestrel und Etonogestrel; für die Hauptgruppe der Protease-Inhibitor Atazanavir und Lopinavir; für die Hauptgruppe der Proteinnasenhemmer Antithrombin III; für die Hauptgruppe der Protozoenmittel Artemether und Lumefantrin; für die Hauptgruppe der Psychoanalpeptika Atomoxetin, Metamfepramon und Methylphenidat; für die Hauptgruppe der Psychoenergetika Deanol; für die Hauptgruppe der Psychopharmaka Doxepin, Haloperidol, Imipramin, Lithiumsalze, Lorazepam, Medazepam, Memantin, Moclobemid, Modafinil, Olanzapin, Oxazepam, Paliperidon, Perazin, Perphenazin, Phenothiazine, Pimozid, Pipamperon, Prazepam, Promethazin, Prothipendyl, Quetiapin, Risperidon, Sertindol, Sulpirid, Thioridazin, Thiocanthene, Venlafaxin, Ziprasidon, Zotepin und Zuclopenthixol; für die Hauptgruppe der Rhinologika oder Sinusitismittel Emser Salz, künstliches Emser Salz, natürliches Meersalz, Oxymetazolin, Silbereiweißacetyltannat, Tramazolin, Xanthangummi und Xylometazolin; für die Hauptgruppe der Roborantia oder Tonika Eisen(III)-citrat und Glutaminsäure; für die Hauptgruppe der Röntgenkontrastmittel Amidotrizoesäure, Bariumsulfat, Diatrizoate, Iobitridol, Iodixanol, Iohexol, Iomeprol, Iopamidol, Iopromid, Iosarcol, Iotrolan, Iotroxinsäure, Ioxaglinsäure und Ioxitalaminsäure; für die Hauptgruppe der Salurektika Bemetizid, Bendroflumethiazid, Chlortalidon, Clopamid, Hydrochlorothiazid, Hydrochlorothiazid + Amilorid, Hydrochlorothiazid + Triamteren und Mefrusid; für die Hauptgruppe der Schilddrüsentherapeutika Cinacalcet, Kaliumiodid, Levothyroxin, Liothyronin, Natriumiodid, Natriumperchlorat, Propylthiouracil, Thiouracile und L-Thyroxin; für die Hauptgruppe der essentielle Aminosäuren Histidin, Isoleucin, Leucin, Lysin, Phenylalanin, Threonin, Tryptophan, Tyrosin und Valin; für die Hauptgruppe der Sekretolytika Ambroxol und Bromhexin; für die Hauptgruppe der Sera, Immunglobuline und Impfstoffe Immunglobulin (Anti-D), Immunglobulin (Botulismus), Immunglobulin (Cytomegalie), Immunglobulin (Hepatitis B), Immunglobulin (human), Immunglobulin (Tetanus), Immunglobulin (Tollwut) und Immunglobulin (Varizella-Zoster); für die Hauptgruppe der Sexualhormone und ihre Hemmstoffe Estradiol Estradiolvalerat, Mestranol, Mifepriston, Prasteron, Testosteron und Tibolon; für die Hauptgruppe der Spasmolytika oder Anticholinergika Atropin, Atropinsulfat, Biperiden, Bornaprin, Borneol, Butylscopolaminiumbromid, Camphen, Cyclopentolat, Darfenacin, Glycopyrroniumbromid, Hymecromon, Hyoscinbutylbromid, Mebeverin und Pipenzolatbromid; für die Hauptgruppe der Spurenelemente Bis(L-histidinato)zink, Chromchlorid, Chromhydrogenaspartat, Cobalthydrogenaspartat, Eisen(III)-clorid, Kupfer(II)-chlorid, Kupfer(II)-hydrogenaspartat, Mangan(II)-chlorid, Mangan(II)-hydrogenaspartat, Natriummolybdat, Natriumselenit, Zinkaspartat, Zinkbishydrogenaspartat, Zinkchlorid, Zinkgluconat, Zink-Histidin, Zinkorotat und Zinksulfat; für die Hauptgruppe der Substitutionsmittel Dinatriumhydrogencitrat, Magnesiumacetat, Natriumacetat, Natriumhydroxid und Natriumlactat; für die Hauptgruppe der Sympathomimektikum Dipivefrin und Ephedrin; für die Hauptgruppe der Tetracycline Chlortetracyclin, Demeclocyclin, Doxycyclin, Meclocyclin, Minocyclin, Oxytetracyclin und Tetracyclin; für die Hauptgruppe der Thrombozytenaggregationshemmer Abciximab, Cilostazol, Clopidogrel, Eptifibatid, Iloprost, Ticlopidin und Tirofiban; für die Hauptgruppe der Thyreostatika Carbimazol, Methimazol und Thiamazol; für die Hauptgruppe der Tokolytika Atosiban; für die Hauptgruppe der Toxoplasmose-, Pneumocystis-carinii-Pneumonie Mittel Atovaquon; für die Hauptgruppe der Tranquilizer (Benzodiazepin) Alprazolam, Bromazepam, Chlordiazepoxid, Clobazam, Diazepam und Dikaliumclorazepat; für die Hauptgruppe der Tuberkulosemittel Aminosalicylsäure, Ethambutol, Isoniazid, Protionamid, Pyrazinamid, Rifampicin und Terizidon; für die Hauptgruppe der Ulkustherpeutika Bismutnitrat, Bismuttetraoxodialuminat, Cimetidin, Esomeprazol und Famotidin; für die Hauptgruppe der Urikostatika Allopurinol und Benzbromaron; für die Hauptgruppe der Urologika Dutasterid, Fesoterodin, Finasterid, Flavoxat, Kaliumaminobenzoat, Kaliumnatriumhydrogencitrat, Lanthan(III)-carbonat, Mercaptamin, Methionin, Oxybutynin, Phenoxybenzamin, Phytosterol, Polystyroldivinylbezolsulfonsäure, Polystyrolsulfonsäure, Propiverin, Propyl-4-hydroxybenzoat, Sevelamer, Solifenacin, Tamsulosin, Tiopronin, Tolterodin, Trospiumchlorid und Yohimbin; für die Hauptgruppe der Uterusmittel Dinoproston; für die Hauptgruppe der Vasodilatatoren Adenosin, Buflomedil, Carvedilol, Codergocrin, Dihydralazin, Dihydroergotoxin, Dipyridamol, Glyceroltrinitrat, Isosorbiddinitrat, Isosorbidmononitrat Nitroglycerin, Pentaerythrityltetranitrat, Sildenafil, Tadalafil, Trapidil und Vardenafil; für die Hauptgruppe der Venentherapeutika Heparinoide, Mucopolysaccharidpolyschwefelsäureester, oligo(O-Sulfo)rutosid, Polidocanol, Rutin und Rutosid; für die Hauptgruppe der Venentonisierendes Mittel Diosmin; für die Hauptgruppe der Virustatika Abacavir, Aciclovir, Adefovir, Amantadin, Brivudin, Cidofovir, Darunavir, Didanosin, Efavirenz, Emtricitabin, Entecavir, Etravirin, Famciclovir, Fosamprenavir, Ganciclovir, Idoxuridin, Imiquimod, Indinavir, Interferon beta, Lamivudin, Maraviroc, Nelfinavir, Nevirapin, Oseltamivir, Raltegravir, Ribavirin, Ritonavir, Saquinavir, Stavudin, Telbivudin, Tenofovir, Tipranavir, Trifluridin, Tromantadin, Valaciclovir, Valganciclovir, Zanamivir und Zidovudin; für die Hauptgruppe der Vitamine Aneurin, Ascorbinsäure, Benfotiamin, Biotin, Calcifediol, Ergocalciferol, Calciferol, Calcipotriol, Calcitriol, Calciumpantothenat, Colecalciferol, Cyanocobalamin, Dihdrotachysterol, Hydroxocobalamin, gereinigte Kieselerde, Natriumascorbat, Natriumpantothenat, Nicotinamid, Nicotinsäureamid, Pyridoxin, Retinol, Riboflavin, Thiamin, Thiamindihydrogenphosphat-dihydrogenphosphat (Ester-Salz), Thiamindisulfid, Thiaminnitrat, α-Tocopherol, RRR-α-Tocopherol, α-Tocopherolacetat, RRR-α-Tocopherolacetat, DL-α-Tocopherolhydrogensuccinat, RRR-α-Tocopherolhydrogensuccinat, Vitamin A, Vitamin-A-Säure, Vitamin B₁, Vitamin B₂, Vitamin B₆, Vitamin B₁₂, Vitamin C, Vitamin D₂, Vitamin D₃, Vitamin E und Vitamin K₁; für die Hauptgruppe der Wund- und Narbenbehandlungsmittel Allantoin, Calciumalginat, Dexpanthenol, Ethylcyanoacrylat, Lactid-Caprolacton-Copolymere, Poly(butylmethacrylatco-methylmethacrylat) (x:y), Polyurethane und Titandioxid; für die Hauptgruppe der Mittel zur Harnansäuerung Ammoniumchlorid; für die Hauptgruppe der zytoreduktiven Mittel Anagrelid; für die Hauptgruppe der Zytostatika, andere antineoplastische Mittel und Protektiva Adriamycin, Amethopterin, Bendamustin, Bleomycin, Bortezombi, Busulfan, Capecitabin, Carboplatin, CCNU, Lomustin, Chlorambucil, Cisplatin, Cyclophosphamid, Cytarabin, Dacarbazin, Dasatinib, Daunorubicin, Dexrazoxan, Docetaxel, Doxorubicin, Epirubicin, Erlotinib, Estramustin, Etoposid, Fludarabin, Fluorouracil, Flutamid, 5-FU, Fulvestrant, Gemcitabin, Goserelin, Hydroxycarbamid, Ibritumomabtiuxetan, Idarubicin, Ifosfamid, Imatinib, Irinotecan, Lapatinib, Leuprorelin, Melphalan, Mercaptopurin, Mesna, Methotrexat, Methylaminooxopentanoat, Miltefosin, Mitomycin, Mitotan, Mitoxantron, Nelarabin, Nilotinib, Nimustin, Oxaliplatin, Paclitaxel, Palifermin, Panitumumab, Pegaspargase, Pemetrexed, Porfimer-Natrium, Procarbazin, Rasburicase, Rituximab, Sorafenib, Sunitinib, Tamoxifen, Tegafur, Temoporfin, Temozolomid, Temsirolimus, Thiotepa, Tioguanin, Topotecan, Toremifen, Trabectedin, Trastuzumab, Treosulfan, Triptorelin, Trofosfamid, Uracil, Vinblastin, Vincristin Vindesin und Vinorelbin; für die Hauptgruppe der Biomaterialien oder medizinische Kunststoffe oder Varia Hydroxylapatit, Methylmethacrylat, Poly(methylacrylat-co-methylmethacrylat) (x:y), Tricalciumbisphosphat und Zirconium(IV)-oxid als spezieller Wirkstoff bzw. Wirkstoffmischung ausgewählt.

Eine besonders geeignete Ausführungsform der erfindungsgemäßen Zusammensetzung sieht vor, daß diese Ausgestaltung der erfindungsgemäßen Zusammensetzung als Wirkstoff ein Analgetikum, insbesondere ein solches Analgetikum, das aus den zuvor aufgeführten speziellen Analgetika ausgewählt ist, aufweist, wobei die Konzentration dieses Analgetikums in der flüssigen Zusammensetzung, die mechanisch aufgeschäumt wird, insbesondere zwischen 0,1 Gew.% und 20 Gew.%, vorzugsweise in einer Konzentration zwischen 2 Gew.% und 10 Gew.%, variiert.

Soll die erfindungsgemäße Zusammensetzung als Schaum zur Behandlung von Pilzinfektionen verwendet werden, so enthält sie als pharmazeutischen Wirkstoff mindestens ein Antimykotikum, insbesondere ein solches Antimykotikum, das aus den zuvor aufgeführten speziellen Antimykotika ausgewählt ist. Vorzugsweise ist dieses Antimykotikum aus der Gruppe ausgewählt, die Clotrimazol, Bifonazol, Econazol, Fenticonazol, Isoconazol, Oxiconazol, Sertaconazol, Tioconazol, Terbinafin, Miconazol, Ketoconazol, Itraconazol, Fluconazol, Voriconazol sowie Derivate der vorgenannten Substanzen umfaßt. Insbesondere variiert die Konzentration dieses antimykotischen Wirkstoffes zwischen 0,01 Gew.% und 10 Gew.%, insbesondere zwischen 0,2 Gew.% und 5 Gew.%, jeweils bezogen auf die flüssige Zusammensetzung, die bei der Anwendung ausschließlich mechanisch aufgeschäumt wird. Ein aus einer solchen flüssigen Zusammensetzung bei der Applikation ausschließlich mechanisch hergestellter Schaum läßt sich dann insbesondere mit einer sehr hohen pharmazeutischen Wirksamkeit bei Nagel- und Fußpilz-Infektionen sowie bei Hefepilzinfektionen anwenden, wobei sich die hohe pharmazeutische Wirksamkeit dadurch ausdrückt, daß bereits nach wenigen Anwendungen die diesbezügliche Pilzinfektion eingeschränkt und bei einer Wiederholung der Anwendung auch nach kurzer Zeit geheilt wird.

Eine andere Ausgestaltung der erfindungsgemäßen Zusammensetzung weist als Wirkstoff mindestens einen kortikoiden Wirkstoff, insbesondere einen solchen kortikoiden Wirkstoff, der aus den zuvor aufgeführten speziellen kortikoiden Wirkstoffen ausgewählt ist, auf, wobei der kortikoide Wirkstoff vorzugsweise aus der Gruppe ausgewählt ist, die Glucokortikoide, Mineralkortikoide und Derivate hiervon umfaßt. Abhängig von dem jeweiligen kortikoiden Wirkstoff, der in der erfindungsgemäßen Zusammensetzung enthalten ist, variiert die Konzentration dieses kortikoiden Wirkstoffes zwischen 0,001 Gew.% und 3 Gew.%, vorzugsweise zwischen 0,1 und 0,8 Gew.%.

Bevorzugte Glukokortikoide sind aus der Gruppe ausgewählt, die Clobetasol-17-propionat, Diflucortolon-21-valerat, Amcinonid, Betamethason-17,21-dipropionat, Betamethason-17-valerat, Desocimetason, Diflucortolon-21-valerat, Fluocinolonacetonid, Fluocinonid, Fluocortolon, Flupredniden-21-acetat, Fluticason-17-propionat, Halcinonid, Hydrocortison-21-acetat-17-propionat, Hydrocortison-17-butyrat-21-propionat, Hydrocortison-17-butyrat, Methylpredisolonaceponat, Mometason, Mometasonfuroat, Prednicarbat, Triamcinolonacetonid, Clobetasonbutyrat, Clocortolon-21-pivalat, Fluocortinbutyl, Flumetason-21-pivalat, Hydrocortison sowie Derivate der vorgenannten Substanzen umfaßt.

Eine weitere Ausgestaltung der erfindungsgemäßen Zusammensetzung schlägt vor, daß hierbei die erfindungsgemäße Zusammensetzung als Wirkstoff mindestens ein Lokalanästhetikum, insbesondere ein solches Lokalanästhetikum, das aus den zuvor aufgeführten speziellen Lokalanästhetika ausgewählt ist, enthält, wobei die Konzentration dieses Lokalanästhetikum, abhängig von dem jeweils konkreten Wirkstoffes insbesondere zwischen 3 Gew.% und 15 Gew.%, vorzugsweise zwischen 6 Gew.% und 12 Gew.%, bezogen auf die Wirkstoffkonzentration in der flüssigen Zusammensetzung, variiert.

Besonders bevorzugte Lokalanästhetika sind dabei aus der Gruppe ausgewählt, die die Benzocain, Procain, Tetracain, Lidocain, Etidocain, Prilocain, Mepivacain, Bupivacain, S-Ropivacain, Articain und deren Derivate umfaßt.

Eine andere Ausgestaltung der erfindungsgemäßen Zusammensetzung sieht vor, daß die Zusammensetzung mindestens einen Immunmodulator in einer Konzentration zwischen 0,03 Gew.% und 0,1 Gew.% enthält, wobei die vorstehend aufgeführten speziellen Immunmodulatoren besonders bevorzugt sind.

Eine besonders geeignete Weiterbildung der erfindungsgemäßen Zusammensetzung enthält als Wirkstoff bzw. Wirkstoffgemisch ein nichtopioides Analgetikum/Antiphlogistikum, insbesondere ein solches nichtopioides Analgetikum/Antiphlogistikum, das aus den zuvor aufgeführten speziellen nicht-opioiden Analgetika/Antiphlogistika ausgewählt ist. Hierunter fallen insbesondere die Salicylate, so vorzugsweise Acetylsalicylsäure und/oder Diflunisal, Essigsäure-Derivate, so insbesondere Indometacin, Acemetacin, Diclofenac und/oder Lonazolac, Propionsäure-Derivate, so insbesondere Ibuprofen, Flurbi-profen, Ketoprofen, Dexketoprofen, Dexibuprofen, Tarenflurbil, Nimesulide, Naproxen und/oder Tiaprofensäure, Oxicame, so insbesondere Piroxicam, Tenoxicam, Meloxicam und/oder Lornoxicam, Anthranilsäure-Derivate, so insbesondere Mefenaminsäure und/oder Flufenaminsäure, Anilin-Derivate, so insbesondere Paracetamol, und 1-Phenyl-2,3-dimethyl-3-pyrazolin-5-on-Derivate, so insbesondere Phenazon, Propyphenazon und/oder Metamizol, deren Salze und deren Derivate umfaßt.

Abhängig von der jeweiligen Anwendung der zuvor beschriebenen Ausführungsformen der erfindungsgemäßen Zusammensetzung variiert die Wirkstoffkonzentration des Analgetikum/Anti-phlogistikum in der flüssigen Zusammensetzung zwischen 0,5 Gew.% und 8 Gew.%, insbesondere zwischen 1 Gew.% und 5 Gew.%.

Selbstverständlich ist es auch möglich, daß die erfindungsgemäße Zusammensetzung als Wirkstoff ein Gemisch von Wirkstoffen enthält, sofern dieses Wirkstoffgemisch untereinander verträglich ist. Derartige Weiterbildungen der erfindungsgemäßen Zusammensetzung werden dann verwendet, wenn allgemein leichtere Hauterkrankungen, wie beispielsweise leichtere Formen von Ekzemen, Akne, Flechten, Insektenstichen, Mykosen und/oder Behandlungen von oberflächlichen Wunden mit dem aus der erfindungsgemäßen Zusammensetzung erzeugten Schaum behandelt werden, wobei dann der Wirkstoff bzw. das Wirkstoffgemisch ausgewählt ist aus der Gruppe, die Terbinafin, Clobetasonbutyrat, Erythromycin, Benzocain, Dexamethason, Calcipotriol, Tretinoin, Minoxidil, Bifonazol, Dexpanthenol, Salicylsäure, Prednicarbat, Mometasonfuroat enthalten.

Klarstellend ist festzuhalten, daß sich alle, in dieser Beschreibung wiedergegebenen Konzentrationsangaben jeweils auf die flüssige Zusammensetzung vor ihrem Aufschäumen beziehen, es sei denn, daß ausdrücklich etwas anderes ausgeführt ist.

Insbesondere wenn dann, wenn dieser phospholipidische Schaumbildner maximal 15 Gew.% Lyso-Phosphatidylcholin, maximal 10 Gew.% Phosphatidsäure und maximal 10 Gew.% Phosphatidylethanolamin enthält, läßt sich mit Hilfe dieses speziellen Schaumbildners ein Schaum erzeugen, der durch Variation seiner Konzentration vielfältig auf die jeweiligen Anforderungen des Applikationsortes anpaßbar ist.

Grundsätzlich ist festzuhalten, daß die Konzentration des phospholipidischen Schaumbildners, der in der flüssigen Zusammensetzung enthalten ist, so ausgewählt wird, daß die eingangs bei dem erfindungsgemäßen Verfahren sowie der erfindungsgemäßen Zusammensetzung aufgeführten und durch das Schaumvolumen sowie durch die Schaumstabilität spezifizierten Schäume erzeugt werden können. Vorzugsweise weist die flüssige Zusammensetzung, die rein mechanisch aufgeschäumt wird, den phospholipdischen Schaumbildner in einer Konzentration zwischen 2 Gew.% und 25 Gew.%, insbesondere in einer Konzentration zwischen 4 Gew.% und 15 Gew.%, auf.

Die vorstehend beim erfindungsgemäßen Verfahren wiedergegebenen allgemeinen Ausführungen gelten auch für die erfindungsgemäße Zusammensetzung, wobei die erfindungsgemäße Zusammensetzung neben Wasser vorzugsweise einen Alkohol und insbesondere Propylenglykol enthält, dessen Konzentration, abhängig von dem erwünschten und vorstehend spezifizierten Schaum, zwischen 2 Gew.% und 25 Gew.%, insbesondere zwischen 5 Gew.% und 15 Gew.%, variiert.

Bezüglich des pH-Wertes ist festzuhalten, daß insbesondere die erfindungsgemäße flüssige Zusammensetzung einen pH-Wert aufweist, der hautverträglich ist und abhängig von dem jeweiligen Applikationsort zwischen 4,8 und 8,8 liegt.

Um den zuvor angesprochenen pH-Wert sicherzustellen, bietet es sich als besonders vorteilhaft an, wenn der erfindungsgemäßen Zusammensetzung mindestens ein Puffer, insbesondere Natriumdihydrogenphosphat-Dihydrat und/oder Di-Natriumhydrogenphosphat-Dodecahydrat, zugesetzt wird.

Grundsätzlich läßt sich die erfindungsgemäße Zusammensetzung, wie sie vorstehend ausführlich beschrieben ist, mit jedem geeigneten Schaumapplikator aufschäumen, wobei besonders geeignete Applikatoren von der Firma Rexam/Airspray (www.rexamairspray.com) unter der Bezeichnung "M3 Minischäumer" (M3 mini foamer) oder von der Firma Calmar/MeadWestvaco (Keltec) hergestellt und vertrieben werden. In bezug auf diese speziellen Schaumapplikatoren wird auch auf die EP 0 565 713 sowie die EP 0 613 728 verwiesen, aus denen weitere technische Details zu diesen Schaumapplikatoren zu entnehmen sind.

Insbesondere betrifft somit auch die vorliegende Erfindung einen Schaumapplikator, der mit der erfindungsgemäßen Zusammensetzung, wie diese vorstehend ausführlich beschrieben ist, befüllt ist.

Ferner betrifft die vorliegende Erfindung in einer ersten Ausführungsform die Verwendung einer topisch als Schaum auf die Haut eines warmblütigen Säugetiers zu applizierenden und ohne Treibmittel aufschäumbaren flüssigen Zusammensetzung der vorstehend beschriebenen Art zur Herstellung eines Arzneimittels zur Behandlung von atopischem Ekzem oder Neurodermitis, wobei das Arzneimittel den Immunmodulator Tacrolimus enthält. Abhängig von dem jeweils ausgewählten Immunmodulator varriert dessen Konzentration vorzugsweise zwischen 0,03 Gew.% und 0,1 Gew.%.

Eine zweite Ausführungsform sieht die Verwendung einer topisch als Schaum auf die Haut eines warmblütigen Säugetieres zu applizierenden und ohne Treibmittel aufschäumbaren flüssigen Zusammensetzung der vorstehend beschriebenen Art zur Herstellung eines Arzneimittels zur Behandlung von entzündlichen oder juckenden Hauterkrankungen, Psoriasis, Dermatitis, Neurodermitis oder Schuppenflechte vor. Vorzugsweise wird bei dieser Verwendung das Glucocorticoid aus der Gruppe, die Betamethason, Dexamethason, Predincarbat, Mometasonfuroat und Clobetasonbutyrat umfaßt, ausgewählt. Je nach Glucocorticoid variiert dessen Konzentration zwischen 0,01 Gew.% und 0,4 Gew.%.

Eine dritte Ausführungsform sieht die Verwendung einer topisch als Schaum auf die Haut eines warmblütigen Säugetieres zu applizierenden und ohne Treibmittel aufschäumbaren flüssigen Zusammensetzung der vorstehend beschriebenen Art zur Herstellung eines Arzneimittels zur Behandlung von Schmerzen, Entzündungen, rheumatischen Erkrankungen oder akuten Traumen vor. Vorzugsweise wird bei dieser Verwendung das Analgetikum aus der Gruppe, die Diclofenac, Ketoprofen und Ibuprofen umfaßt, ausgewählt. Je nach Analgetikum variiert dessen Konzentration zwischen 0,1 Gew.% und 20 Gew.%, vorzugsweise in einer Konzentration zwischen 2 Gew.% und 10 Gew.%.

Eine vierte Ausführungsform sieht die Verwendung einer topisch als Schaum auf die Haut und/oder die Nägel eines warmblütigen Säugetieres zu applizierenden und ohne Treibmittel aufschäumbaren flüssigen Zusammensetzung der vorstehend beschriebenen Art zur Herstellung eines Arzneimittels zur Behandlung von mykotischen Infektionen vor. Vorzugsweise wird bei dieser Verwendung das Antimykotikum aus der Gruppe, die Bifonazol, Terbinafin umfaßt, ausgewählt. Je nach Antimykotikum variiert dessen Konzentration zwischen 0,1 Gew.% und 20 Gew.%, vorzugsweise zwischen 2 Gew.% und 10 Gew.%.

Eine fünfte Ausführungsform sieht die Verwendung einer topisch als Schaum auf die Haut eines warmblütigen Säugetieres zu applizierenden und ohne Treibmittel aufschäumbaren flüssigen Zusammensetzung der vorstehend beschriebenen Art zur Herstellung eines Arzneimittels zur Behandlung von Infektionen mit grampositiven Keimen, anaeroben Keimen und Mykoplasmen, insbesondere zur Behandlung von Akne vor. Vorzugsweise wird bei dieser Verwendung als Antibiotikum Erythromycin, insbesondere in einer Konzentration zwischen 2 Gew.% und 4 Gew.%, ausgewählt.

Eine sechste Ausführungsform sieht die Verwendung einer topisch als Schaum auf die Haut eines warmblütigen Säugetieres zu applizierenden und ohne Treibmittel aufschäumbaren flüssigen Zusammensetzung der vorstehend beschriebenen Art zur Herstellung eines Arzneimittels zur Behandlung von Juckreiz der Haut vor. Vorzugsweise wird bei dieser Verwendung als Lokalanästhetikum Benzocain und/oder Lidocain, insbesondere in einer Konzentration zwischen 1 Gew.% und 20 Gew.%, vorzugsweise 2 Gew.% und 10 Gew.%, ausgewählt.

Eine siebte Ausführungsform sieht die Verwendung einer topisch als Schaum auf die Haut eines warmblütigen Säugetieres zu applizierenden und ohne Treibmittel aufschäumbaren flüssigen Zusammensetzung der vorstehend beschriebenen Art zur Herstellung eines Arzneimittels zur Behandlung von Schuppenflechte vor. , wobei das Arzneimittel den Wirkstoff Calcipotriol in einer Konzentration zwischen 0,005 Gew.% und 0,05 Gew.% enthält. Vorzugsweise wird bei dieser Verwendung das Calcipotriol in einer Konzentration zwischen 0,005 Gew.% und 0,05 Gew.% vorgesehen.

Eine achte Ausführungsform sieht die Verwendung einer topisch als Schaum auf die Haut eines warmblütigen Säugetieres zu applizierenden und ohne Treibmittel aufschäumbaren flüssigen Zusammensetzung der vorstehend beschriebenen Art zur Herstellung eines Arzneimittels zur Behandlung von Akne, insbesondere Akne comedonica und Akne papulopustulosa vor. Vorzugsweise wird bei dieser Verwendung das Tretinoin in einer Konzentration zwischen 0,05 Gew.% und 0,1 Gew.% verwendet.

Eine neunte Ausführungsform sieht die Verwendung einer topisch als Schaum auf die Haut eines warmblütigen Säugetieres zu applizierenden und ohne Treibmittel aufschäumbaren flüssigen Zusammensetzung der vorstehend beschriebenen Art zur Herstellung eines Arzneimittels zur Behandlung von Haarausfall vor. Vorzugsweise wird bei dieser Verwendung das Minoxidil in einer Konzentration zwischen 3 Gew.% und 6 Gew.% vorgesehen.

Eine zehnte Ausführungsform sieht die Verwendung einer topisch als Schaum auf die Haut eines warmblütigen Säugetieres zu applizierenden und ohne Treibmittel aufschäumbaren flüssigen Zusammensetzung der vorstehend beschriebenen Art zur Herstellung eines Arzneimittels zur antiseptischen Behandlung von oberflächlichen Wunden vor. Vorzugsweise wird bei dieser Verwendung das Dexpanthenol in einer Konzentration zwischen 0,03 Gew.% und 1 Gew.% vorgesehen.

Eine elfte Ausführungsform sieht die Verwendung einer topisch als Schaum auf die Haut eines warmblütigen Säugetieres zu applizierenden und ohne Treibmittel aufschäumbaren flüssigen Zusammensetzung der vorstehend beschriebenen Art zur Herstellung eines Arzneimittels zur Behandlung von Herpes und den Herpes-begleitenden Nebenwirkungen vor. Vorzugsweise wird bei dieser Verwendung das Aciclovir in einer Konzentration zwischen 3 Gew.% und 7 Gew.% vorgesehen.

Eine zwölte Ausführungsform sieht die Verwendung einer topisch als Schaum auf die Haut eines warmblütigen Säugetieres zu applizierenden und ohne Treibmittel aufschäumbaren flüssigen Zusammensetzung der vorstehend beschriebenen Art zur Herstellung eines Arzneimittels zur Behandlung von leichter bis mittelschwerer Psoriasis der Kopfhaut vor. Vorzugsweise wird bei dieser Verwendung die Salicylsäure in einer Konzentration zwischen 8 Gew.% und 12 Gew.% vorgesehen. Vorstehend ist bei der Beschreibung der verschiedenen Ausführungsformen der erfindungsgemäßen Verwendung der Begriff Auftragen im Singular verwendet. Hierunter soll jedoch innerhalb einer vorgegebenen Zeit, insbesondere innerhalb von 24 Stunden, auch das wiederholte, zeitversetzte Auftragen fallen.

Ebenso deckt der in dem gesamten Text verwendete Begriff Haut nicht nur die jeweils erkrankten Hautbereiche sondern auch alle, für das Auftragen des aus der erfindungsgemäßen Zusammensetzung erstellten Schaumes zugänglichen Oberflächen des menschlichen oder tierischen Körpers ab, so insbesondere neben der eigentlichen Haut oder Kopfhaut auch Nägel, Haare, Zähne, Hufe oder die Schleimhaut in Mund, Nase, Vagina oder Vorhaut, die Bereiche des Ohrs und insbesondere die Bereiche des inneren Ohres, den Bereich des Darmausgangs und des Enddarmes, den Bereich der Augen, insbesondere den Bereich unter dem Augenlid, wie beispielsweise Bindehaut, Hornhaut und Tränensack, während der Begriff Säugetiere Tiere und Menschen umfaßt.

Klarstellend wird zur Vermeidung von Wiederholungen darauf hingewiesen, daß die eingangs im Zusammenhang mit dem erfindungsgemäßen Verfahren beschriebenen Ausführungen, Details und Vorteile analog für die erfindungsgemäße Zusammensetzung und auch für das vorstehend beschriebene erfindungsgemäße Behandlungsverfahren sowie die im Zusammenhang mit der erfindungsgemäßen Zusammensetzung beschriebenen Ausführungen, Details und Vorteile analog auch für das erfindungsgemäße Verfahren sowie das erfindungsgemäße Behandlungsverfahren gelten.

Desweiteren betrifft die vorliegende Erfindung eine topisch zu applizierende flüssige Zusammensetzung, die mindestens einen phospholipidischen Schaumbildner, mindestens einen pharmazeutischen Wirkstoff und wenigstens ein Lösungsmittel aufweist, wobei die erfindungsgemäße Zusammensetzung ohne Verwendung eines Treibmittels allein durch eine mechanische Behandlung derart aufschäumbar ist, daß beim mechanischen Aufschäumen von 250 ml der flüssigen Zusammensetzung ein solcher Schaum entsteht, dessen Schaumvolumen wenigstens 400 ml beträgt und dessen Schaumstabilität derart ist, daß wenigstens 50 % des ursprünglichen Schaumvolumens nach etwa fünf Minuten und vorzugsweise nach wenigstens fünf Minuten, gemessen bei 25 °C, noch vorhanden ist. Sowohl die zuvor angesprochene Schaumstabilität als auch das zuvor genannte Schaumvolumen werden durch ein standardisiertes, nachfolgend noch beschriebenes SITA-Meßverfahren erfaßt. In diesem Zusammenhang ist anzumerken, daß die so anhand des Schaumvolumens und der Schaumstabilität spezifizierte flüssige Zusammensetzung unmittelbar vor ihrer Anwendung allein durch eine mechanische Behandlung ohne Verwendung eines Treibmittels, wie diese vorstehend bereits wiederholt beschrieben ist, aufgeschäumt wird, so daß dementsprechend der so erzeugte Schaum zur topischen Anwendung verwendet wird. Diese Ausführungsform der erfindungsgemäßen Zusammensetzung weist analog oder identisch alle die Vorteile auf, wie diese vorstehend wiederholt beschrieben sind. Dies gilt auch für die vorstehend beschriebenen Weiterbildungen.

Vorzugsweise weist die zuvor beschriebene erfindungsgemäße Zusammensetzung ein Schaumvolumen auf, das zwischen 450 ml und 1.400 ml, insbesondere zwischen 600 ml und 1.200 ml, variiert.

Besonders dann, wenn die erfindungsgemäße Zusammensetzung eine solche Schaumstabilität besitzt, daß nach fünf Minuten nach Erzeugung des Schaumes noch ein Schaumvolumen resultiert, das 55 % bis 85 % des Schaumvolumens entspricht, das unmittelbar nach dem Erzeugen des Schaumes vorhanden ist, weist diese Weiterbildung der erfindungsgemäßen Zusammensetzung eine hohe pharmazeutische Wirksamkeit auf. Dieser Vorteil ist auch dann gegeben, wenn das Schaumvolumen innerhalb von zehn Minuten nach Erzeugung des Schaumes mindestens 50 %, vorzugsweise zwischen 85 % und 100 %, bezogen auf das Schaumvolumen unmittelbar nach Erzeugung, beträgt.

Insbesondere weist der aus der erfindungsgemäßen flüssigen Zusammensetzung hergestellte Schaum eine Dichte auf, die zwischen 0,05 g/ml und 0,8 g/ml, vorzugsweise zwischen 0,15 g/ml und 0,4 g/ml, variiert.

Bezüglich des in der erfindungsgemäßen Zusammensetzung enthaltenen Lösungsmittels ist festzuhalten, daß es sich hierbei insbesondere um ein anorganisches und/oder organisches Lösungsmittel handelt, wobei das anorganische Lösungsmittel bevorzugt Wasser und das organische Lösungsmittel ein Alkohol oder ein Alkoholgemisch und vorzugsweise ein Polyalkohol und/oder die zuvor in Verbindung mit Patentanspruch 5 beschriebenen Lösungsmittel sind.

Darüber hinaus betrifft die vorliegende Erfindung insbesondere auch ein Verfahren zur Herstellung und/oder zur Entwicklung einer aufschäumbaren flüssigen Zusammensetzung, die für die topische Anwendung vorgesehen ist, wobei das erfindungsgemäße Verfahren folgende Schritte umfaßt:
a) Auswahl einer flüssigen Zusammensetzung, die wenigstens einen pharmazeutischen Wirkstoff, wenigenstens ein Lösungsmittel und wenigstens einen phospholipidischen Schaumbildner umfaßt;
b) mechanische Erzeugung eines Schaumes aus dieser flüssigen Zusammensetzung;
c) Erfassung der Schaumoberfläche, um die Schaumstabilität und das Schaumvolumen zu bestimmen;
d) Variation der Konzentration des wenigstens einen pharmazeutischen Wirkstoffes, des wenigstens einen Lösungsmittels oder des wenigstens einen phospholipidischen Schaumbildners;
e) Wiederholung der Schritte b) und c) so lange, bis 250 ml der flüssigen Zusammensetzung nach mechanischer Erzeugung des Schaumes ein Schaumvolumen von wenigstens 400 ml und eine solche Schaumstabilität aufweist, daß wenigstens 50 % des ursprünglichen Schaumvolumens nach wenigstens fünf Minuten bei 25 °C noch vorhanden ist.

Auch für dieses erfindungsgemäße Verfahren gelten identisch oder analog die zuvor beschriebenen Vorteile und Weiterbildungen.

Vorteilhafte Weiterbildungen der zvuor beschriebenen Erfindung sind in den Unteransprüchen angegeben.

Die vorliegende Erfindung wird nachfolgend anhand von Ausführungsbeispielen näher beschrieben.

### Ausführungsbeispiele

### Beschreibung des SITA-Meßverfahrens

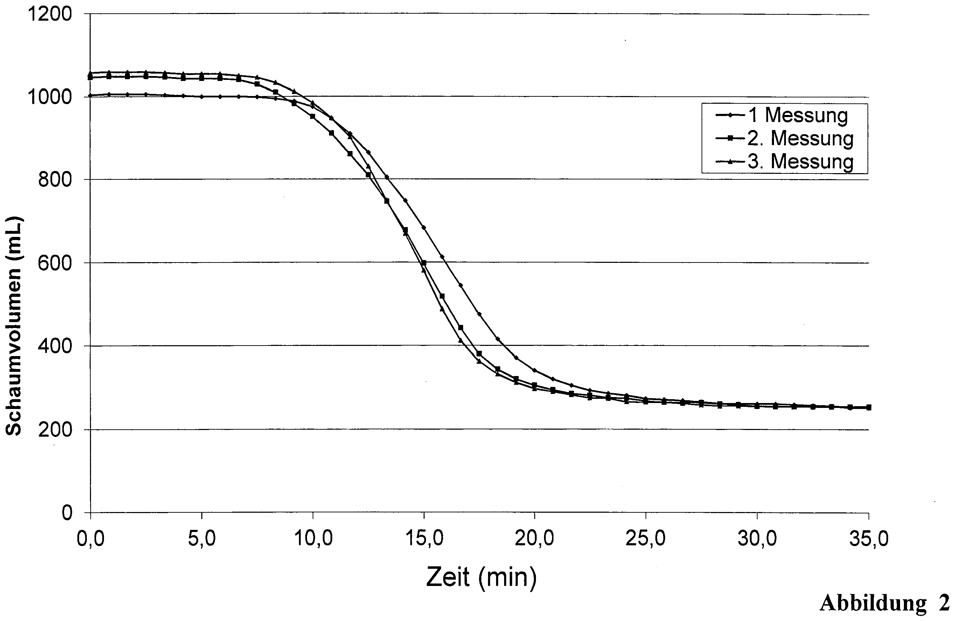

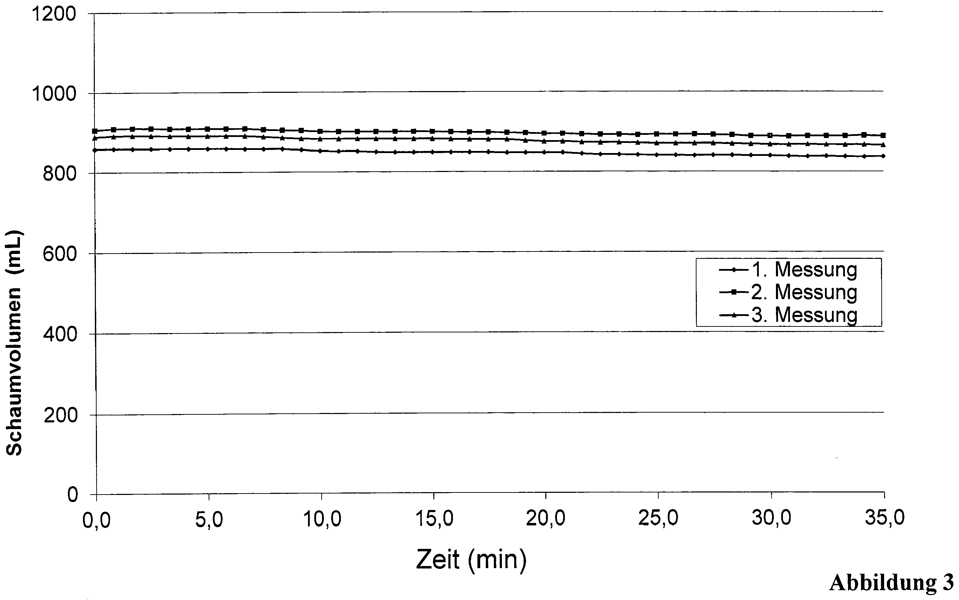

Zur Bestimmung des Schaumvolumens und der Schaumstabilität wurde ein "SITA foam tester R-2000" (Hersteller: SITA Meßtechnik GmbH, Dresden) verwendet, der in der EP 1 092 970 im Detail beschrieben ist. Hierbei war dieses Meßgerät mit einem Rotor versehen, wie dieser in den Figuren 2 und 3 der DE 197 40 095 abgebildet und dort auch beschrieben ist. Dieser Rotor besteht aus einer Rührwelle und einer horizontal hierzu ausgerichteten Kreisscheibe mit einem Durchmesser von 70 mm, wobei oberhalb und unterhalb der Kreisscheibe jeweils vier, rechtwinklig zueinander ausgerichtete, symmetrische Rührblätter vorgesehen sind. Jedes Rührblatt besitzt eine rechteckige Grundfläche von 23 mm x 12 mm. Im Querschnitt gesehen ist jedes Rührblatt dreieckig ausgebildet und weist eine Höhe von 5 mm auf, so daß jede Rührfläche dementsprechend ein Dach mit einem Firstwinkel von 90° ausbildet. Die Rührblätter bestehen jeweils aus einem Conidur-Feinlochblech (Hersteller: Firma HeinLehmann, Krefeld) und besitzen eine Blechdicke von 0,5 mm, eine Lochung von 0,5 sowie eine Teilung von 3,2.

Bei allen Messungen betrug das Probevolumen 250 ml, die automatisch durch das Meßgerät dem Vorratsbehälter, der mit mindestens 300 ml Probe gefüllt wurde, entnommen wurden. Die Probe wurde jeweils vorsichtig und möglichst unter Vermeidung von Schaumbildung in den Vorratsbehälter gegeben. Nach einer Wartezeit von zehn Minuten, so daß eventuell beim Einfüllen entstandene Luftblasen an die Oberfläche wandern konnten und somit keine Verfälschung des Volumens hierdurch eintrat, wurden jeweils 250 ml der zu untersuchenden Probe in den Meßraum eingezogen und vermessen.

Mit einer Rotordrehzahl von 2.000 U/min wurde die jeweils zu untersuchenden Proben im Meßraum fünf Rotorzyklen von jeweils 20 Sekunden zur Schaumerzeugung unterworfen. Zwischen den Rotorzyklen lag eine Meßpause von etwa 15 Sekunden.

Mittels der in der DE 199 49 922 beschriebenen Sensoren, die automatisch die Schaumoberfläche kontinuierlich abtastete, wurde das Schaumvolumen unmittelbar nach Ablauf der fünf Rotorzyklen gemessen. Die Schaumstabilität wurde über einen Zeitraum von insgesamt 35 Minuten automatisch durch das Gerät erfaßt, wobei das Schaumvolumen hierzu mittels der Nadeldetektoren alle 50 Sekunden gemessen wurde. Die so ermittelten Volumenwerte wurden über eine dem Gerät zugeordnete Soft- und Hardware direkt registriert.

Die Steuerung des SITA-foam testers ist derart, daß nach der Befüllung des Meßraumes mit 250 ml Probe die Nadeldetektoren nur bis zur Oberfläche der Probe fahren und dementsprechend den Nullpunkt für das Schaumvolumen auf die Oberfläche der Meßprobe und nicht auf den Boden des Meßraumes legen. Nach Ablauf der zuvor genannten Rotorzyklen zum Aufschäumen der jeweiligen Probe verblieb, abhängig von der Zusammensetzung der jeweils untersuchten Probe, bisweilen noch im Meßraum eine flüssige Phase der Probe, so daß dementsprechend zu dem jeweiligen Meßwert des Schaumvolumens das Volumen dieser flüssigen Phase bei der zuvor beschriebenen Messung noch miterfaßt wurde und dementsprechend als Schaumvolumen im Sinne der vorliegenden Beschreibung definiert wird, wobei die zeitliche Änderung dieses Schaumvolumens die Schaumstabilität ist. Mit anderen Worten bedeutet dies, daß sich, abhängig von der jeweils gemessenen Probe, das Schaumvolumen nicht nur aus dem Volumen des eigentlichen Schaums sondern auch aus dem im Meßraum verbleibenden Volumen der nicht aufgeschäumten flüssigen Probe zusammensetzt.

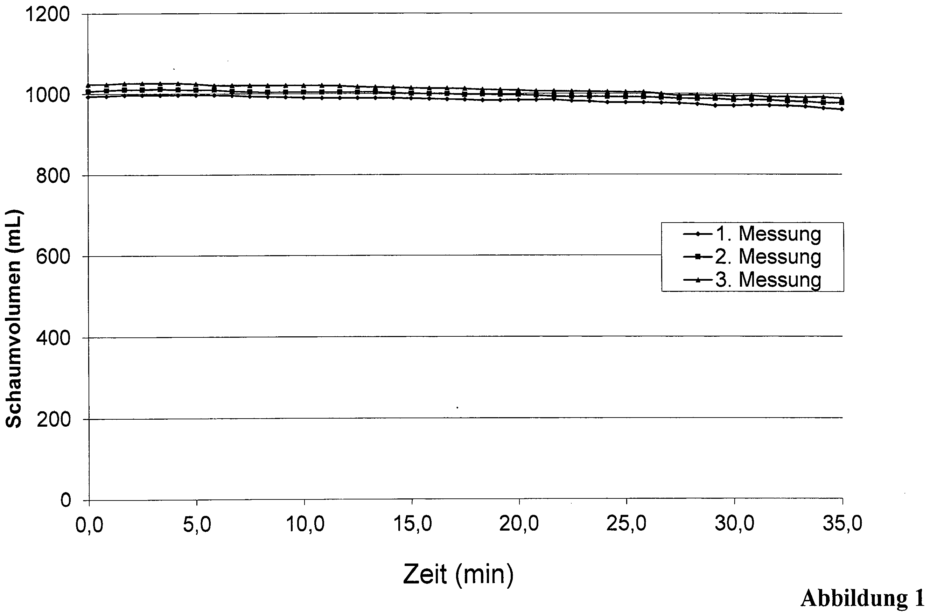

Die folgenden Abbildungen 1 bis 3 und 5 bis 19 bilden für die jeweilige Zusammensetzung jeweils alle drei Meßwerte ab, so daß hieraus sehr gut die Reproduzierbarkeit der Meßmethode zu erkennen ist.

Alle die nachfolgenden Ausführungsbeispiele, bei denen als Wirkstoff Diclofenac angegeben ist, weisen diesen Wirkstoff in Form des Natriumsalzes des Diclofenacs, d.h. somit Diclofenac-Natrium auf.

### Ausführungsbeispiele 1 bis 3 und 5

Es wurde nach dem üblichen Herstellungsverfahren eine Ketoprofenhaltige Zusammensetzung, eine Lidocainhydrochlorid-haltige Zusammensetzung, eine Prednicarbat-haltige Zusammensetzung und eine Clotrimazol-haltige Zusammensetzung hergestellt, die folgende Inhaltsstoffe aufwiesen:

| | **Inhaltsstoffe** | **Zusammensetzung in Gew.%** |
|---|---|---|
| 1 | Ketoprofen | 10.00 |
| 2 | Propylenglycol | 10.00 |
| 3 | 2-Propanol | 8.00 |
| 4 | phospholipidischer Schaumbildner A | 10.00 |
| 5 | Natriumhydrogenphosphat-Dihydrat | 0.25 |
| 6 | di-Natriumhydrogenphosphat-Dodecahydrat krist. | 0.57 |
| 7 | Natrium Hydroxid | 1.55 |
| 8 | Pfefferminzöl | 0.15 |
| 9 | Wasser reinst | 59.48 |
| | **TOTAL** | **100.00** |

Das Schaumverhalten dieser Zusammensetzung 1 ist in der Abbildung 1 wiedergegeben.

| | **Inhaltsstoff** | **Zusammensetzung in Gew.%** |
|---|---|---|
| 1 | Lidocainhydrochlorid | 10.00 |
| 2 | Propylenglycol | 10.00 |
| 3 | 2-Propanol | 11.00 |
| 4 | phospholipidischer Schaumbildner A | 10.00 |
| 5 | Natriumdihydrogenphosphat-Dihydrat | 0.12 |
| 6 | di-Natriumhydrogenphosphat-Dodecahydrat krist. | 0.66 |
| 7 | Natrium Hydroxid 20% w/w | 4.00 |
| 8 | Pfefferminzöl | 0.15 |
| 9 | Wasser reinst | 54.07 |
| | **TOTAL** | **100.00** |

Das Schaumverhalten dieser Zusammensetzung 2 ist in der Abbildung 2 wiedergegeben.

| | **Inhaltsstoff** | **Zusammensetzung in Gew.%** |
|---|---|---|
| 1 | Prednicarbat | 0.10 |
| 2 | Propylenglycol | 15.00 |
| 3 | 2-Propanol | 9.35 |
| 4 | phospholipidischer Schaumbildner B | 5.00 |
| 5 | Natriumdihydrogenphosphat-Dihydrat | 0.50 |
| 6 | di-Natriumhydrogenphosphat-Dodecahydrat krist. | 1.14 |
| 7 | Natrium Hydroxid 10% w/w | 1.00 |
| 8 | Tegosoft GC | 8.60 |
| 9 | Wasser reinst | 59,31 |
| | **TOTAL** | **100.00** |

Das Schaumverhalten dieser Zusammensetzung 3 ist in der Abbildung 3 wiedergegeben.

| | **Inhaltsstoff** | **Zusammensetzung in Gew.%** |
|---|---|---|
| 1 | Clotrimazol | 0.5 |
| 2 | Propylenglycol | 20.00 |
| 3 | 2-Propanol | 8.00 |
| 4 | phospholipidischer Schaumbildner A | 8.00 |
| 5 | Natriumdihydrogenphosphat-Dihydrat | 0.12 |
| 6 | di-Natriumhydrogenphosphat-Dodecahydrat krist. | 0.66 |
| 7 | Natrium Hydroxid 20% w/w | 1.00 |
| 8 | Pfefferminzöl | 0.20 |
| 9 | Polysorbat 80 | 13.00 |
| 10 | Wasser reinst | 48.52 |
| | **TOTAL** | **100.00** |

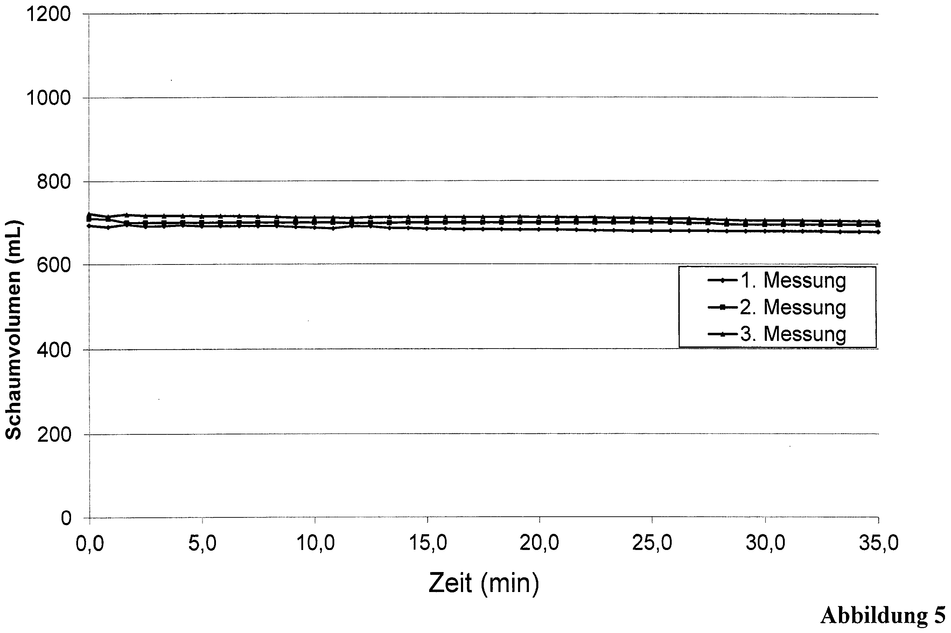

Das Schaumverhalten dieser Zusammensetzung 5 ist in der Abbildung 5 wiedergegeben.

An 20 Probanden (11 weiblich, 9 männlich), die an Pilzerkrankungen der Zwischenzehenräume und teilweise auch der Zehennägel litten, wurden jeweils zweimal täglich die mit Pilz erkrankten Bereiche des linken Fußes mit einem Schaum behandelt, der nach dem zuvor beschriebenen SITA-Meßverfahren hergestellt worden ist. Die Behandlung erfolgte derart, daß der jeweils erkrankte Bereich mit einer etwa 0,5 bis 1 cm dicken Schaumschicht abgedeckt und hiernach dieser Schaum manuell eingerieben wurde. Die Gesamtbehandlungszeit dauerte bis zu 14 Tagen an.

Der jeweils erkrankte Bereich des rechten Fußes wurde mit einer von den Inhaltsstoffen identischen Zusammensetzung 5 behandelt, wobei diese Zusammensetzung 5 mit Hilfe eines "M3 Minischäumer" (M3 mini foamer) von Rexam/Airspray unmittelbar vor der Applikation verschäumt wurde.

Unabhängig davon, welcher Schaum auf die erkrankten Bereiche aufgetragen wurde, berichteten 16 Probanden bereits nach dem erstmaligen Auftragen des entsprechenden Schaumes von einem direkten Nachlassen des Juckreizes. Zwei weitere Probanden konnten dieses Nachlassen des Juckreizes nach zweimaligem Auftragen feststellen, die restlichen beiden Probanden stellten das Nachlassen des Juckreizes nach viermaligem Auftragen fest.

Bei zehn Probanden waren die Pilzinfektionen nach einer Gesamtbehandlungszeit von acht Tagen beseitigt, bei sechs Probanden betrug die Ausheilzeit elf Tage und bei vier Probanden betrug die Ausheilzeit 14 Tage. Hierzu ist anzumerken, daß die letzten vier Probanden am stärksten von der Pilzinfektion betroffen waren. Einen Unterschied zwischen dem nach dem SITA-Meßverfahren erzeugten Schaum und dem durch den "M3 Minischäumer" erstellten Schaum konnte kein Proband feststellen.

### Ausführungsbeispiele 6 bis 8

Um den Einfluß der Konzentration des Wirkstoffes auf die Schaumbildung zu untersuchen, wurden die nachfolgenden Zusammensetzungen 6 bis 8 hergestellt, die sich bezüglich der Wirkstoffkonzentrationen an Diclofenac unterscheiden.

| | **Inhaltsstoffe** | **Zusammensetzung in Gew.%** |
|---|---|---|
| 1 | Diclofenac | 1,000 |
| 2 | Propylenglycol | 15.000 |
| 3 | 2-Propanol | 10,250 |
| 4 | Ascorbylpalmitat | 0,020 |
| 5 | phospholipidischer Schaumbildner A | 13,330 |
| 6 | Natriumdihydrogenphosphat-Dihydrat | 0,120 |
| 7 | di-Natriumhydrogenphosphat-Dodecahydrat krist. | 0,660 |
| 8 | EDTA | 0,040 |
| 9 | Wasser reinst | 59,380 |
| 10 | Pfefferminzöl rektifiziert | 0,200 |
| | **TOTAL** | **100.00** |

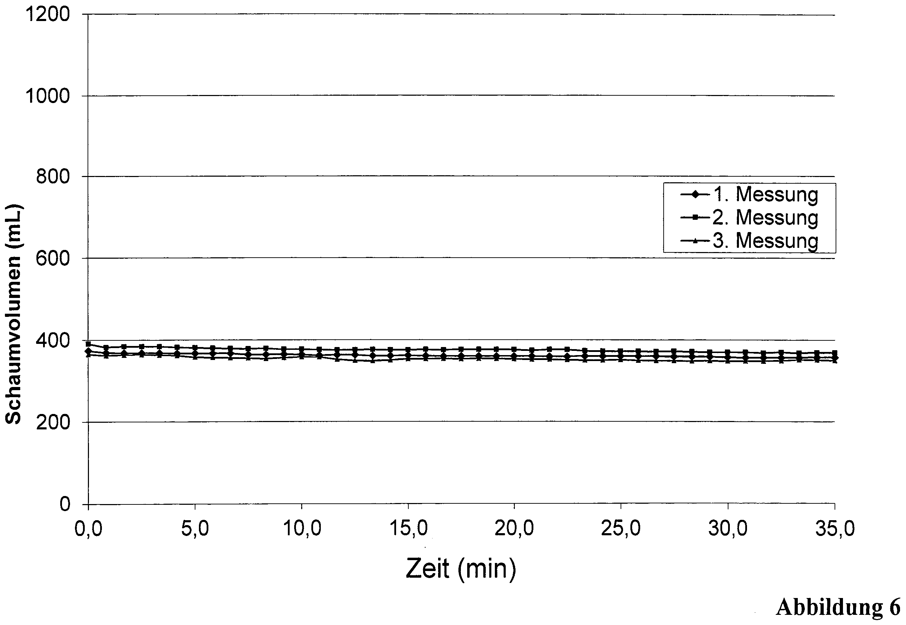

Das Schaumverhalten dieser Zusammensetzung 6 ist in der Abbildung 6 wiedergegeben.

| | **Inhaltsstoff** | **Zusammensetzung in Gew.%** |
|---|---|---|
| 1 | Diclofenac | 2,000 |
| 2 | Propylenglycol | 15.000 |
| 3 | 2-Propanol | 10,250 |
| 4 | Ascorbylpalmitat | 0,020 |
| 5 | phospholipidischer Schaumbildner A | 13,330 |
| 6 | Natriumdihydrogenphosphat-Dihydrat | 0,120 |
| 7 | di-Natriumhydrogenphosphat-Dodecahydrat krist. | 0,660 |
| 8 | EDTA | 0,040 |
| 9 | Wasser reinst | 58,380 |
| 10 | Pfefferminzöl rektifiziert | 0,200 |
| | **TOTAL** | **100.00** |

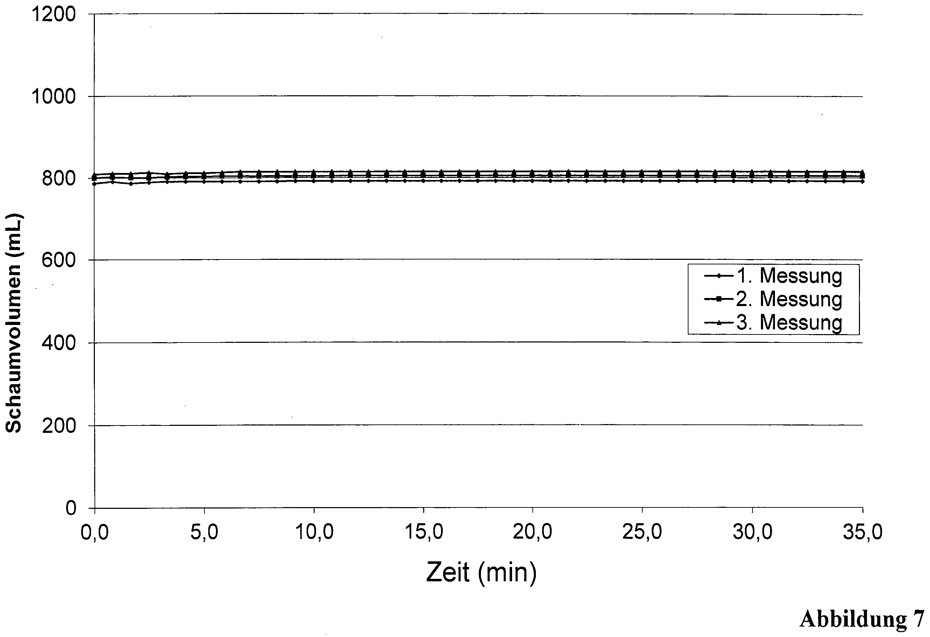

Das Schaumverhalten dieser Zusammensetzung 7 ist in der Abbildung 7 wiedergegeben.

| | **Inhaltsstoff** | **Zusammensetzung in Gew.%** |
|---|---|---|
| 1 | Diclofenac | 8,000 |
| 2 | Propylenglycol | 15.000 |
| 3 | 2-Propanol | 10,250 |
| 4 | Ascorbylpalmitat | 0,020 |
| 5 | phospholipidischer Schaumbildner A | 13,330 |
| 6 | Natriumdihydrogenphosphat-Dihydrat | 0,120 |
| 7 | di-Natriumhydrogenphosphat-Dodecahydrat krist. | 0,660 |
| 8 | EDTA, Titriplex III | 0,040 |
| 9 | Wasser reinst | 52,380 |
| 10 | Pfefferminzöl rektifiziert | 0,200 |
| | **TOTAL** | **100.00** |

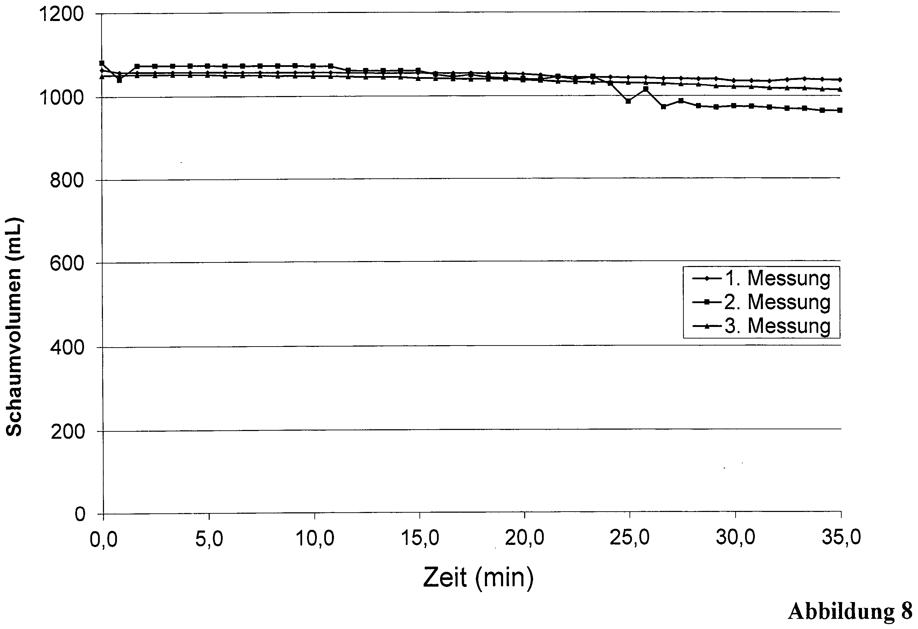

Das Schaumverhalten dieser Zusammensetzung 8 ist in der Abbildung 8 wiedergegeben.

Aufgrund des Vergleichs der Zusammensetzungen 6 bis 8 und der zugehörigen Abbildungen 6 bis 8 ist festzuhalten, daß mit steigender Konzentration des Wirkstoffs das Schaumvolumen bei nahezu gleichbleibender Schaumstabilität zunimmt.

### Ausführungsbeispiele 9 bis 11

Um den Einfluß der Konzentration des phospholipidischen Schaumbildners auf die Schaumbildung zu untersuchen, wurden die nachfolgenden Zusammensetzungen 9 bis 11 hergestellt, die sich bezüglich der Konzentration an phospholipidischem Schaumbildner unterscheiden.

| | **Inhaltsstoff** | **Zusammensetzung in Gew.%** |
|---|---|---|
| 1 | Diclofenac | 4,000 |
| 2 | 1-2-Propandiol | 15,000 |
| 3 | 2-Propanol | 10,250 |
| 4 | L(+)-Ascorbylpalmitat | 0,020 |
| 5 | phospholipidischer Schaumbildner A | 2,000 |
| 6 | Natriumdihydrogenphosphat-Dihydrat reinst | 0,120 |
| 7 | di-Natriumhydrogenphosphat-Dodecahydrat reinst | 0,660 |
| 8 | EDTA | 0,040 |
| 9 | Wasser reinst | 67,710 |
| 10 | Pfefferminzöl rektifiziert | 0,200 |
| | **TOTAL** | **100.00** |

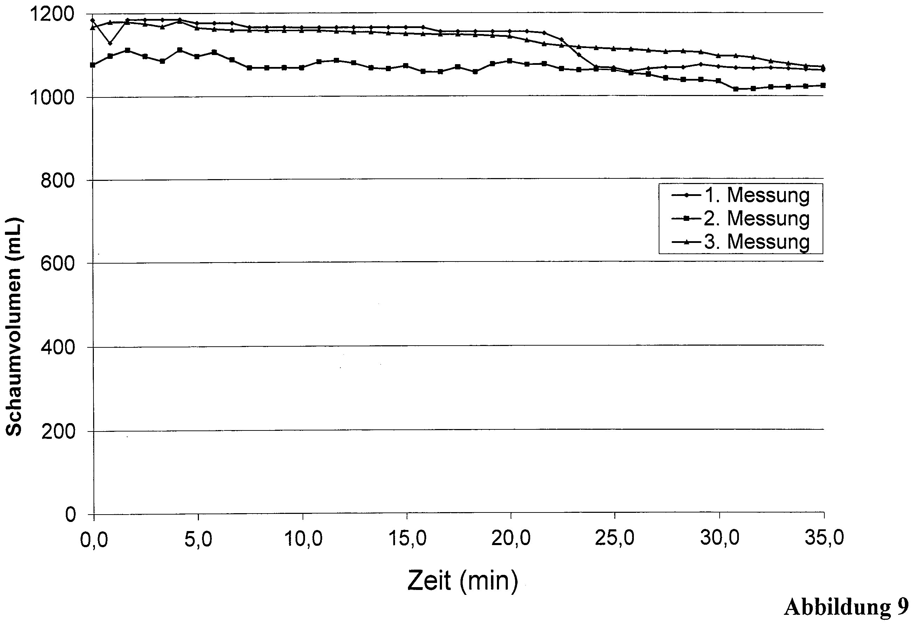

Das Schaumverhalten dieser Zusammensetzung 9 ist in der Abbildung 9 wiedergegeben.

| | **Inhaltsstoff** | **Zusammensetzung in Gew.%** |
|---|---|---|
| 1 | Diclofenac | 4,000 |
| 2 | 1-2-Propandiol | 15,000 |
| 3 | 2-Propanol | 10,250 |
| 4 | L(+)-Ascorbylpalmitat | 0,020 |
| 5 | phospholipidischer Schaumbildner A | 5,000 |
| 6 | Natriumdihydrogenphosphat-Dihydrat reinst | 0,120 |
| 7 | di-Natriumhydrogenphosphat-Dodecahydrat reinst | 0,660 |
| 8 | EDTA | 0,040 |
| 9 | Wasser reinst | 64,710 |
| 10 | Pfefferminzöl rektifiziert | 0,200 |
| | **TOTAL** | **100.00** |

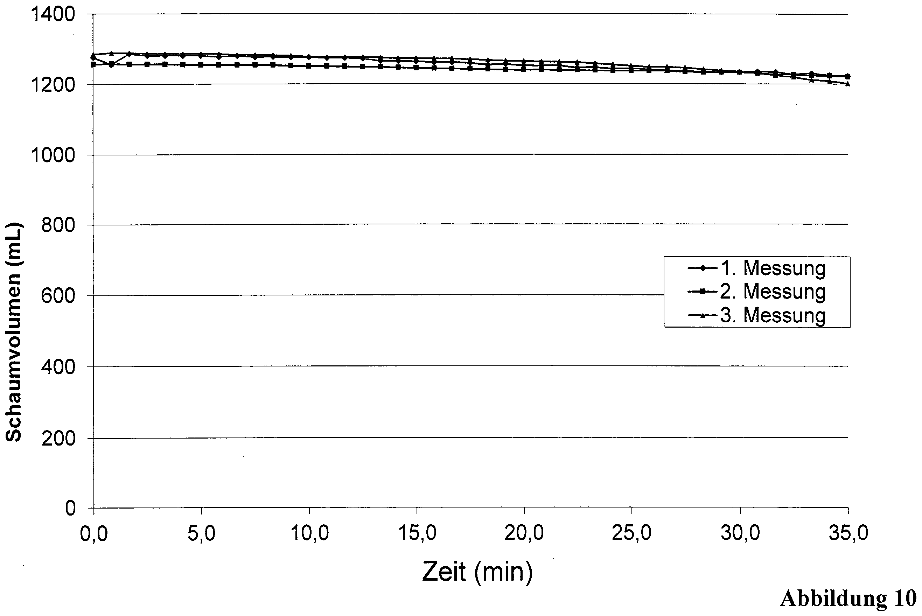

Das Schaumverhalten dieser Zusammensetzung 10 ist in der Abbildung 10 wiedergegeben.

| | **Inhaltsstoff** | **Zusammensetzung in Gew.%** |
|---|---|---|
| 1 | Diclofenac | 4,000 |
| 2 | 1-2-Propandiol | 15,000 |
| 3 | 2-Propanol | 10,250 |
| 4 | L(+)-Ascorbylpalmitat | 0,020 |
| 5 | phospholipidischer Schaumbildner A | 20,000 |
| 6 | Natriumdihydrogenphosphat-Dihydrat reinst | 0,120 |
| 7 | di-Natriumhydrogenphosphat-Dodecahydrat reinst | 0,660 |
| 8 | EDTA, Titriplex III | 0,040 |
| 9 | Wasser reinst | 49,710 |
| 10 | Pfefferminzöl rektifiziert | 0,200 |
| | **TOTAL** | **100.00** |

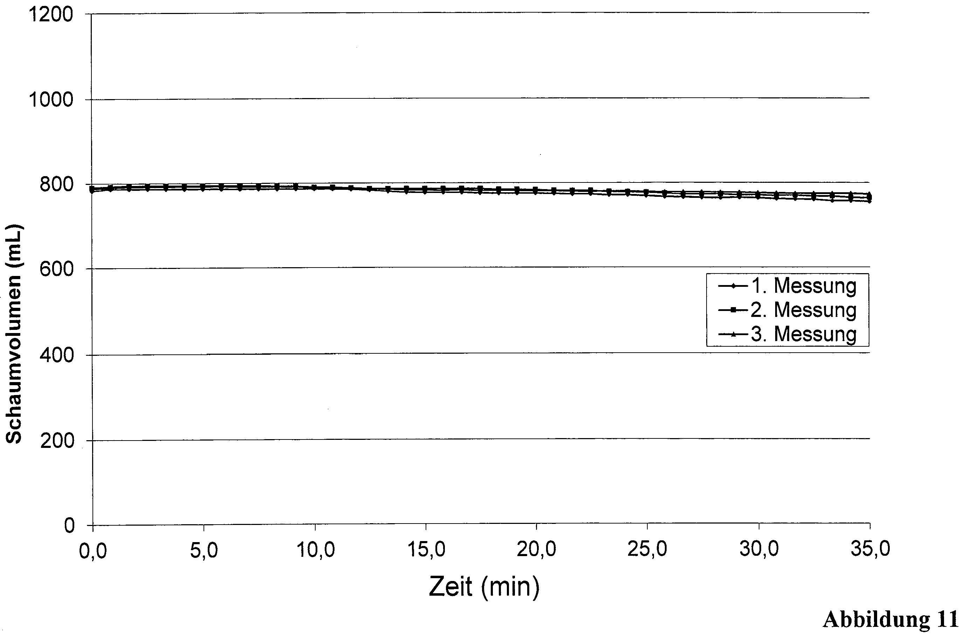

Das Schaumverhalten dieser Zusammensetzung 11 ist in der Abbildung 11 wiedergegeben.

Aufgrund des Vergleichs der Zusammensetzungen 9 bis 11 und der zugehörigen Abbildungen 9 bis 11 ist festzuhalten, daß mit steigender Konzentration des phospholipidischen Schaumbildners das Schaumvolumen bei nahezu gleichbleibender Schaumstabilität abnimmt.

### Ausführungsbeispiele 12 bis 14

Um den Einfluß der Konzentration von Isopropanol auf die Schaumbildung zu untersuchen, wurden die nachfolgenden Zusammensetzungen 12 bis 14 hergestellt, die sich bezüglich der Konzentration an Isopropanol unterscheiden.

| | **Inhaltsstoffe** | **Zusammensetzung in Gew.%** |
|---|---|---|
| 1 | Diclofenac | 4,000 |
| 2 | Propylenglycol | 15.000 |
| 3 | 2-Propanol | 5,000 |
| 4 | Ascorbylpalmitat | 0,020 |
| 5 | phospholipidischer Schaumbildner A | 13,330 |
| 6 | Natriumdihydrogenphosphat-Dihydrat | 0,120 |
| 7 | di-Natriumhydrogenphosphat-Dodecahydrat krist. | 0,660 |
| 8 | EDTA | 0,040 |
| 9 | Wasser reinst | 61,630 |
| 10 | Pfefferminzöl rektifiziert | 0,200 |
| | **TOTAL** | **100.00** |

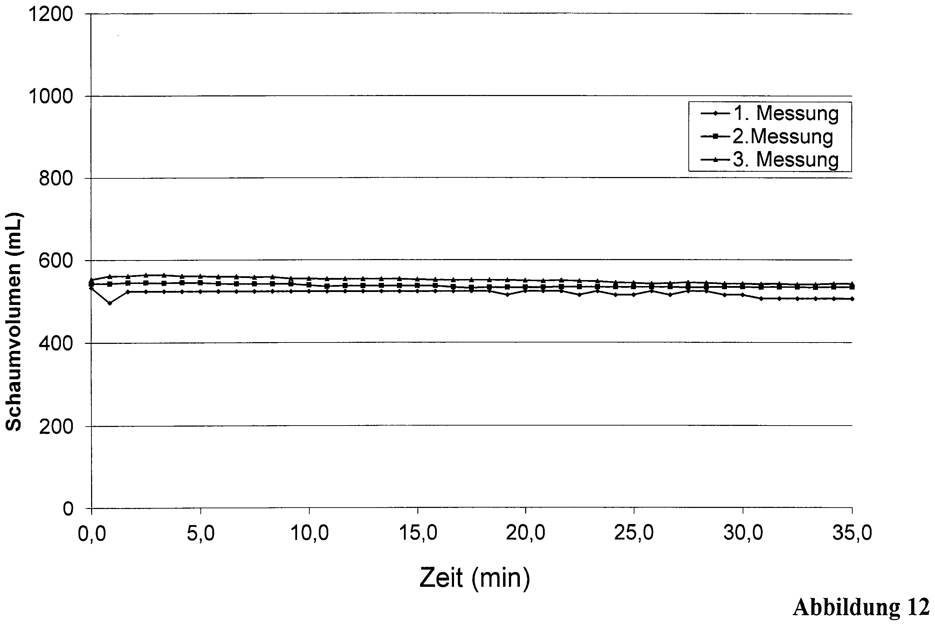

Das Schaumverhalten dieser Zusammensetzung 12 ist in der Abbildung 12 wiedergegeben.

| | **Inhaltsstoff** | **Zusammensetzung in Gew.%** |
|---|---|---|
| 1 | Diclofenac | 4,000 |
| 2 | Propylenglycol | 15.000 |
| 3 | 2-Propanol | 10,000 |
| 4 | Ascorbylpalmitat | 0,020 |
| 5 | phospholipidischer Schaumbildner A | 13,330 |
| 6 | Natriumdihydrogenphosphat-Dihydrat | 0,120 |
| 7 | di-Natriumhydrogenphosphat-Dodecahydrat krist. | 0,660 |
| 8 | EDTA | 0,040 |
| 9 | Wasser reinst | 56,630 |
| 10 | Pfefferminzöl rektifiziert | 0,200 |
| | **TOTAL** | **100.00** |

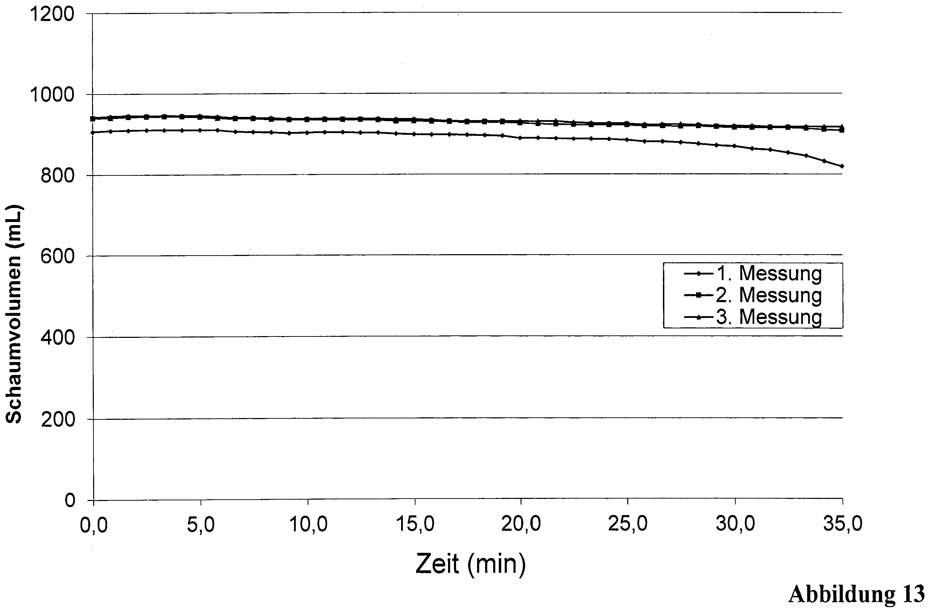

Das Schaumverhalten dieser Zusammensetzung 13 ist in der Abbildung 13 wiedergegeben.

| | **Inhaltsstoff** | **Zusammensetzung in %** |
|---|---|---|
| 1 | Diclofenac | 4,000 |
| 2 | Propylenglycol | 15.000 |
| 3 | 2-Propanol | 20,000 |
| 4 | Ascorbylpalmitat | 0,020 |
| 5 | phospholipidischer Schaumbildner A | 13,330 |
| 6 | Natriumdihydrogenphosphat-Dihydrat | 0,120 |
| 7 | di-Natriumhydrogenphosphat-Dodecahydrat krist. | 0,660 |
| 8 | EDTA | 0,040 |
| 9 | Wasser reinst | 46,630 |
| 10 | Pfefferminzöl rektifiziert | 0,200 |
| | **TOTAL** | **100.00** |

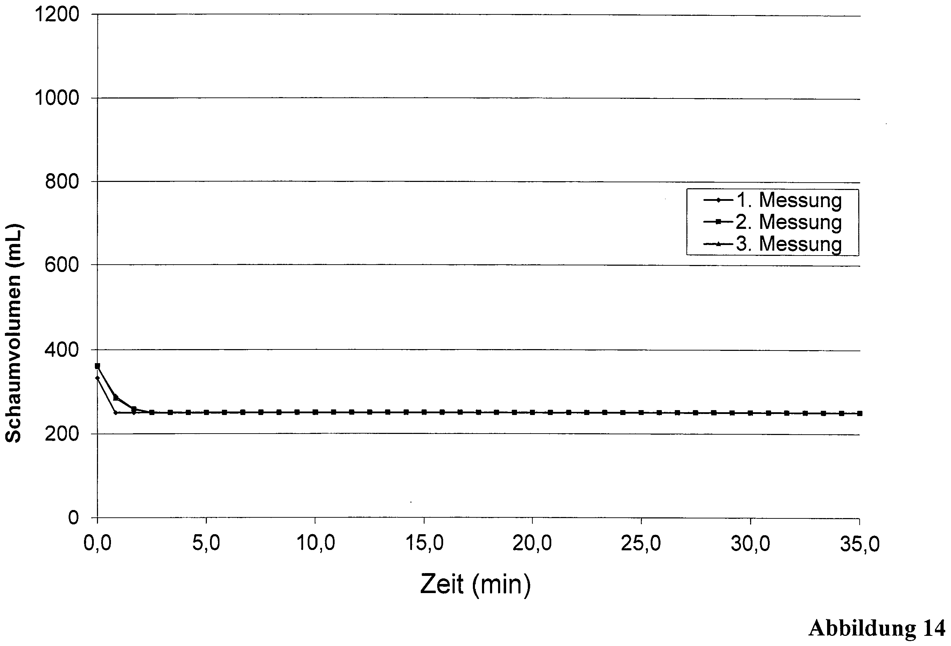

Das Schaumverhalten dieser Zusammensetzung 14 ist in der Abbildung 14 wiedergegeben.

Aufgrund des Vergleichs der Zusammensetzungen 12 bis 14 und der zugehörigen Abbildungen 12 bis 14 ist festzuhalten, daß abhängig von der Konzentration des Isopropanols das Schaumvolumen mit steigender Konzentration des Isopropanols von 5 Gew.% auf 10 Gew.% zunimmt und hiernach im Bereich von 10 Gew.% bis 20 Gew.% bei wieder abnimmt, so daß bei einer Konzentration von 20 Gew.% Isopropanol kein stabiler Schaum ausgebildet wird. Das in Abbildung 14 anfänglich gezeigte geringe Schaumvolumen dürfte zu vernachlässigen sein.

### Ausführungsbeispiele 15 bis 17

Um den Einfluß der Konzentration von Propylenglykol auf die Schaumbildung zu untersuchen, wurden die nachfolgenden Zusammensetzungen 15 bis 17 hergestellt, die sich bezüglich der Konzentration an Propylenglykol unterscheiden.

| | **Inhaltsstoffe** | **Zusammensetzung in Gew.%** |
|---|---|---|
| 1 | Diclofenac | 4,000 |
| 2 | 1-2-Propandiol | 5,000 |
| 3 | 2-Propanol | 10,250 |
| 4 | L(+)-Ascorbylpalmitat | 0,020 |
| 5 | phospholipidischer Schaumbildner A | 13,330 |
| 6 | Natriumdihydrogenphosphat-Dihydrat reinst | 0,120 |
| 7 | di-Natriumhydrogenphosphat-Dodecahydrat reinst | 0,660 |
| 8 | EDTA | 0,040 |
| 9 | Wasser reinst | 66,380 |
| 10 | Pfefferminzöl rektifiziert | 0,200 |
| | **TOTAL** | **100.00** |

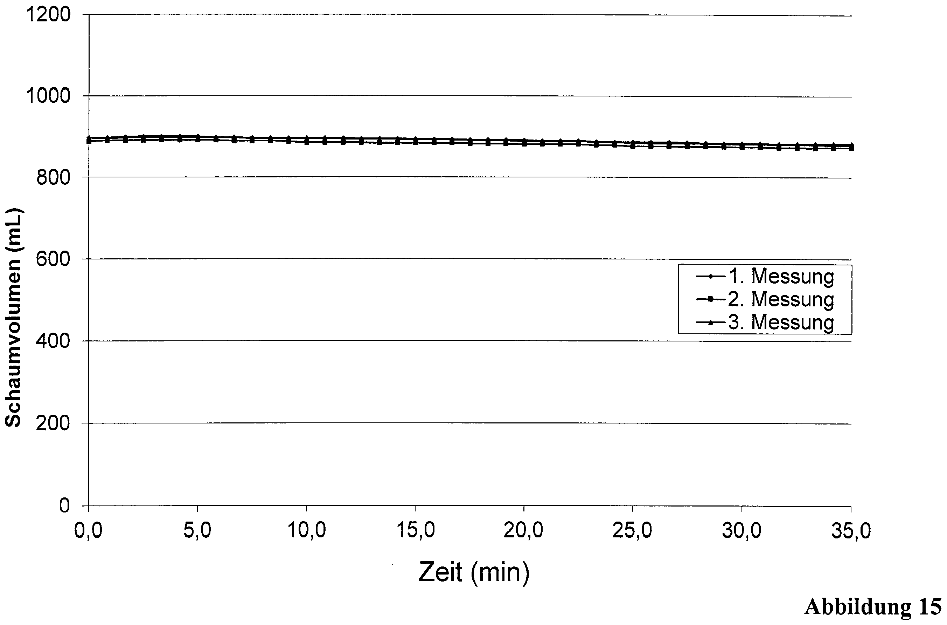

Das Schaumverhalten dieser Zusammensetzung 15 ist in der Abbildung 15 wiedergegeben.

| | **Inhaltsstoff** | **Zusammensetzung in Gew.%** |
|---|---|---|
| 1 | Diclofenac | 4,000 |
| 2 | 1-2-Propandiol | 10,000 |
| 3 | 2-Propanol | 10,250 |
| 4 | L(+)-Ascorbylpalmitat | 0,020 |
| 5 | phospholipidischer Schaumbildner A | 13,330 |
| 6 | Natriumdihydrogenphosphat-Dihydrat reinst | 0,120 |
| 7 | di-Natriumhydrogenphosphat-Dodecahydrat reinst | 0,660 |
| 8 | EDTA | 0,040 |
| 9 | Wasser reinst | 61,380 |
| 10 | Pfefferminzöl rektifiziert | 0,200 |
| | **TOTAL** | **100.00** |

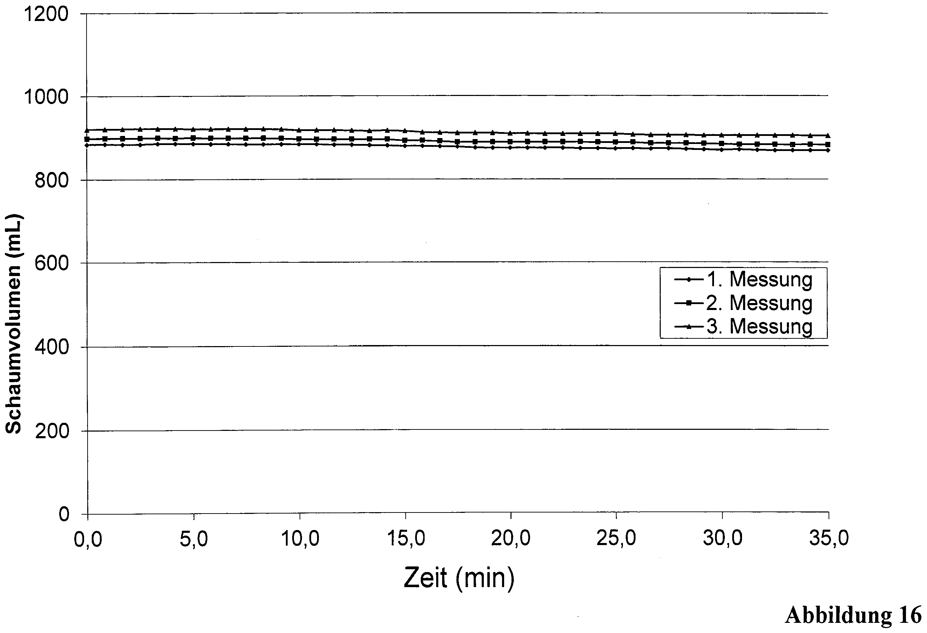

Das Schaumverhalten dieser Zusammensetzung 16 ist in der Abbildung 16 wiedergegeben.

| | **Inhaltsstoff** | **Zusammensetzung in Gew.%** |
|---|---|---|
| 1 | Diclofenac | 4,000 |
| 2 | 1-2-Propandiol | 20,000 |
| 3 | 2-Propanol | 10,250 |
| 4 | L(+)-Ascorbylpalmitat | 0,020 |
| 5 | phospholipidischer Schaumbildner A | 13,330 |
| 6 | Natriumdihydrogenphosphat-Dihydrat reinst | 0,120 |
| 7 | di-Natriumhydrogenphosphat-Dodecahydrat reinst | 0,660 |
| 8 | EDTA | 0,040 |
| 9 | Wasser reinst | 51,380 |
| 10 | Pfefferminzöl rektifiziert | 0,200 |
| | **TOTAL** | **100.00** |

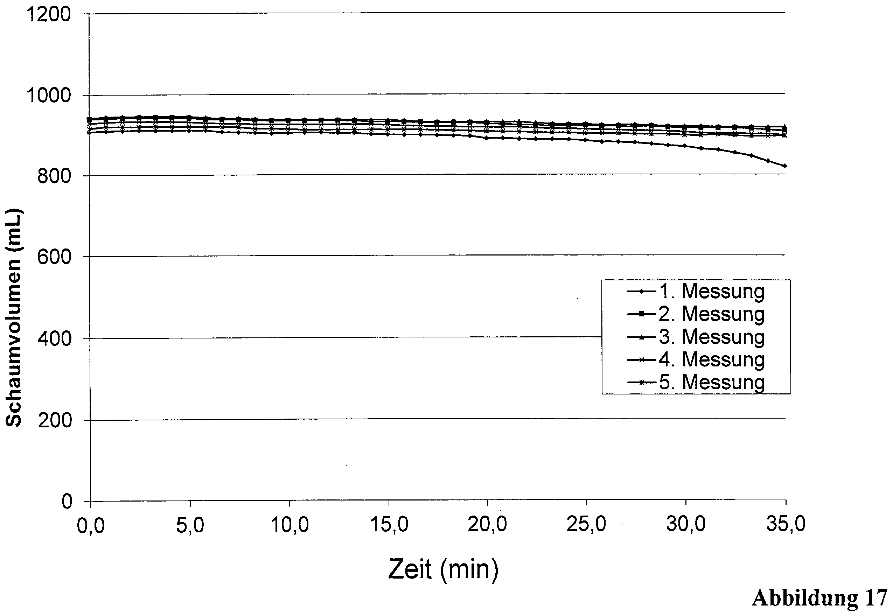

Das Schaumverhalten dieser Zusammensetzung 17 ist in der Abbildung 17 wiedergegeben.

Aufgrund des Vergleichs der Zusammensetzungen 15 bis 17 und der zugehörigen Abbildungen 15 bis 17 ist festzuhalten, daß die Konzentration des Propylenglykols keinen oder allenfalls nur einen sehr geringen Einfluß auf das Schaumvolumen und die Schaumstabilität hat.

### Ausführungsbeispiel 18

In einer Vergleichsuntersuchung an 14 Probanden (8 weiblich, 6 männlich) mit ausgeprägter Akne im Gesichtsbereich, wurden die Probanden zweimal täglich (morgens und abends) mit einem Schaum behandelt, der aus einer Zusammensetzung erstellt worden ist, deren Inhaltsstoffe in der nachfolgenden Tabelle gemäß Zusammensetzung 18 quantifiziert wird.

| | **Inhaltsstoff** | **Zusammensetzung in Gew.%** |
|---|---|---|
| 1 | Erythromycin | 1.50 |
| 2 | Propylenglycol | 15.00 |
| 3 | 2-Propanol | 9.35 |
| 4 | phospholipidischer Schaumbildner B | 8.00 |
| 5 | Natriumdihydrogenphosphat-Dihydrat | 0.50 |
| 6 | di-Natriumhydrogenphosphat-Dodecahydrat krist. | 1.14 |
| 7 | Natrium Hydroxid 10% w/w | 1.00 |
| 8 | Tegosoft GC | 7.70 |
| 9 | Wasser reinst | 55.81 |
| | **TOTAL** | **100.00** |

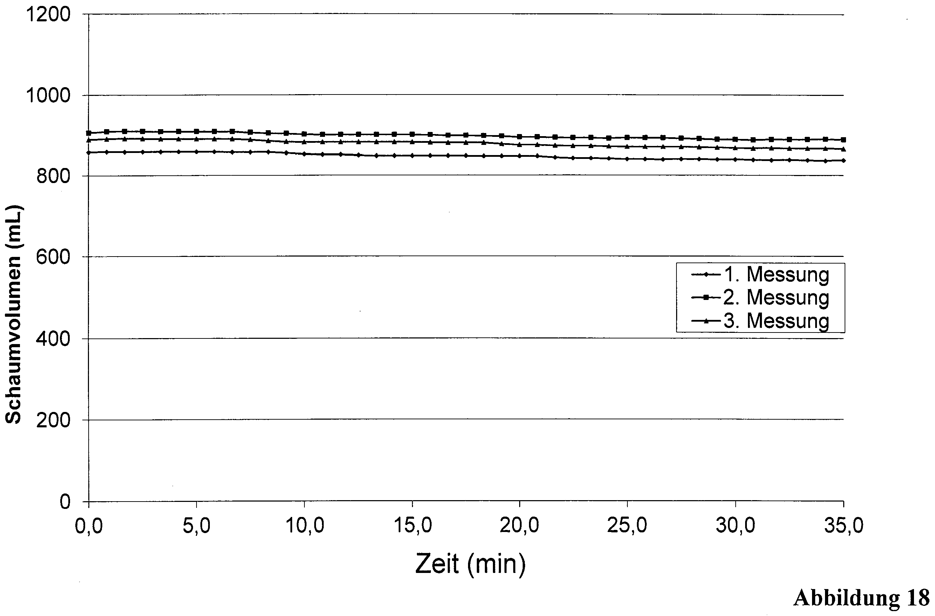

Das Schaumverhalten dieser Zusammensetzung 18 ist in der Abbildung 18 wiedergegeben.

Die Behandlung erfolgte pro Anwendung mit einer definierten Menge von 0,4 mg Schaum pro Gesichtshälfte. Der Schaum wurde direkt auf die zu behandelnde Fläche appliziert und durch Kreisbewegungen mit 2 Fingern einmassiert. Die Therapiezeit betrug insgesamt 60 Tage.

Jeweils die linke Gesichtshälfte eines jeden Probanden wurde mit dem mittels des zuvor beschriebenen SITA-Meßverfahren hergestellten Schaums und die jeweils rechte Gesichtshälfte eines jeden Probanden mittels eines mit einem "M3 Minischäumer" von Rexam/Airspray hergestellten Schaums behandelt, wobei beide Schäume aus der Zusammensetzung 18 erstellt worden sind.

Bereits erste Erfolge konnten für beide Schäume nach 30 Tagen Anwendung festgestellt werden, wobei die Läsionen der linken Gesichtshälfte um ca. 19 % und der rechten Gesichthälfte um ca. 21 % reduziert wurden.

Bei der Endauswertung am 60. Tag der Untersuchung wurde für die linke Gesichtshälfte eine Abnahme der Läsionen um ca. 36 % und für die rechte Gesichtshälfte eine Abnahme von ca. 34 % festgestellt. Einen signifikanten Unterschied zwischen den beiden Gesichtshälften konnte nicht festgestellt werden. Keiner der Probanden empfand die Behandlung als unangenehm oder berichtete über schmerzhafte Reizungen der behandelten Flächen. Ebensowenig konnten Unterschiede in der Verträglichkeit der Behandlung zwischen den beiden Gesichtshälften festgestellt werden.

### Ausführungsbeispiel 19

In einer doppelblinden, randomisierten und Placebo kontrollierten Vergleichsuntersuchung an 12 Probanden (5 weiblich, 7 männlich) wurde die Wirksamkeit
a) der vorstehend beschriebenen Zusammensetzung 10, die 4 % Diclofenac-Natrium enthielt,
b) der vorstehend beschriebenen Zusammensetzung 1, die 10 % Ketoprofen enthielt, und
c) die Wirksamkeit einer Ketoprofen-freien Zusammensetzung, die bezüglich der Inhaltsstoffe 2 bis 9 identisch war mit der Zusammensetzung 1, wobei diese Ketoprofen-freie Zusammensetzung kein Ketoprofen und stattdessen 10 Gew.% mehr Wasser als die Zusammensetzung 1 enthielt,
bei der Behandlung eines künstlich induzierten UV-Erythems untersucht. Alle eingesetzten Schäume wurden jeweils mittels des vorbeschriebenen SITA-Meßverfahren als auch dem "M3 Minischäumer" von Rexam/Airspray auf Basis der gleichen Ausgangszusammensetzungen erzeugt.

Die künstlichen UV-Erytheme wurden an 16 Testfeldern (je 2 x 2 cm) auf dem Rücken, jeweils 4 Testfelder links und rechts der Wirbelsäule sowie jeweils 4 Testfelder im oberen und unteren Bereich des Rückens erzeugt. Die 8 oberen Testfelder wurden mit einer UV-Dosis von 1.5 x MED und die unteren Testfelder mit 2.5 x MED bestrahlt.

Nach der UV-Bestrahlung wurden ECG-Ringe mit einem Innendurchmesser von 16 mm in die Zentren der UV-bestrahlten Testfelder geklebt. Die unbehandelten Felder wurden ebenfalls mit ECG-Ringen markiert. Die Distanz zwischen den Testfeldern betrug ca. 3 cm.

Anschließend wurde ca. 10-15 Minuten nach Beendigung der UV-Bestrahlung eine Dosis von jeweils 25 µg Schaum, gem. Randomliste, in die dafür vorgesehenen ECG-Ringe appliziert und mittels eines Rundspatels gleichmässig verteilt.

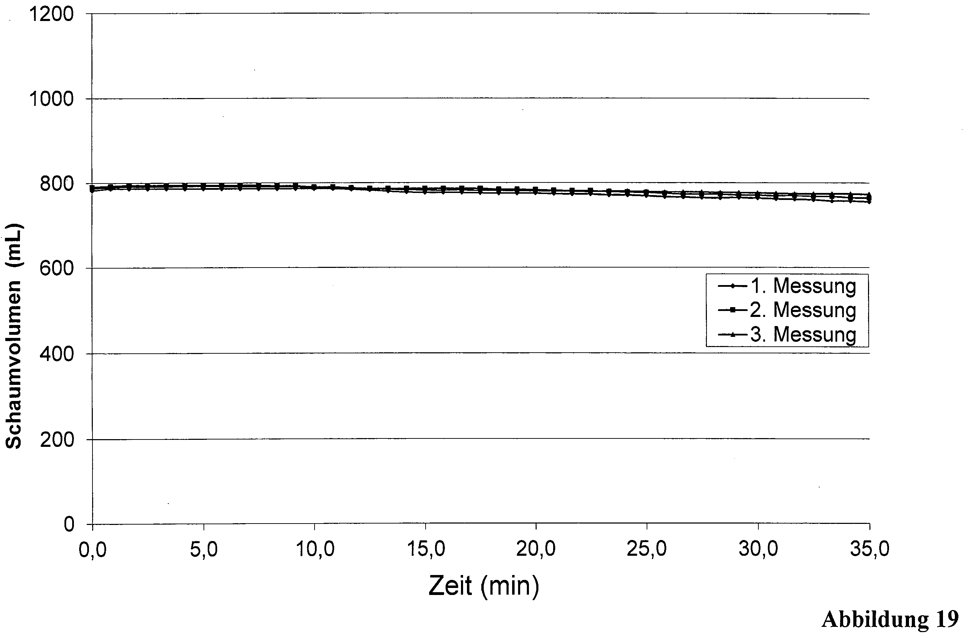

Das Schaumverhalten der Zusammensetzung 19 c) (Ketoprofen-freie Zusammensetzung) ist in der Abbildung 19 wiedergegeben.

Die Beurteilung der Unterschiede wurde durch die optische Prüfung durch einen Dermatologen vorgenommen. Sie erfolgte auf der Basis der international anerkannten, visuellen Bewertungsmethode von 0 = kein sichtbares Erythem bis 4 = intensives Erythem für die unbehandelte Fläche und nach der Bewertung -1 = intensive Erythem bis 3 = komplett unterdrücktes Erythem für die bestrahlten und behandelten Flächen. Kontrollzeitpunkte waren nach jeweils 2, 3, 4, 5, 6 und 8 Stunden am selben Tag nach der Anwendung.

Zwischen den nach dem SITA-Meßverfahren und den mittels "M3 Minischäumer" erzeugten Schäumen wurde nur bei einem Messzeitpunkt, 6 Stunden, ein geringfügiger Unterschied bei dem Wirkstoff Ketoprofen festgestellt. Dieser Unterschied war nicht signifikant. Hier wurde bei dem Schaum, der nach dem SITA-Meßverfahren erzeugt wurde, ein Erythem-Wert von 2 und bei dem Schaum, der mittels des "M3 Minischäumers" erzeugt wurde, ein Wert von 1 festgestellt. Weitere Differenzen zwischen den unterschiedlich erzeugten, wirkstoffhaltigen Schäumen konnten nicht festgestellt werden.

Im Vergleich zu der Ketoprofen-freien Zusammensetzung (c)) und den nicht behandelten Testfeldern haben die wirkstoffhaltigen Schäume bei der Abschlußmessung nach 8 Stunden deutliche Unterschiede bei 1.5 MED und 2.5 MED gezeigt.

Insbesondere bei 2.5 MED wurde bei dem Diclofenac-haltigen Schaum ein Wert von 1 (leichte Unterdrückung des Erythems, gut identifizierbar), bei dem Ketoprofen-haltigen Schaum ein Wert von 2 (eindeutige Unterdrückung des Erythems aber noch sichtbar) und bei dem Ketoprofenfrein Schaum ein Wert von -1 (stärkere Ausprägung des Erythems) festgestellt. Der Schaum, der aus der Ketoprofen-haltigen Zusammensetzung hergestellt ist, zeigt eine klare therapeutische Überlegenheit gegenüber dem Diclofenac-haltigen Schaum, dem Ketoprofen-freien Schaum und den nicht behandelten Testfeldern.

Alle wirkstoffhaltigen Schäume zeigten eine gute Verträglichkeit. Lediglich bei dem Ketoprofen-freie Schaum wurden 3 Nebenwirkungen festgestellt.

Der vorstehend bei den Ausführungsbeispielen 1 und 2 sowie 5 bis 17 verwendete phospholidischer Schaumbildner A weist folgende Zusammensetzung auf, wobei die nachfolgenden Werte sich auf die Trockensubstanz beziehen:

| | |
|---|---|
| Phosphatidylcholin | 80 Gew.% ± 10 Gew.% |
| Lysophosphatidylcholin | 3 Gew. % ± 3 Gew.% |
| Phosphatidsäure | ≤ 8 Gew.% |
| Phosphatidylethanolamin | ≤ 4 Gew.% |
| sonstige ölartige Bestandteile | max. 6 Gew.% |
| Säurezahl | 2 |
| Peroxidzahl | 6 |

Der vorstehend bei dem Ausführungsbeispiel 3 und 18 verwendete phospholidischer Schaumbildner B weist folgende Zusammensetzung auf, wobei die nachfolgenden Werte sich auf die Trockensubstanz beziehen:

| | |
|---|---|
| Phosphatidylcholin | 85 Gew.% ± 10 Gew.% |
| Lysophosphatidylcholin | 3 Gew. % ± 3 Gew.% |
| Tocopherol | max. 0,3 Gew.% |
| Säurezahl | 1 |
| Peroxidzahl | 5 |

Die vorstehend aufgeführte Peroxidzahl gibt die Milliäquivalente des Sauerstoffs an, die in 1.000 g einer Probe (Trockensubstanz) enthalten sind. Dieser Wert wird nach Umsetzung der Probe mit Kaliumjodid in einer Mischung aus Chloroform und Essigsäure dadurch bestimmt, daß das auf diese Weise erzeugte Jod titrimetrisch mit Natriumthiosulfat potentiometrisch bestimmt wird.

Die Säurezahl gibt an, wieviel mg Kaliumhydroxid erforderlich sind, um die freien, nicht veresterten Fettsäuren, die in 1 g phospholipidischen Schaumbildner (Trockensubstanz) enthalten sind, benötigt werden. Dieser Wert wird durch Titration einer entsprechenden gelösten Probe mit Kaliumhydroxidlösung unter Verwendung von Phenolphthalein als Indikator ermittelt.

## Patentansprüche

1. Verfahren zur Entwicklung einer als Schaum auf die Haut zu applizierenden flüssigen pharmazeutischen Zusammensetzung, wobei die aufschäumbare, flüssige Zusammensetzung mindestens ein Lösungsmittel, mindestens einen pharmazeutischen Wirkstoff sowie mindestens einen phospholipidischen Schaumbildner aufweist, **dadurch gekennzeichnet, daß**
a) der phospholipidische Schaumbildner ein aus Sojabohnen isolierter phospholipidischer Schaumbildner ist, der Phosphatidylcholin in einer Konzentration zwischen 50 Gew.% und 95 Gew.% enthält, wobei das Phosphatidylcholin eine Säurezahl von maximal 10, eine Peroxidzahl von maximal 10 und eine Ölkonzentration von maximal 6 Gew.%, bezogen auf das Trockengewicht des Phosphatidylcholins, aufweist, daß
b) das Schaumvolumen und die Schaumstabilität des nach dem bei den Ausführungsbeispielen beschriebenen SITA-Meßverfahren ohne Verwendung eines Treibmittels erzeugten Schaumes bestimmt werden, wobei bei dem SITA-Meßverfahren 250 ml der flüssigen Zusammensetzung bei einer Rotordrehzahl von 2.000 U/min durch fünf Rotorzyklen von jeweils 20 Sekunden unter Einhaltung jeweils einer Meßpause von etwa 15 Sekunden zwischen den Rotorzyklen aufgeschäumt werden und das jeweilige Schaumvolumen über Nadeldetektoren alle 50 Sekunden über einen Zeitraum von 35 Minuten erfaßt wird, und daß
c) in der flüssigen Zusammensetzung der mindestens eine Schaumbildner, das mindestens eine Lösungsmittel und der mindestens eine pharmazeutische Wirkstoff von ihrer chemischen Art und/oder ihrer Konzentration her so lange variiert werden, bis der so durch das SITA-Meßverfahren erzeugte Schaum
d) eine Schaumdichte zwischen 0,05 g/ml und 0,8 g/ml besitzt,
e) ein Schaumvolumen zwischen 450 ml und 1.400 ml aufweist und
f) eine solche Schaumstabilität hat, daß der Schaum nach einer Verweilzeit von bis zu fünf Minuten noch mindestens 50 % des Schaumvolumens besitzt, das unmittelbar nach der Erzeugung des Schaumes ursprünglich vorgelegen hat.

2. Verfahren nach Anspruch 1, **dadurch gekennzeichnet, daß** der durch das SITA-Meßverfahren erzeugte Schaum ein Schaumvolumen zwischen 600 ml und 1.200 ml aufweist.

3. Verfahren nach Anspruch 1 oder 2, **dadurch gekennzeichnet, daß** der durch das SITA-Meßverfahren erzeugte Schaum eine solche Schaumstabilität besitzt, daß der Schaum nach einer Verweilzeit von bis zu 10 Minuten noch mindestens zwischen 55 % und 100 % des Schaumvolumens besitzt, das unmittelbar nach der Erzeugung des Schaumes ursprünglich vorgelegen hat.

4. Verfahren nach Anspruch 3, **dadurch gekennzeichnet, daß** der Schaum nach einer Verweilzeit von bis zu 10 Minuten noch mindestens zwischen 85 % und 99 % des Schaumvolumens besitzt, das unmittelbar nach der Erzeugung des Schaumes ursprünglich vorgelegen hat.

5. Verfahren nach einem der vorangehenden Ansprüche, **dadurch gekennzeichnet, daß** eine Korrelation zwischen dem nach dem SITA-Meßverfahren spezifizierten Schaum und den pharmazeutischen Eigenschaften hergestellt wird.

6. Verfahren nach einem der vorangehenden Ansprüche, **dadurch gekennzeichnet, daß** ein Schaumapplikator zum mechanischen Aufschäumen der flüssigen Zusammensetzung ausgewählt wird, daß aus der flüssigen Zusammensetzung durch den ausgewählten Schaumapplikator ein Schaum erzeugt wird und daß eine Korrelation zwischen den Eigenschaften, insbesondere der pharmazeutischen Eigenschaften des über den Schaumapplikator erzeugten Schaumes und dem über das SITA-Meßverfahren ermittelten Schaumvolumen und/oder der Schaumstabilität hergestellt wird.

7. Verfahren nach einem der vorangehenden Ansprüche, **dadurch gekennzeichnet, daß** aus der flüssigen Zusammensetzung durch das SITA-Meßverfahren ein solcher Schaum erzeugt wird, der Schaumdichten zwischen 0,15 g/ml und 0,4 g/ml besitzt.

8. Verfahren nach einem der vorangehenden Ansprüche, **dadurch gekennzeichnet, daß** das Lösungsmittel für die Herstellung der flüssigen Zusammensetzung aus der Gruppe ausgewählt wird, die Wasser, mindestens einen Alkohol, insbesondere mindestens einen 1- bis 3-wertigen Alkohol, mindestens einen Polyalkohol und Mischungen der zuvor genannten Lösungsmittel umfaßt.

9. Verfahren nach einem der vorangehenden Ansprüche, **dadurch gekennzeichnet, daß** zur Herstellung der flüssigen Zusammensetzung desweiteren ein Komplexbildner, ein Puffer, ein Verdickungsmittel, ein Antioxidationsmittel und/oder einen Stabilisator vorgesehen wird.

10. Topisch applizierbare Zusammensetzung mit mindestens einem systemisch und/oder lokal wirkenden pharmazeutischen Wirkstoff, wobei die Zusammensetzung neben dem einen pharmazeutischen Wirkstoff desweiteren mindestens ein Lösungsmittel sowie mindestens einen phospholipidischen Schaumbildner enthält und eine solche flüssige Konsistenz aufweist, daß sie während der Applikation einen Schaum ausbildet, **dadurch gekennzeichnet, daß**
a) der phospholipidische Schaumbildner ein aus Sojabohnen isolierter phospholipidischer Schaumbildner ist, der Phosphatidylcholin in einer Konzentration zwischen 50 Gew.% und 95 Gew.% enthält, wobei das Phosphatidylcholin eine Säurezahl von maximal 10, eine Peroxidzahl von maximal 10 und eine Ölkonzentration von maximal 6 Gew.%, bezogen auf das Trockengewicht des Phosphatidylcholins, aufweist,
b) daß der mindestens eine phospholipidische Schaumbildner, das mindestens eine Lösungsmittel und der mindestens eine Wirkstoff von ihrer chemischen Art und/oder ihrer Konzentration her derart aufeinander abgestimmt sind, daß die Zusammensetzung ohne Verwendung eines zusätzlichen Treibmittels mechanisch aufschäumbar ist, und daß der so erzeugte Schaum
c) eine Schaumdichte zwischen 0,05 g/ml und 0,8 g/ml besitzt,
d) ein Schaumvolumen zwischen 450 ml und 1.400 ml aufweist und
e) eine solche Schaumstabilität hat, daß der Schaum nach einer Verweilzeit von bis zu fünf Minuten noch mindestens 50 % des Schaumvolumens besitzt, das unmittelbar nach der Erzeugung des Schaumes ursprünglich vorgelegen hat, wobei sowohl das Schaumvolumen als auch die Schaumstabilität nach dem bei den Ausführungsbeispielen beschriebenen SITA-Meßverfahren bestimmt sind und wobei das SITA-Meßverfahren 250 ml der flüssigen Zusammensetzung bei einer Rotordrehzahl von 2.000 U/min durch fünf Rotorzyklen von jeweils 20 Sekunden unter Einhaltung jeweils einer Meßpause von etwa 15 Sekunden zwischen den Rotorzyklen aufschäumt und das jeweilige Schaumvolumen über Nadeldetektoren alle 50 Sekunden über einen Zeitraum von 35 Minuten erfaßt.

11. Zusammensetzung nach Anspruch 10, **dadurch gekennzeichnet, daß** der durch das SITA-Meßverfahren erzeugte Schaum ein Schaumvolumen zwischen 600 ml und 1.200 ml besitzt.

12. Zusammensetzung nach Anspruch 10 oder 11, **dadurch gekennzeichnet, daß** der durch das SITA-Meßverfahren erzeugte Schaum eine solche Schaumstabilität besitzt, daß der Schaum nach einer Verweilzeit von bis zu 10 Minuten noch mindestens zwischen 55 % und 100 % des Schaumvolumens besitzt, das unmittelbar nach der Erzeugung des Schaumes ursprünglich vorgelegen hat.

13. Zusammensetzung nach Anspruch 12, **dadurch gekennzeichnet, daß** der Schaum nach einer Verweilzeit von bis zu 10 Minuten noch mindestens zwischen 85 % und 99 % des Schaumvolumens besitzt, das unmittelbar nach der Erzeugung des Schaumes ursprünglich vorgelegen hat.

14. Zusammensetzung nach Anspruch 10, **dadurch gekennzeichnet, daß** der aus der Zusammensetzung durch das SITA-Meßverfahren erzeugte Schaum eine Schaumdichte zwischen 0,15 g/ml und 0,4 g/ml besitzt.

15. Zusammensetzung nach einem der Ansprüche 10 bis 14, **dadurch gekennzeichnet, daß** die Zusammensetzung als Lösungsmittel Wasser, mindestens einen Alkohol, insbesondere mindestens einen 1- bis 3-wertigen Alkohol, und/oder mindestens einen Polyalkohol enthält.

16. Zusammensetzung nach einem der Ansprüche 10 bis 15, **dadurch gekennzeichnet, daß** der mindestens eine Wirkstoff ein Wirkstoff zur Anwendung bei Mensch oder Tier ist und aus der Gruppe ausgewählt ist, die Lokalanästhetika, Antiallergika, Dermatika, Wirkstoffe gegen grippale Infekte und Erkältungskrankheiten, Wirkstoffe zur Behandlung von Neuro-pathien, Wirkstoffe zur Behandlung von Durchblutungsstörungen, Chemotherapeutika, Chinin, Antimykotika, Antibiotika, Thalidomid, Sero-tonin, Eicosanoide, Analgetika, Antikonvulsiva, Nicht-steroidale Antirheumatika, Leukotriene, Leukotrienhemmer, Androgene, Antiandrogene, Kortikoide, Opiatrezeptor-Antagonisten, Blutgerinnung hemmende Stoffe, Thrombozytenaggregationshemmer, Histaminantagonisten, regulatorisch und enzymatisch wirkende Peptide und Proteine, Nukleinsäuren (einzel- und doppelsträngige DNA, einzel- und doppelsträngige RNA, snRNA, DNA-Oligonukleotide, RNA-Oligonukleotide) und Oligopeptide, Antipruriginosa, Antidiabetika, Prostaglandine, Prostaglandinsynthesehemmer, Antiviral wirkende oder virostatisch wirkende Substanzen, Antimikrobiell-wirkende Substanzen, Wirkstoffe gegen Prione, Immunsupressiva, Hormone, Wirkstoffe zur Behandlung von Warzen oder Wunden, insbesondere chronischen Wunden, Vitamine, Pflanzenextrakte oder Auszüge aus Pflanzenextrakten, Psychopharmaka, den Schlaf beeinflussende Wirkstoffe, Analeptika, Allgemeinanästhetika, Muskelrelaxantien, Antiepileptika, Antiparkinsonmittel, Antiemetika, Antiparasitika, Ganglionär angreifende Substanzen, am Symphatikus angreifende Substanzen, am Parasymphatikus angreifende Substanzen, antibakteriell wirkende Pharmaka, Calciumantagonisten, Herz-/Kreislaufmittel, Antiasthmatika, Antitussiva, Expektorantien, Hepatika, Diuretika, Choleretika, Desinfektionsmittel, Spurenelemente, Antiinfektiva, Zytostatika, Antimetaboliten, Hormonantagonisten, Immunmodulatoren sowie Derivate und Salze der zuvor genannten Wirkstoffe umfaßt.

17. Zusammensetzung nach Anspruch 16, **dadurch gekennzeichnet, daß** das mindestens eine Analgetikum in einer Konzentration zwischen 0,1 Gew.% und 20 Gew.%, vorzugsweise in einer Konzentration zwischen 2 Gew.% und 10 Gew.%, in der Zusammensetzung enthalten ist.

18. Zusammensetzung nach Anspruch 16 oder 17, **dadurch gekennzeichnet, daß** die Zusammensetzung das mindestens eine Antimykotikum in einer Konzentration zwischen 0,01 Gew.% und 10 Gew.%, vorzugsweise in einer Konzentration zwischen 0,2 Gew.% und 5 Gew.%, in der Zusammensetzung enthalten ist.

19. Zusammensetzung nach einem der Ansprüche 16 bis 18, **dadurch gekennzeichnet, daß** die Zusammensetzung den mindestens einen kortikoiden Wirkstoff in einer Konzentration zwischen 0,001 Gew.% und 3 Gew.%, vorzugsweise zwischen 0,1 Gew.% und 0,8 Gew.%, aufweist.

20. Zusammensetzung nach Anspruch 16 bis 19, **dadurch gekennzeichnet, daß** die Zusammensetzung das mindestens eine Lokalanästhetikum in einer Konzentration zwischen 3 Gew.% und 15 Gew.%, insbesondere zwischen 6 Gew.% und 12 Gew.%, aufweist.

21. Zusammensetzung nach einem der Ansprüche 10 bis 20, **dadurch gekennzeichnet, daß** die Zusammensetzung den mindestens einen Immunmodulator in einer Konzentration zwischen 0,03 Gew.% und 0,1 Gew.% enthält.

22. Zusammensetzung nach Anspruch 10, **dadurch gekennzeichnet, daß** in dem phospholipidischen Schaumbildner maximal 15 Gew.% Lyso-Phosphatidylcholin, maximal 10 Gew.% Phosphatidsäure und maximal 10 Gew.% Phosphatidylethanolamin enthalten sind.

23. Zusammensetzung nach einem der Ansprüche 10 bis 22, **dadurch gekennzeichnet, daß** die Zusammensetzung den phospholipidischen Schaumbildner in einer Konzentration zwischen 2 Gew.% und 25 Gew.%, vorzugsweise in einer Konzentration zwischen 4 Gew.% und 15 Gew.%, aufweist.

24. Zusammensetzung nach Anspruch 15, **dadurch gekennzeichnet, daß** die Zusammensetzung als Alkohol Propylenglykol in einer Konzentration zwischen 2 Gew.% und 25 Gew.%, vorzugsweise in einer Konzentration zwischen 5 Gew.% und 15 Gew.%, enthält.

25. Verwendung einer topisch als Schaum auf die Haut eines warmblütigen Säugetieres zu applizierenden und ohne Treibmittel aufschäumbaren flüssigen Zusammensetzung nach einem der Ansprüche 10 bis 24 zur Herstellung eines Arzneimittels zur Behandlung von atopischem Ekzem oder Neurodermitis, wobei das Arzneimittel den Immunmodulator Tacrolimus in einer Konzentration zwischen 0,03 Gew.% und 0,1 Gew.% enthält.

26. Verwendung einer topisch als Schaum auf die Haut eines warmblütigen Säugetieres zu applizierenden und ohne Treibmittel aufschäumbaren flüssigen Zusammensetzung nach einem der Ansprüche 10 bis 24 zur Herstellung eines Arzneimittels zur Behandlung von entzündlichen oder juckenden Hauterkrankungen, Psoriasis, Dermatitis, Neurodermitis oder Schuppenflechte, wobei das Arzneimittel ein Glucocorticoid, insbesondere Betamethason, Dexamethason, Prednicarbat, Mometasonfuroat und/oder Clobetasonbutyrat, in einer Konzentration zwischen 0,01 Gew.% und 0,4 Gew.% enthält.

27. Verwendung einer topisch als Schaum auf die Haut eines warmblütigen Säugetieres zu applizierenden und ohne Treibmittel aufschäumbaren flüssigen Zusammensetzung nach einem der Ansprüche 10 bis 24 zur Herstellung eines Arzneimittels zur Behandlung von Schmerzen, Entzündungen, rheumatischen Erkrankungen oder akuten Traumen, wobei das Arzneimittel ein Analgetikum, insbesondere Diclofenac, Ketoprofen und/oder Ibuprofen, in einer Konzentration zwischen 0,1 Gew.% und 20 Gew.%, vorzugsweise in einer Konzentration zwischen 2 Gew.% und 10 Gew.%, enthält.

28. Verwendung einer topisch als Schaum auf die Haut und/oder die Nägel eines warmblütigen Säugetieres zu applizierenden und ohne Treibmittel aufschäumbaren flüssigen Zusammensetzung nach einem der Ansprüche 10 bis 24 zur Herstellung eines Arzneimittels zur Behandlung von mykotischen Infektionen, wobei das Arzneimittel ein Antimykotikum, insbesondere Bifonazol und/oder Terbinafin, in einer Konzentration zwischen 0,5 Gew.% und 10 Gew.% enthält.

29. Verwendung einer topisch als Schaum auf die Haut eines warmblütigen Säugetieres zu applizierenden und ohne Treibmittel aufschäumbaren flüssigen Zusammensetzung nach einem der Ansprüche 10 bis 24 zur Herstellung eines Arzneimittels zur Behandlung von Infektionen mit grampositiven Keimen, anaeroben Keimen und Mykoplasmen, insbesondere zur Behandlung von Akne, wobei das Arzneimittel ein Antibiotikum, insbesondere Erythromycin, in einer Konzentration zwischen 2 Gew.% und 4 Gew.% enthält.

30. Verwendung einer topisch als Schaum auf die Haut eines warmblütigen Säugetieres zu applizierenden und ohne Treibmittel aufschäumbaren flüssigen Zusammensetzung nach einem der Ansprüche 10 bis 24 zur Herstellung eines Arzneimittels zur Behandlung von Juckreiz der Haut, wobei das Arzneimittel ein Lokalanästhetikum, insbesondere Benzocain und/oder Lidocain, in einer Konzentration zwischen 1 Gew.% und 20 Gew.%, vorzugsweise 2 Gew.% und 10 Gew.%, enthält.

31. Verwendung einer topisch als Schaum auf die Haut eines warmblütigen Säugetieres zu applizierenden und ohne Treibmittel aufschäumbaren flüssigen Zusammensetzung nach einem der Ansprüche 10 bis 24 zur Herstellung eines Arzneimittels zur Behandlung von Schuppenflechte, wobei das Arzneimittel den Wirkstoff Calcipotriol in einer Konzentration zwischen 0,005 Gew.% und 0,05 Gew.% enthält.

32. Verwendung einer topisch als Schaum auf die Haut eines warmblütigen Säugetieres zu applizierenden und ohne Treibmittel aufschäumbaren flüssigen Zusammensetzung nach einem der Ansprüche 10 bis 24 zur Herstellung eines Arzneimittels zur Behandlung von Akne, insbesondere Akne comedonica und Akne papulopustulosa, wobei das Arzneimittel den Wirkstoff Tretinoin in einer Konzentration zwischen 0,05 Gew.% und 0,1 Gew.% enthält.

33. Verwendung einer topisch als Schaum auf die Haut eines warmblütigen Säugetieres zu applizierenden und ohne Treibmittel aufschäumbaren flüssigen Zusammensetzung nach einem der Ansprüche 10 bis 24 zur Herstellung eines Arzneimittels zur Behandlung von Haarausfall, wobei das Arzneimittel den Wirkstoff Minoxidil in einer Konzentration zwischen 3 Gew.% und 6 Gew.% enthält.

34. Verwendung einer topisch als Schaum auf die Haut eines warmblütigen Säugetieres zu applizierenden und ohne Treibmittel aufschäumbaren flüssigen Zusammensetzung nach einem der Ansprüche 10 bis 24 zur Herstellung eines Arzneimittels zur antiseptischen Behandlung von oberflächlichen Wunden, wobei das Arzneimittel den Wirkstoff Dexpanthenol in einer Konzentration zwischen 0,03 Gew.% und 1 Gew.% enthält.

35. Verwendung einer topisch als Schaum auf die Haut eines warmblütigen Säugetieres zu applizierenden und ohne Treibmittel aufschäumbaren flüssigen Zusammensetzung nach einem der Ansprüche 10 bis 24 zur Herstellung eines Arzneimittels zur Behandlung von Herpes und den Herpes-begleitenden Nebenwirkungen, wobei das Arzneimittel den Wirkstoff Aciclovir in einer Konzentration zwischen 3 Gew.% und 7 Gew.% enthält.

36. Verwendung einer topisch als Schaum auf die Haut eines warmblütigen Säugetieres zu applizierenden und ohne Treibmittel aufschäumbaren flüssigen Zusammensetzung nach einem der Ansprüche 10 bis 24 zur Herstellung eines Arzneimittels zur Behandlung von leichter bis mittelschwerer Psoriasis der Kopfhaut, wobei das Arzneimittel den Wirkstoff Salicylsäure in einer Konzentration zwischen 8 Gew.% und 12 Gew.% enthält.

## Claims

1. A method for development of a liquid pharmaceutical composition to be applied as a foam to the skin, wherein the foamable liquid composition contains at least one solvent, at least one pharmaceutical active ingredient, and at least one phospholipidic foaming agent, **characterized in that**
a) the phospholipid foaming agent is such a phospholipidic foaming agent, which is isolated from soybeans and contains phosphatidylcholine in a concentration between 50 % by weight and 95 % by weight, whereby said phosphatidylcholine has an acid number of at most 10, a peroxide number of at most 10, and an oil concentration of at most 6 % by weight, relative to the dry weight of the phosphatidylcholine, that
b) the foam volume and the foam stability of the foam are determined by a SITA measurement method as being described by the examples, whereby the foam is created without the use of a propellant and whereby using the SITA measurement method 250 ml of the liquid composition are foamed with a rotor speed of 2000 rpm during five rotor cycles each of which have a duration of 20 seconds and a pause of 15 seconds between each rotor cycle and whereby the foam volume is measured every 50 seconds by means of the needle detectors over a period of 35 minutes, and that
c) in the liquid composition the at least one foaming agent, the at least one solvent and the at least one pharmaceutical active ingredient are varied so long in regard to their chemical nature and/or concentration until the foam created by the SITA measurement method has
d) a foam density between 0,05 g/ml and 0,8 g/ml,
e) a foam volume between 450 and 1400 ml, and
f) such a foam stability that the foam still has, after a dwell time of up to five minutes, at least 50% of the foam volume that was originally present immediately after the creation of the foam.

2. The method of claim 1, **characterized in that** the foam created by the SITA measurement method has a foam volume between 600 ml and 1200 ml.

3. The method according to claim 1 or 2, **characterized in that** the foam created by the SITA measurement method has such a foam stability that the foam still has, after a dwell time of up to ten minutes, between 55% and 100% of the foam volume that was originally present immediately after the creation of the foam.

4. The method according to claim 3, **characterized in that** the foam has after a dwell time of up to ten minutes at least a foam volume between 85% and 99% of the foam volume that was originally present immediately after the creation of the foam.

5. The method according to one of the preceding claims, **characterized in that** a correlation is produced between the foam specified by the SITA measurement method and the pharmaceutical properties.

6. The method according to one of the preceding claims, **characterized in that** a foam applicator is selected for the mechanical foaming of the liquid composition, that a foam is created from the liquid composition by the selected foam applicator and that a correlation is produced between the properties, especially the pharmaceutical properties of the foam created by the foam applicator and the foam volume and/or the foam stability as determined by the SITA measurement method.

7. The method according to one of the preceding claims, **characterized in that** such a foam is created from the liquid composition that possess a foam densities between 0,15 g/ml and 0,4 g/ml by the SITA measurement method.

8. The method according to one of the preceding claims, **characterized in that** the solvent for the production of the liquid composition is chosen from the group comprising water, at least one alcohol, especially at least one monovalent to trivalent alcohol, at least one polyalcohol and mixtures of the aforementioned solvents.

9. The method according to one of the preceding claims, **characterized in that** for producing the liquid composition furthermore a complexing agent, a buffer, a thickening agent, an antioxidant and/or a stabilizer are provided for the production of the liquid composition are used.

10. A composition suitable for topical use comprising at least one systemically and/or local acting pharmaceutical active ingredient, wherein the composition in addition to the pharmaceutical active ingredient also contains at least one solvent as well as at least one phospholipidic foaming agent and has such a fluid consistency that it forms a foam when applied, **characterized in that**
a) the phospholipid foaming agent is such a phospholipidic foaming agent, which is isolated from soybeans and contains phosphatidylcholine in a concentration between 50 % by weight and 95 % by weight, whereby said phosphatidylcholine has an acid number of at most 10, a peroxide number of at most 10, and an oil concentration of at most 6 % by weight, relative to the dry weight of the phosphatidylcholine, that
b) the at least one phospholipidic foaming agent, the at least one solvent and the at least one active ingredient are attuned to each other in such a way that the composition is mechanically foamable without using additional propellant and that the foam created in such a way has
c) a foam density between 0,05 g/ml and 0,8 g/ml,
d) a foam volume between 450 and 1400 ml, and
e) such a foam stability that the foam still has, after a dwell time of up to five minutes, at least 50% of the foam volume that was originally present immediately after the creation of the foam, whereby the foam volume as well as the foam stability are determined by the SITA measurement method as being described by the examples, and whereby using the SITA measurement method 250 ml of the liquid composition are foamed with a rotor speed of 2000 rpm during five rotor cycles each of which have a duration of 20 seconds and a pause of 15 seconds between each rotor cycle and whereby the foam volume is measured every 50 seconds by means of the needle detectors over a period of 35 minutes.

11. The composition according to claim 10, **characterized in that** the foam created by the SITA measurement method has a foam volume between 600 ml and 1200 ml.

12. The composition according to claim 10 or 11, **characterized in that** the foam created by the SITA measurement method has such a foam stability that the foam still has, after a dwell time of up to ten minutes, at least a foam volume between 55% and 100% of the foam volume that was originally present immediately after the creation of the foam.

13. The composition according to claim 12, **characterized in that** the foam has after a dwell time of up to ten minutes at least a foam volume between 85% and 99% of the foam volume that was originally present immediately after the creation of the foam.

14. The composition according to claim 10, **characterized in that** the foam created by the SITA measurement method using the composition has a foam density between 0,15 g/ml and 0,4 g/ml.

15. The composition according to one of the claims 10 to 14, **characterized in that** the composition contains as solvent water, at least one alcohol, especially at least one monovalent to trivalent alcohol and/or at least one polyalcohol.

16. The composition according to one of the claims 10 to 15, **characterized in that** the at least one active ingredient is an active ingredient for use on humans or animals and is chosen from the group comprising local anesthetics, anti-allergic agents, dermatics, active ingredients against flu infections and colds, active ingredients for the treatment of neuropathies, active ingredients for the treatment of disturbed circulation, chemotherapy drugs, quinine, antimycotics, antibiotics, thalidomide, serotonin, eicosanoids, analgesics, anticonvulsants, nonsteroidal antirrheumatics, leukotrienes, leukotriene inhibitors, androgens, antiandrogens, corticoids, opiate receptor antagonists, blood clotting inhibitory substances, thrombocyte aggregation inhibitors, histamine antagonists, regulatory and enzymatically acting peptides and proteins, nucleic acids (single and
double-stranded DNA, single and double-stranded RNA, snRNA, DNA oligonucleotides, RNA oligonucleotides) and oligopeptides, antipruritics, antidiabetics, prostaglandins, prostaglandin synthesis inhibitors, antiviral-acting or virostatic-acting substances, antimicrobial-acting substances, active ingredients against prions, immune suppressants, hormones, active ingredients for treatment of warts or wounds, especially chronic wounds, vitamins, plant extracts or essences of plant extracts, psychoactive drugs, active ingredients influencing sleep, analeptics, general anesthetics, muscle relaxants, antiepileptics, antiparkinson agents, antiemetics, antiparasitics, ganglion-active ingredients, sympathetic-active ingredients, parasympathetic-active ingredients, antibacterial-acting drugs, calcium antagonists, cardiovascular agents, antiasthmatics, antitussives, expectorants, hepatics, diuretics, choleretics, disinfectants, trace elements, antiinfectives, cytostatics, antimetabolites, hormone antagonists, immune modulators, as well as derivates and salts of the aforementioned active ingredients.

17. The composition according to claim 16, **characterized in that** the at least one analgesic is contained in the composition in a concentration between 0,1 % by weight and 20 % by weight, preferably in a concentration between 2 % by weight and 10 % by weight.

18. The composition according to one of the claims 16 or 17, **characterized in that** the at least one antimycotic is contained in the composition in a concentration between 0,01 % by weight and 10 % by weight, preferably in a concentration between 0,2 % by weight and 5 % by weight.

19. The composition according to one of the claims 16 to 18, **characterized in that** the composition has the at least one corticoid active ingredient in a concentration between 0,001 % by weight and 3 % by weight, preferably between 0,1 % by weight and 0,8 % by weight.

20. The composition according to one of the claims 16 to 19, **characterized in that** the composition has the at least one local anesthetic in a concentration between 3 % by weight and 15 % by weight, especially between 6 % by weight and 12 % by weight.

21. The composition according to one of the claims 10 to 20, **characterized in that** the composition contains the at least one immunomodulator in a concentration between 0,03 % by weight and 0,1 % by weight.

22. The composition according to claim 10, **characterized in that** at most 15 % by weight of lyso-phosphatidyl choline, at most 10 % by weight of phosphatidic acid and at most 10 % by weight of phosphatidyl ethanolamine are contained in the phospholipidic foaming agent.

23. The composition according to one of the claims 10 to 22, **characterized in that** the composition has the phospholipidic foaming agent in a concentration between 2 % by weight and 25 % by weight, preferably in a concentration between 4 % by weight and 15 % by weight.

24. The composition according to claim 15, **characterized in that** the composition contains, as alcohol propylene glycol in a concentration between 2 % by weight and 25 % by weight, preferably in a concentration between 5 % by weight and 15 % by weight.

25. The use of a liquid composition according to one of the claims 10 to 24, which is topically to apply on the skin of a warm-blooded mammal and foamable without the use of a propellant for producing a drug for treatment of atopic eczema or neurodermatitis, whereby the drug contains the immunomodulator tacrolimus in a concentration between 0,03 % by weight and 0,1 % by weight.

26. The use of a liquid composition according to one of the claims 10 to 24, which is topically to apply on the skin of a warm-blooded mammal and foamable without the use of a propellant for producing a drug for treatment of inflammatory or pruritic skin ailments, psoriasis, dermatitis, neurodermatitis or psoriasis whereby the drug contains a glucocorticoid, preferably betamethasone, dexamethasone, predincarbate, mometasone furoate and/or clobetasone butyrate, in a concentration between 0,01 % by weight and 0,4 % by weight.

27. The use of a liquid composition according to one of the claims 10 to 24, which is topically to apply on the skin of a warm-blooded mammal and foamable without the use of a propellant for producing a drug for treatment of pain, inflammation, rheumatic ailments or acute trauma, whereby the drug contains an analgesic, preferably diclofenac, ketoprofen and/or ibuprofen in a concentration between 0,1 % by weight and 20 % by weight, preferably 2 % by weight and 10 % by weight.

28. The use of a liquid composition according to one of the claims 10 to 24, which is topically to apply on the skin of a warm-blooded mammal and foamable without the use of a propellant for producing a drug for treatment of mycotic infections, whereby the drug contains an antimycotic, preferably bifonazole and/or terbinafin, in a concentration between 0,5 % by weight and 10 % by weight.

29. The use of a liquid composition according to one of the claims 10 to 24, which is topically to apply on the skin of a warm-blooded mammal and foamable without the use of a propellant for producing a drug for treatment of infections with Gram positive microbes, anaerobic microbes and mycoplasms, especially for treatment of acne, whereby the drug contains an antibiotic, preferably erythromycin, in a concentration between 2 % by weight and 4 % by weight.

30. The use of a liquid composition according to one of the claims 10 to 24, which is topically to apply on the skin of a warm-blooded mammal and foamable without the use of a propellant for producing a drug for treatment of itching of the skin, whereby the drug contains a local anesthetic, preferably benzocaine and/or lidocaine, in a concentration between 1 % by weight and 20 % by weight, preferably 2 % by weight and 10 % by weight.

31. The use of a liquid composition according to one of the claims 10 to 24, which is topically to apply on the skin of a warm-blooded mammal and foamable without the use of a propellant for producing a drug for treatment of psoriasis, whereby the drug contains the active ingredient calcipotriol in a concentration between 0,005 % by weight and 0,05 % by weight.

32. The use of a liquid composition according to one of the claims 10 to 24, which is topically to apply on the skin of a warm-blooded mammal and foamable without the use of a propellant for producing a drug for treatment of acne, especially acne comedonica and acne papulopustulosa, whereby the drug contains the active ingredient tretinoin in a concentration between 0,05 % by weight and 0,1 % by weight.

33. The use of a liquid composition according to one of the claims 10 to 24, which is topically to apply on the skin of a warm-blooded mammal and foamable without the use of a propellant for producing a drug for treatment of hair loss, whereby the drug contains the active ingredient minoxidil in a concentration between 3 % by weight and 6 % by weight.

34. The use of a liquid composition according to one of the claims 10 to 24, which is topically to apply on the skin of a warm-blooded mammal and foamable without the use of a propellant for producing a drug for treatment of antiseptic treatment of superficial wounds, whereby the drug contains the active ingredient dexpanthenol in a concentration between 0,03 % by weight and 1 % by weight.

35. The use of a liquid composition according to one of the claims 10 to 24, which is topically to apply on the skin of a warm-blooded mammal and foamable without the use of a propellant for producing a drug for treatment of herpes and the side effects accompanying herpes, whereby the drug contains the active ingredient aciclovir in a concentration between 3 % by weight and 7 % by weight.

36. The use of a liquid composition according to one of the claims 10 to 24, which is topically to apply on the skin of a warm-blooded mammal and foamable without the use of a propellant for producing a drug for treatment of mild to medium severe psoriasis of the scalp, whereby the drug contains the active agent salicylic acid in a concentration between 8 % by weight and 12 % by weight.

## Revendications

1. Procédé servant à mettre au point une composition pharmaceutique liquide à application cutanée, dans lequel la composition liquide moussante présente au moins un solvant, au moins un principe actif pharmaceutique ainsi qu'au moins un agent moussant phospholipidique, **caractérisé en ce que**
a) l'agent moussant phospholipidique est un agent moussant phospholipidique isolé de fèves de soja, lequel contient de la phosphatidylcholine en une concentration comprise entre 50 % en poids et 95 % en poids, dans lequel la phosphatidylcholine présente un indice d'acide de maximum 10, un indice de peroxyde de maximum 10 et une concentration en huile de maximum 6 %, par rapport au poids à sec de la phosphatidylcholine,
b) le volume de mousse et la stabilité de mousse de la mousse produite selon le procédé de mesure SITA, décrit dans les exemples de réalisation sans utilisation d'un agent porogène, sont définis, dans lequel 250 ml de la composition liquide sont moussés, lors du procédé de mesure SITA, à une vitesse de rotation de rotor de 2.000 t/min par cinq cycles de rotor de respectivement 20 secondes en respectant respectivement une pause de mesure d'environ 15 secondes entre les cycles de rotor et le volume de mousse respectif est relevé toutes les 50 secondes sur une durée de 35 minutes par l'intermédiaire de détecteurs à aiguilles, et
c) la nature chimique et/ou la concentration de l'au moins un agent moussant, de l'au moins un solvant et de l'au moins un principe actif varient dans la composition liquide jusqu'à ce que la mousse ainsi produite par le procédé de mesure SITA
d) possède une densité de mousse comprise entre 0,05 g/ml et 0,8 g/ml,
e) présente un volume de mousse compris entre 450 ml et 1.400 ml, et
f) a une stabilité de mousse telle que la mousse possède, après un temps de séjour pouvant aller jusqu'à 5 minutes encore au moins 50 % du volume de mousse, qui était présent initialement immédiatement après la production de la mousse.

2. Procédé selon la revendication 1, **caractérisé en ce que** la mousse produite par le procédé de mesure SITA présente un volume de mousse compris entre 600 ml et 1.200 ml.

3. Procédé selon la revendication 1 ou 2, **caractérisé en ce que** la mousse produite par le procédé de mesure SITA possède une stabilité de mousse telle que la mousse possède après un temps de séjour pouvant aller jusqu'à 10 minutes encore au moins entre 55 % et 100 % du volume de mousse, qui était présent initialement directement après la production de la mousse.

4. Procédé selon la revendication 3, **caractérisé en ce que** la mousse possède après un temps de séjour pouvant aller jusqu'à 10 minutes encore au moins entre 85 % et 99 % du volume de mousse, qui était présent initialement directement après la production de la mousse.

5. Procédé selon l'une quelconque des revendications précédentes, **caractérisé en ce qu'**une corrélation entre la mousse spécifiée selon le procédé de mesure SITA et les propriétés pharmaceutiques est établie.

6. Procédé selon l'une quelconque des revendications précédentes, **caractérisé en ce qu'**un applicateur de mousse servant à faire mousser la composition liquide est choisi, **en ce qu'**une mousse est produite à partir de la composition liquide par l'applicateur de mousse choisi, et **en ce qu'**une corrélation entre les propriétés, en particulier les propriétés pharmaceutiques, de la mousse produite par l'intermédiaire de l'applicateur de mousse et le volume de mousse et/ou la stabilité de mousse déterminés par l'intermédiaire du procédé de mesure SITA est établie.

7. Procédé selon l'une quelconque des revendications précédentes, **caractérisé en ce qu'**est produite, à partir de la composition liquide, par le procédé de mesure SITA, une mousse de ce type, qui possède des densités de mousse comprises entre 0,15 g/ml et 0,4 g/ml.

8. Procédé selon l'une quelconque des revendications précédentes, **caractérisé en ce que** le solvant pour la fabrication de la composition liquide est choisi parmi le groupe, qui comprend l'eau, au moins un alcool, en particulier au moins un alcool monovalent à trivalent, au moins un polyalcool et des mélanges des solvants mentionnés préalablement.

9. Procédé selon l'une quelconque des revendications précédentes, **caractérisé en ce qu'**est prévu par ailleurs, aux fins de la fabrication de la composition liquide, un agent complexant, un tampon, un agent épaississant, un agent antioxydant et/ou un agent stabilisant.

10. Composition à application topique comprenant au moins un principe actif pharmaceutique ayant une action systémique et/ou locale, dans lequelle la composition contient, outre l'au moins un principe actif pharmaceutique, par ailleurs au moins un solvant ainsi qu'au moins un agent moussant phospholipidique et présente une consistance liquide telle qu'elle forme au cours de l'application une mousse, **caractérisée en ce que**
a) l'agent moussant phospholipidique est un agent moussant phospholipidique isolé de fèves de soja, qui contient de la phosphatidylcholine en une concentration comprise entre 50 % en poids et 95 % en poids, dans laquelle la phosphatidylcholine présente un indice d'acide de maximum 10, un indice peroxyde de maximum 10 et une concentration en huile de maximum 6 % en poids par rapport au poids à sec de la phosphatidylcholine,
b) la nature chimique et/ou la concentration de l'au moins un agent moussant phospholipidique, de l'au moins un solvant et de l'au moins un principe actif sont adaptées les unes aux autres de telle manière que la composition peut mousser de manière mécanique sans utiliser un agent porogène supplémentaire, et **en ce que** la mousse ainsi produite
c) présente une densité de mousse comprise entre 0,05 g/ml et 0,8 g/ml,
d) présente un volume de mousse compris entre 450 ml et 1.400 ml, et
e) a une stabilité de mousse telle que la mousse possède, après un temps de séjour pouvant aller jusqu'à 5 minutes, encore au moins 50 % du volume de mousse, qui était présent initialement directement après la production de mousse, dans laquelle aussi bien le volume de mousse que la stabilité de mousse également sont définis selon le procédé de mesure SITA décrit dans les exemples de réalisation et dans laquelle le procédé de mesure SITA fait mousser 250 ml de la composition liquide à une vitesse de rotation de rotor de 2.000 t/min par cinq cycles de rotor de respectivement 20 secondes en respectant respectivement une pause de mesure d'environ 15 secondes entre les cycles de rotor et le volume de mousse respectif est relevé toutes les 50 secondes sur une durée de 35 minutes par l'intermédiaire de détecteurs à aiguilles.

11. Composition selon la revendication 10, **caractérisé en ce que** la mousse produite par le procédé de mesure SITA possède un volume de mousse compris entre 600 ml et 1.200 ml.

12. Composition selon la revendication 10 ou 11, **caractérisée en ce que** la mousse produite par le procédé de mesure SITA possède une stabilité de mousse telle que la mousse possède après un temps de séjour pouvant aller jusqu'à 10 minutes encore au moins entre 55 % et 100 % du volume de mousse, qui était présent initialement directement après la production de la mousse.

13. Composition selon la revendication 12, **caractérisée en ce que** la mousse possède, après un temps de séjour pouvant aller jusqu'à 10 minutes, encore au moins entre 85 % et 99 % du volume de mousse, qui était présent initialement directement après la production de la mousse.

14. Composition selon la revendication 10, **caractérisée en ce que** la mousse produite à partir de la composition par le procédé de mesure SITA possède une densité de mousse comprise entre 0,15 g/ml et 0,4 g/ml.

15. Composition selon l'une quelconque des revendications 10 à 14, **caractérisée en ce que** la composition contient en tant que solvant de l'eau, au moins un alcool, en particulier au moins un alcool monovalent à trivalent, et/ou au moins un polyalcool.

16. Composition selon l'une quelconque des revendications 10 à 15, **caractérisé en ce que** l'au moins un principe actif est un principe actif pouvant être utilisé chez l'homme ou chez les animaux et est choisi parmi le groupe, qui comprend les anesthésiques locaux, les antiallergiques, les produits de dermatologie, les principes actifs contre les états grippaux et les rhumes, des principes actifs pour le traitement de neuropathies, des principes actifs pour le traitement des troubles circulatoires, des médicaments chimiothérapeutiques, la quinine, des antibiotiques, la thalidomide, la sérotonine, les éicosanoïdes, les analgésiques, les anticonvulsivants, les anti-rhumastismaux non stéroïdiens, les leucotriènes, des inhibiteurs de leucotriène, des androgènes, des antiandrogènes, des corticoïdes, des antagonistes de récepteur d'opiacée, des substances inhibant la coagulation du sang, des antiagrégants plaquettaires, des antagonistes des récepteurs d'histamine, des peptides et protéines à action régulatrice et enzymatique, des acides nucléiques (ADN monobrin et à double brin, ARN monobrin et à double brin, snARN, des oligonucléotides d'ADN, des oligonucléotides d'ARN) et des oligopeptides, des antiprurigineux, des antidiabétiques, de la prostaglandine, des inhibiteurs de synthèse de prostaglandine, des substances à action antivirale ou à action virostatique, des substances anti-microbiennes, des principes actifs contre les prions, des immunosuppresseurs, des hormones, des principes actifs pour le traitement des verrues ou des plaies, en particulier de plaies chroniques, des vitamines, des extraits de plantes ou des dérivés issus d'extraits de plantes, des psychotropes, des principes actifs ayant une incidence sur le sommeil, des analeptiques, des anesthésiques généraux, des décontractants, des antiépileptiques, des antiparkinsoniens, des antiémétiques, des antiparasitaires, des substances attaquant les ganglions, des substances attaquant le système nerveux sympathique, des substances attaquant le système nerveux parasympathique, des produits pharmaceutiques à action antibactérienne, des antagonistes du calcium, des médicaments pour le coeur/pour la circulation, des antiasthmatiques, des antitussifs, des expectorants, des médicaments hépatiques, des diurétiques, des cholérétiques, des produits désinfectants, des oligo-éléments, des anti-infectieux, des médicaments cytostatiques, des antimétabolites, des antagonistes d'hormone, des agents immunomodulateurs ainsi que des dérivés et des sels des principes actifs susmentionnés.

17. Composition selon la revendication 16, **caractérisée en ce que** l'au moins un analgésique est contenu dans la composition en une concentration comprise entre 0,1 % en poids et 20 % en poids, de préférence en une concentration comprise entre 2 % en poids et 10 % en poids.

18. Composition selon la revendication 16 ou 17, **caractérisée en ce que** l'au moins un antimycosique est contenu dans la composition en une concentration comprise entre 0,01 % en poids et 10 % en poids, de préférence en une concentration comprise entre 0,2 % en poids et 5 % en poids.

19. Composition selon l'une quelconque des revendications 16 à 18, **caractérisée en ce que** la composition présente l'au moins un principe actif à base de corticoïde en une concentration comprise entre 0,001 % en poids et 3 % en poids, de préférence entre 0,1 % en poids et 0,8 % en poids.

20. Composition selon la revendication 16 à 19, **caractérisée en ce que** la composition présente l'au moins un anesthésique local en une concentration comprise entre 3 % en poids et 15 % en poids, en particulier comprise entre 6 % en poids et 12 % en poids.

21. Composition selon l'une quelconque des revendications 10 à 20, **caractérisée en ce que** la composition contient l'au moins un agent immunomodulateur en une concentration comprise entre 0,03 % en poids et 0,1 % en poids.

22. Composition selon la revendication 10, **caractérisée en ce qu'**au maximum 15 % en poids de lysophosphatidylcholine, au maximum 10 % en poids d'acide phosphatidique et au maximum 10 % en poids de phosphatidyléthanolamine sont contenus dans l'agent moussant phospholipidique.

23. Composition selon l'une quelconque des revendications 10 à 22, **caractérisée en ce que** la composition présente l'agent moussant phospholipidique en une concentration comprise entre 2 % en poids et 25 % en poids, de préférence en une concentration comprise entre 4 % en poids et 15 % en poids.

24. Composition selon la revendication 15, **caractérisée en ce que** la composition contient en tant qu'alcool du propylène glycol en une concentration comprise entre 2 % en poids et 25 % en poids, de préférence en une concentration comprise entre 5 % en poids et 15 % en poids.

25. Utilisation d'une composition liquide à appliquer sous la forme d'une mousse topique sur la peau d'un mammifère à sang chaud et pouvant mousser sans agent porogène selon l'une quelconque des revendications 10 à 24 afin de fabriquer un médicament pour le traitement de l'eczéma atopique ou de la neurodermatite, dans laquelle le médicament contient l'agent immunomudulateur qu'est le tacrolimus, en une concentration comprise entre 0,03 % en poids et 0,1 % en poids.

26. Utilisation d'une composition liquide à appliquer sous la forme d'une mousse topique sur la peau d'un mammifère à sang chaud et pouvant mousser sans agent porogène selon l'une quelconque des revendications 10 à 24 afin de fabriquer un médicament pour le traitement de maladies de la peau inflammatoires et provoquant des démangeaisons, le psoriasis, la dermatite, la neurodermatite, dans laquelle le médicament contient un glucocorticoïde, en particulier de la betaméthasone, de la dexaméthasone, le prednicarbate, le furoate de mométasone et/ou le butyrate de clobétasone, en une concentration comprise entre 0,01 % en poids et 0,4 % en poids.

27. Utilisation d'une composition liquide à appliquer sous la forme d'une mousse topique sur la peau d'un mammifère à sang chaud et pouvant mousser sans agent porogène selon l'une quelconque des revendications 10 à 24 afin de fabriquer un médicament pour le traitement de douleurs, d'inflammations, d'affections rhumatologiques ou de traumatismes aigus, dans laquelle le médicament contient un analgésique, en particulier du diclofenac, du kétoprofène et/ou de l'ibuprofène, en une concentration comprise entre 0,1 % en poids et 20 % en poids, de préférence en une concentration comprise entre 2 % en poids et 10 % en poids.

28. Utilisation d'une composition liquide à appliquer sous la forme d'une mousse topique sur la peau d'un mammifère à sang chaud et pouvant mousser sans agent porogène selon l'une quelconque des revendications 10 à 24 afin de fabriquer un médicament pour le traitement d'infections mycosiques, dans laquelle le médicament contient un antimycosique, en particulier le bifonazole et/ou la terbinafine, en une concentration comprise entre 0,5 % en poids et 10 % en poids.

29. Utilisation d'une composition liquide à appliquer sous la forme d'une mousse topique sur la peau d'un mammifère à sang chaud et pouvant mousser sans agent porogène selon l'une quelconque des revendications 10 à 24 afin de fabriquer un médicament pour le traitement d'infections bactériennes dues à des germes Gram+, des germes anaérobies et à des mycoplasmas, en particulier pour le traitement de l'acné, dans laquelle le médicament contient un antibiotique, en particulier de l'érythromycine, en une concentration comprise entre 2 % en poids et 4 % en poids.

30. Utilisation d'une composition liquide à appliquer sous la forme d'une mousse topique sur la peau d'un mammifère à sang chaud et pouvant mousser sans agent porogène selon l'une quelconque des revendications 10 à 24 afin de fabriquer un médicament pour le traitement des démangeaisons cutanées, dans laquelle le médicament contient un anesthésique local, en particulier de la benzocaïne et/ou de la lidocaïne, en une concentration comprise entre 1 % en poids et 20 % en poids, de préférence entre 2 % en poids et 10 % en poids.

31. Utilisation d'une composition liquide à appliquer sous la forme d'une mousse topique sur la peau d'un mammifère à sang chaud et pouvant mousser sans agent porogène selon l'une quelconque des revendications 10 à 24 afin de fabriquer un médicament pour le traitement du psoriasis, dans laquelle le médicament contient le principe actif qu'est le calcipotriol en une concentration comprise entre 0,005 % en poids et 0,05 % en poids.

32. Utilisation d'une composition liquide à appliquer sous la forme d'une mousse topique sur la peau d'un mammifère à sang chaud et pouvant mousser sans agent porogène selon l'une quelconque des revendications 10 à 24 afin de fabriquer un médicament pour le traitement de l'acné, en particulier de l'acné comédonienne ou de l'acné papulo-pustuleuse, dans laquelle le médicament contient le principe actif qu'est le trétinoïne, en une concentration comprise entre 0,05 % en poids et 0,1 % en poids.

33. Utilisation d'une composition liquide à appliquer sous la forme d'une mousse topique sur la peau d'un mammifère à sang chaud et pouvant mousser sans agent porogène selon l'une quelconque des revendications 10 à 24 afin de fabriquer un médicament pour le traitement de la chute des cheveux, dans laquelle le médicament contient le principe actif qu'est le minoxidil, en une concentration comprise entre 3 % en poids et 6 % en poids.

34. Utilisation d'une composition liquide à appliquer sous la forme d'une mousse topique sur la peau d'un mammifère à sang chaud et pouvant mousser sans agent porogène selon l'une quelconque des revendications 10 à 24 afin de fabriquer un médicament pour le traitement antiseptique de plaies superficielles, dans laquelle le médicament contient le principe actif qu'est le dexpanthénol, en une concentration comprise entre 0,03 % en poids et 1 % en poids.

35. Utilisation d'une composition liquide à appliquer sous la forme d'une mousse topique sur la peau d'un mammifère à sang chaud et pouvant mousser sans agent porogène selon l'une quelconque des revendications 10 à 24 afin de fabriquer un médicament pour le traitement de l'herpès et des effets secondaires accompagnant l'herpès, dans laquelle le médicament contient le principe actif qu'est l'aciclovir en une concentration comprise entre 3 % en poids et 7 % en poids.

36. Utilisation d'une composition liquide à appliquer sous la forme d'une mousse topique sur la peau d'un mammifère à sang chaud et pouvant mousser sans agent porogène selon l'une quelconque des revendications 10 à 24 afin de fabriquer un médicament pour le traitement du psoriasis du cuir chevelu à stade léger à moyen, dans laquelle le médicament contient le principe actif qu'est l'acide salicylique en une concentration comprise entre 8 % en poids et 12 % en poids.
